# EUROPEAN PATENT SPECIFICATION

(11) **EP 3 008 088 B1**
(45) Date of publication and mention of the grant of the patent: **21.08.2019**
(21) Application number: 14738954.8
(22) Date of filing: 12.06.2014
(51) Int. Cl.: C07K 16/12, A61K 39/104

(54) **COMPOSITIONS AND METHODS FOR THE TREATMENT OF BURKHOLDERIA INFECTIONS**
ZUSAMMENSETZUNGEN UND VERFAHREN ZUR BEHANDLUNG VON BURKHOLDERIA-INFEKTIONEN
COMPOSITIONS ET PROCÉDÉS POUR LE TRAITEMENT D'INFECTIONS PAR BURKHOLDERIA

(30) Priority: 13.06.2013 US 201361834846 P
(43) Date of publication of application: 20.04.2016
(73) Proprietor: Research Institute at Nationwide Children's Hospital, Columbus, Ohio 43205 (US); University of Southern California, Los Angeles, CA 90015 (US); Ohio State Innovation Foundation, Columbus, OH 43201 (US)
(72) Inventor: GOODMAN, Steven D., Los Angeles, California 90015 (US); BAKALETZ, Lauren O., Columbus, Ohio 43205 (US); AMER, Amal, Columbus, Ohio 43201 (US)
(74) Representative: Tomkins & Co
(86) International application number: PCT/US2014/042201
(87) International publication number: WO 2014/201305

(56) References cited:
- WO-A1-2011/123396
- WO-A1-2012/034090
- JODI E. GUSTAVE ET AL: "Targeting bacterial integration host factor to disrupt biofilms associated with cystic fibrosis", JOURNAL OF CYSTIC FIBROSIS, vol. 12, no. 4, 17 November 2012 (2012-11-17), pages 384-389, XP055151085, ISSN: 1569-1993, DOI: 10.1016/j.jcf.2012.10.011 cited in the application
- GOODMAN S D ET AL: "Biofilms can be dispersed by focusing the immune system on a common family of bacterial nucleoid-associated proteins", MUCOSAL IMMUNOLOGY, NATURE PUBLISHING GROUP, US, vol. 4, no. 6, 1 November 2011 (2011-11-01), pages 625-637, XP009155507, ISSN: 1933-0219, DOI: 10.1038/MI.2011.27 cited in the application
- KATHLEYN A. BRANDSTETTER ET AL: "Antibodies directed against integration host factor mediate biofilm clearance from nasopore", THE LARYNGOSCOPE, vol. 123, no. 11, 13 May 2013 (2013-05-13) , pages 2626-2632, XP055147801, ISSN: 0023-852X, DOI: 10.1002/lary.24183
- ESTRELA A B ET AL: "Combining biofilm-controlling compounds and antibiotics as a promising new way to control biofilm infections", PHARMACEUTICALS, M D P I AG, CH, vol. 3, no. 5, 1 May 2010 (2010-05-01), pages 1374-1393, XP009155567, ISSN: 1424-8247, DOI: 10.3390/PH3051374 [retrieved on 2010-05-11]
- J R GOVAN ET AL: "Microbial pathogenesis in cystic fibrosis: mucoid Pseudomonas aeruginosa and Burkholderia cepacia.", MICROBIOL. REV, vol. 60, no. 3, 1 January 1996 (1996-01-01), pages 539-574, XP055153835,
- C. B. WHITCHURCH: "Extracellular DNA Required for Bacterial Biofilm Formation", SCIENCE, vol. 295, no. 5559, 22 February 2002 (2002-02-22), pages 1487-1487, XP055002505, ISSN: 0036-8075, DOI: 10.1126/science.295.5559.1487 cited in the application -& Cynthia B Whitchurch ET AL: "Extracellular DNA Required for Bacterial Biofilm Formation - Supplemental Data", , 22 February 2002 (2002-02-22), XP055153639, Retrieved from the Internet: URL:http://www.sciencemag.org/content/295/ 5559/1487/suppl/DC1 [retrieved on 2014-11-18]
- LAURA A. NOVOTNY ET AL: "Structural Stability of Burkholderia cenocepacia Biofilms Is Reliant on eDNA Structure and Presence of a Bacterial Nucleic Acid Binding Protein", PLOS ONE, vol. 8, no. 6, 14 June 2013 (2013-06-14), page e67629, XP055147798, ISSN: 1932-6203, DOI: 10.1371/journal.pone.0067629
- M. ELIZABETH BROCKSON ET AL: "Evaluation of the kinetics and mechanism of action of anti-integration host factor-mediated disruption of bacterial biofilms", MOLECULAR MICROBIOLOGY, 19 September 2014 (2014-09-19), pages n/a-n/a, XP055147799, ISSN: 0950-382X, DOI: 10.1111/mmi.12735 -& M. ELIZABETH BROCKSON ET AL: "Evaluation of the kinetics and mechanism of action of anti-integration host factor-mediated disruption of bacterial biofilms; Supplementary Materials", MOLECULAR MICROBIOLOGY, vol. 93, no. 6, 19 September 2014 (2014-09-19), XP055151782,

## Description

### BACKGROUND

Throughout this disclosure references, such as technical, scientific and patent publications, are noted within parentheses. This information is incorporated by reference in its entirety into the present disclosure to more fully describe the state of the art and support the claimed subject matter.

Cystic Fibrosis (CF) is the most common inherited lethal disorder affecting Caucasians (Campana et al. (2004) J. Cyst. Fibros. 3:159-163). The greatest impact of this genetic disorder is the inability of CF patients to eradicate bacterial pathogens from their lungs. As a result, a chronic infectious state develops, which includes a vicious cycle of bacterial proliferation followed by retreat into an adhered extracellular biofilm state and/or invasion of local host cells. With each cycle of acute proliferation, an intense host response is elicited, and in an attempt to clear these infections, the over-exuberant inflammatory response causes damage to lung tissues, resulting in scarring that impairs pulmonary function and is often fatal. Even today, at least 90% of CF patients die from respiratory failure. Current treatment modalities rely heavily on antibiotic use, however, certain pathogens remain highly recalcitrant to antibiotic treatment, a phenotype that is significantly contributed to by their ability to reside within a biofilm. Once biofilm formation occurs and infection is established in the CF lung, eradication of bacteria is rare (George et al. (2009) FEMS Microbiol. Lett. 300:153-164).

Whereas the major pathogen in adult CF patients is *Pseudomonas aeruginosa*, amongst the most deleterious CF-associated pathogens are members of the *Burkholderia cepacia complex* (*Bcc*), with perhaps the most virulent member being *Burkholderia cenocepacia*, a Gram-negative opportunistic pathogen. Of the 17 formally named species within the complex, *B. multivorans* and *B. cenocepacia* dominate in CF (Simpson et al. (1994) J. Antimicrob. Chemother. 34:353-361; Butler et al. (1995) J. Clin. Microbiol. 33:1001-1004; Castellani et al. (1995) Arch Dis Child 73:276; LiPuma et al. (1995) N. Engl. J. Med. 332:820-821) accounting for approximately 85-97% of all *Burkholderia* infections. While CF patients infected with any *Bcc* species often have a poor prognosis, infection with *B. cenocepacia* is considered more serious and associated with both reduced survival and greater risk for development of fatal 'cepacia syndrome' (Simpson et al. (1994) J. Antimicrob. Chemother. 34:353-361; Butler et al. (1995) J. Clin. Microbiol. 33:1001-1004; Castellani et al. (1995) Arch. Dis. Child. 73:276; LiPuma et al. (1995) N. Engl. J. Med. 332:820-821; Burns et al. (1999) Pediatr. Infect. Dis. J. 18:155-156; Hopkins et al. (2009) Am. J. Respir. Crit. Care Med. 179:257-258; De Soyza et al. (2010) J. Heart. Lung Transplant 29:1395-1404; Nash et al. (2010) Transpl. Infect. Dis. 12:551-554). *B. cenocepacia* is intrinsically resistant to polymyxins, aminoglycosides and most β-lactams, and can develop resistance to essentially all classes of antimicrobial drugs (Aaron et al. (2000) Am. J. Respir. Crit. Care Med. 161:1206-1212; Golini et al. (2006) Eur. J. Clin. Microbiol. Infect. Dis. 25:175-180; Dubarry et al. (2010) Appl. Environ. Microbiol. 76:1095-1102).

*B. cenocepacia* gained notoriety as a pathogen in CF because it is difficult to identify and treat, and also due to its ability to readily spread between individuals with CF. Even outside CF, highly transmissible epidemic strains of *B. cenocepacia* have been shown to contaminate healthcare settings and spread nosocomially (Graindorge et al. (2010) Diagn. Microbiol. Infect. Dis. 66:29-40). It is also likely that multi-drug resistant *B. cenocepacia* can transfer mechanisms of resistance to other microbes present within the human airway, and particularly those co-colonizing the CF lung. This assumption is supported by the observation that 25-45% of adult CF patients are chronically infected with multiple multi-drug resistant bacteria (Lechtzin et al. (2006) Respiration 73:27-33).

The pathogenesis of CF airway disease is multifactorial and includes defective antimicrobial activity in airway secretions, altered mucociliary clearance, abnormal submucosal gland function and overproduction of reactive oxygen species (ROS). Chronic inflammation is most central to CF pathogenesis as a consequence of pulmonary infections and leads to lung damage resulting in 85% of deaths. Further contributing to enhanced lung pathology observed in CF patients is an overabundance of cytokine-secreting alveolar macrophages. Pro-inflammatory mediators such as IL-1β, IL-8, TNF-α and anti-inflammatory IL-10 are detected in CF patients, even in young patients in the absence of culture-positive infection. Moreover, whereas alveolar macrophages typically engulf microbes that gain access to the lungs, enclose them into vacuoles which fuse with lysosomes where the contents are degraded and eliminated via a process called autophagy, CF macrophages are defective in this regard. This inherent weak autophagy (Luciani et al. (2010) Nat. Cell. Biol. 12:863-875; Luciani et al. (2011) Autophagy 7:104-106; Luciani et al. (2012) Autophagy 8) is further augmented by the ability of *B. cenocepacia* to downregulate essential autophagy molecules (Abdulrahman et al. (2011) Autophagy 7:1359-1370).

WO 2011/123396 discloses recombinant polypeptides for use in vaccinating individuals suffering from chronic or recurrent biofilm diseases and as a therapeutic for those with existing infections.

WO212/034090 discloses methods of breaking down a biofilm, or inhibiting, preventing or treating a microbial infection that produces a biofilm, which involves administration of a polypeptide that has one or more HMG-box domains.

Gustave et al, Journal of Cystic Fibrosis, volume 12, pages 384-389 discloses the targeting of bacterial integration host factors to destruct biofilms associated with cystic fibrosis. Brandstetter et al, The Laryngoscope, vol. 1233, pages 2626-2632 discloses antibodies directed against integration host factors which mediate biofilm clearance from the nasopore. Estrella et al, Pharmaceuticals, volume 3, pages 1374-1393 describes a combination of biofilm-controlling compounds and antibiotics as a way to control biofilm infections. Govan et al, Microbiol. Rev. volume 60, pages 539-574 describes the involvement of mucoid Pseudomonas aeruginosa and Burkholderia cepacia in the microbial pathogenesis of cystic fibrosis.

Infection of a CF patient with *B. cenocepacia* is considered a virtual death sentence as these patients are extremely difficult to treat and are also ineligible for lung transplant, often their last lifesaving resort. Thereby, the need to develop novel, highly effective approaches to eradicate *B. cenocepacia* from the lungs of CF patients, as well as from medical environments, cannot be underestimated. This invention satisfies this need and provides related advantages as well.

### SUMMARY OF THE DISCLOSURE

The inventors have identified a protein target for the development of novel approach for the treatment of CF infections. This protein target, a member of the DNABII family of DNA binding proteins, is essential for biofilm formation and stability by multiple human pathogens (Goodman et al. (2011) Mucosal Immunol. 4:625-637; Justice et al. (2012) PLoS One 7:e48349; Gustave et al. (2012) J. Cyst. Fibros.) due to its contribution to the structural lattice of extracellular DNA (eDNA) within these bacterial communities. The DNABII family is a member of a class of proteins referred to as nucleoid associated proteins (NAPs), bacterial proteins that, in part, shape the intracellular bacterial nucleoid (Browning et al. (2010) Curr. Opin. Microbiol. 13:773-780). In addition, this family is ubiquitous, expressed by virtually all eubacteria. All characterized family members to date function as either a homodimer or heterodimer of subunits. The family is divided into two types, HU (histone-like protein) and IHF (integration host factor) with *B. cenocepacia* capable of expressing both (strain J2315 genes: BCAL3530, *hupA*; BCAL1585, *hupB;* BCAL1487, *ihfA* and BCAL2949, *ihfb*). The primary distinction between these family members is that HU binds DNA in a sequence independent manner, while IHF binds a consensus sequence [WATCAANNNNTTR where W is A or T and R is a purine, SEQ ID NO. 36) conserved across *genera* (Swinger et al. (2004) Curr. Opin. Struct. Biol. 14:28-35)]. All DNABII proteins bind to and bend DNA considerably *e.g. E. coli* IHF can bend DNA into a virtual U-turn (Rice et al. (1996) Cell 87: 1295-1306). In addition, all family members have a preference for pre-bent or curved DNA structures e.g. Holliday junctions, a cruciform-like structure central to DNA recombination. In fact, DNABII proteins function as accessory factors facilitating all intracellular DNA functions, including gene expression, recombination, repair and replication (Swinger et al. (2004) Curr. Opin. Struct. Biol. 14:28-35).

In the past 20 years, multiple investigators discovered that these proteins also exist extracellularly (Gao (2000) Los Angeles: University of Southern California; Winters et al. (1993) Infect. Immun. 61:3259-3264; Lunsford et al. (1996) Curr. Microbiol. 32:95-100; Boleij et al. (2009) Infect. Immun. 77:5519-5527). To date, three extracellular functions have been described. First, streptococcal HU is shown to elicit a powerful inflammatory innate immune response by inducing the release of TNFα and interleukin-1 (Zhang et al. (1999) Infect. Immun. 67:6473-6477). Interestingly, *B. cenocepacia* induces peripheral damage by exacerbating IL-1β production through the host receptor Pyrin by an as yet unknown mechanism (Gavrilin et al. (2012) Immunol. 188:3469-3477; Kotrange et al. (2011) J. Leukoc. Biol. 89:481-488). In this regard, an extracellular role of DNABII family members has yet to be tested for *B. cenocepacia.* Second, extracellular DNABII proteins bind laminin and are thought to elicit direct contact to host cells (Winters et al. (1993) Infect. Immun. 61:3259-3264). Third, DNABII proteins are known to stabilize the structural integrity of eDNA within the extra-cellular polymeric matrix or substance (or EPS) of the biofilm of multiple pathogens (Goodman et al. (2011) Mucosal Immunol. 4:625-637). Antisera directed against these proteins are sufficient to destabilize biofilms, resulting in exposure/release of the resident bacteria, thus sensitizing them to the action of both antimicrobials and effectors of the immune system (Goodman et al. (2011) Mucosal Immunol. 4:625-637). Recently, it's also been shown that both IHF subunits are required for efficient colonization of the urinary bladder by uropathogenic *E. coli* and further, that both IHF subunits influence the community architecture of intracellular bacterial communities (Justice et al. (2012) PLoS One 7:e48349). Without being bound by theory and considering the abundance of eDNA within a *B. cenocepacia* induced biofilm, the inventors investigated whether there existed a role for the DNABII family of proteins in stabilization of these biofilms as well and thus, whether this family of proteins serve as a target for therapeutic intervention. Moreover, the presence of extracellular DNABII proteins, perhaps in association with bacterial cells, might influence the interaction of *B. cenocepacia* with host cells and particularly with its primary reservoir, the macrophage.

Thus, after investigation and study, the inventors herein disclose an immunotherapeutic approach that targets the DNABII proteins, and particularly when coupled with existing conventional therapeutics, was found to be highly effective at both debulking biofilms formed by *B. cenocepacia* as well as rendering the bacteria now released from the biofilm EPS susceptible to the action of antibiotics. The methods can be used to diminish and eradicate reservoirs of *B. cenocepacia* from the lungs of CF patients. Moreover, this method is shown to prevent *recurrence* of CF disease by inhibiting the ability of *B. cenocepacia* to multiply within macrophages isolated from CF mice, an important animal model of CF in humans.

Thus, in one aspect an antibody composition for (i) use in treating or preventing the recurrence of a *Burkholderia* infection in a Cystic Fibrosis (CF) patient in need thereof, (ii) use in treating an infection or disease caused by *Burkholderia* in a CF patient infected with *Burkholderia*, wherein the antibody specifically recognizes and binds the amino acid sequence RPGRNPKTGDVVPVSARRVV is provided.

In another aspect, the invention provides an isolated peptide consisting of the sequence RPGRNPKTGDVVPVSARRVV and optionally further comprising an adjuvant or detectable label, as well as an isolated antibody that specifically recognizes and binds that peptide, the antibody optionally being a polyclonal antibody, a monoclonal antibody or an antigen binding fragment thereof, that optionally further comprises a pharmaceutically acceptable carrier. The invention also provides a hybridoma cell line producing the monoclonal antibody and an isolated polynucleotide encoding an peptide or the antibody.

Also described is a method for treating or preventing the recurrence of a microbial infection in a CF patient or a patient at risk of developing the microbial infection, comprising, or alternatively consisting essentially of, or yet further consisting of administering to the subject an effective amount of an interfering agent, thereby treating or preventing the recurrence of a microbial infection in the CF patient.

The interfering agents can be combined with antimicrobials to further supplement the therapy, e.g., ceftazidime, ciprofloxacin, imipenem and minocycline. Thus, any of the above methods can further comprise, or consist essentially of, or yet further consist of administration or contacting with an effective amount of the antimicrobial, e.g., ceftazidime, ciprofloxacin, imipenem and minocycline. The administration or contacting of the interfering agent is performed in the absence of a DNase treatment. The DNAse treatment that is excluded from the therapy comprises an enzyme that catalyzes the cleavage of phosphodiester linkages in the DNA backbone. Three non-limiting examples of DNase enzymes that are known to target not only cruciform structures, but also a variety of secondary structure of DNA include DNAse I, T4 EndoVII and T7 Endo I. The DNase treatment that is excluded from the therapy comprises, or consists essentially of, or yet further consists of, Pulmozyme® (dornase alpha; Genentech, Inc.).

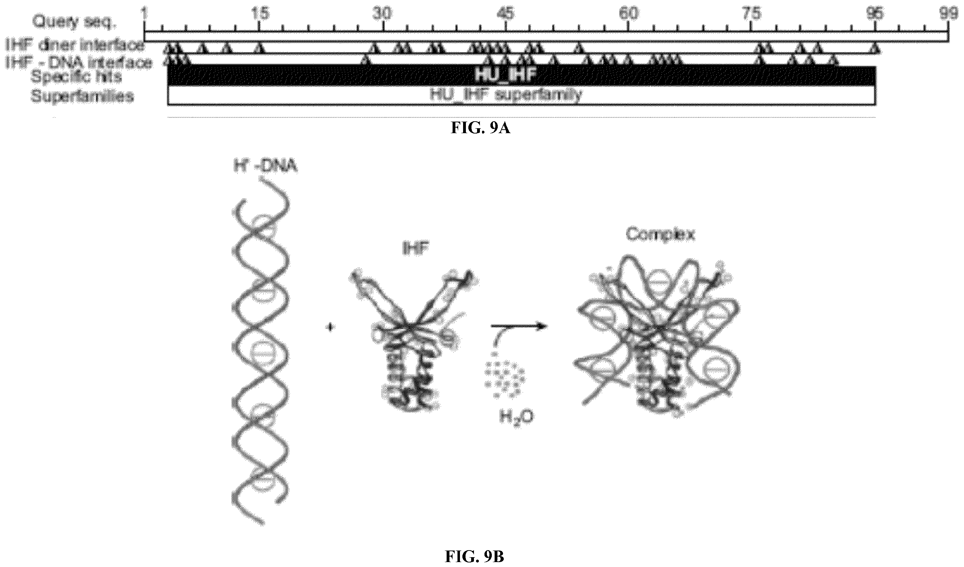

An agent that occludes critical surfaces that are required for intercellular persistence, interferes or impedes the binding of the microbial DNA to the DNABII protein or polypeptide include:
(a) an isolated or recombinant integration host factor (IHF) polypeptide or a fragment or an equivalent of each thereof;
(b) an isolated or recombinant protein or polypeptide identified in Table 1, Table 2, the Arm fragment identified in Table 2, Table 3 or a DNA binding peptide identified in Figure 9, or a fragment or an equivalent of each thereof;
(c) an isolated or recombinant polypeptide of SEQ ID NO. 1 through 33 or an equivalent thereof, or a fragment or an equivalent of each thereof;
(d) an isolated or recombinant c-terminal polypeptide of SEQ ID NO. 5 through 11, 28, 29 or those identified in Table 1, or a fragment or an equivalent of each thereof;
(e) a polypeptide that competes with or occludes critical surfaces without displacement or an integration host factor on binding to a microbial DNA;
(f) a four-way junction polynucleotide resembling a Holliday junction, a 3 way junction polynucleotide resembling a replication fork, a polynucleotide that has inherent flexibility or bent polynucleotide;
(g) an isolated or recombinant polynucleotide encoding any one of (a) through (e);
(h) an antibody or antigen binding fragment that specifically recognizes or binds any one of (a) through (e), or an equivalent or fragment of each thereof;
(i) an isolated or recombinant polynucleotide encoding the antibody or antigen binding fragment of (h); and
(j) a small molecule that competes with the binding of a DNA BII protein or polypeptide to a microbial DNA.

An isolated or recombinant polypeptide consisting essentially of an amino acid sequence selected from SEQ ID NO. 1 to 5 or 12 to 27 or 30 to 33, or a DNA binding peptide identified in Figure 9 is described.

The methods are performed with an isolated or recombinant polypeptide comprising, or alternatively consisting essentially of, or yet further consisting of, SEQ ID NO. 1 or 2, with the proviso that the polypeptide is none of SEQ ID NO. 6 to 11, 28 or 29.

The methods are performed with an isolated or recombinant polypeptide comprising, or alternatively consisting essentially of, or yet further consisting of, SEQ ID NO. 3, 4 or 5, with the proviso that the polypeptide is none of SEQ ID NO. 6 to 11, 28 or 29.

The methods are performed with an isolated or recombinant polypeptide comprising, or alternatively consisting essentially of, or yet further consisting of, SEQ ID NO. 12, 14, 16, 18, 20, 22, 24, 26, 30 or 32, with the proviso that the polypeptide is none of SEQ ID NO. 6 to 11, 28 or 29.

The methods are performed with an isolated or recombinant polypeptide comprising, or alternatively consisting essentially of, or yet further consisting of SEQ ID NO. 13, 15, 17, 19, 21, 23, 25, 27, 31 or 33, with the proviso that the polypeptide is none of SEQ ID NO. 6 to 11, 28 or 29.

The methods are performed with an isolated or recombinant polypeptide comprising, or alternatively consisting essentially of, or yet further consisting of, SEQ ID NO. 12 and 13 or 14 and 15 or 16 and 17 or 18 and 19 or 20 and 21 or 22 and 23 or 24 and 25, or 26 and 27 or 30 and 31 or 32 and 33, with the proviso that the polypeptide is none of SEQ ID NO. 6 to 11, 28 or 29.

The methods are performed with an isolated or recombinant polypeptide comprising, or alternatively consisting essentially of, or yet further consisting of, the c-terminal region or peptide of polypeptide of the group of a DNA BII polypeptide, an IHF polypeptide, SEQ ID NO. 6 through 11, 28, 29 or those identified in Table 1, or a fragment or an equivalent of each thereof.

Additional non-limiting examples of agents that can be used in the methods described include:
(a) an isolated or recombinant integration host factor (IHF) polypeptide or a fragment or an equivalent of each thereof;
(b) an isolated or recombinant histone-like protein from *E.coli* strain U93 (HU) polypeptide or a fragment or an equivalent of each thereof;
(c) an isolated or recombinant protein or polypeptide identified in Table 1, Table 2, the Arm fragment identified in Table 2, Table 3, Table 4 or a DNA binding peptide identified in FIG. 9, or a fragment or an equivalent of each thereof;
(d) an isolated or recombinant polypeptide of SEQ ID NO. 1 through 348, or a fragment or an equivalent of each thereof;
(e) an isolated or recombinant C-terminal polypeptide of SEQ ID NO. 6 through 11, 28, 29, 42 through 100, Table 1 or those C-terminal polypeptides identified in Table 4 or a fragment or an equivalent of each thereof;
(f) a polypeptide or polynucleotide that competes with an integration host factor on binding to a microbial DNA;
(g) a four-way junction polynucleotide resembling a Holliday junction, a 3 way junction polynucleotide resembling a replication fork, a polynucleotide that has inherent flexibility or bent polynucleotide;
(h) an isolated or recombinant polynucleotide encoding any one of (a) through (f) or an isolated or recombinant polynucleotide of SEQ NO. 36 or an equivalent of each thereof, or a polynucleotide that hybridizes under stringent conditions to the polynucleotide its equivalent or its complement;
(i) an antibody or antigen binding fragment that specifically recognizes or binds any one of (a) through (f), or an equivalent or fragment of each antibody or antigen binding fragment thereof;
(j) an isolated or recombinant polynucleotide encoding the antibody or antigen binding fragment of (i) or its complement; or
(k) a small molecule that competes with or occludes the binding of a DNABII protein or polypeptide to a microbial DNA.

Also described herein is a method for inducing an immune response in or conferring passive immunity in a CF subject in need thereof, comprising, or alternatively consisting essentially of or yet further consisting of, administering to the CF subject an effective amount of one or more agents of the group:
(a) an isolated or recombinant integration host factor (IHF) polypeptide, or a fragment or an equivalent of each thereof;
(b) an isolated or recombinant histone-like protein from *E.coli* strain U93 (HU) polypeptide or a fragment or an equivalent of each thereof;
(c) an isolated or recombinant protein polypeptide identified in Table 1, Table 2, the Arm fragment identified in Table 2, Table 3, Table 4, or an DNA binding peptide identified in FIG.9, or a fragment or an equivalent of each thereof;
(d) an isolated or recombinant polypeptide of SEQ ID NO. 1 through 348, or a fragment or an equivalent thereof;
(e) an isolated or recombinant C-terminal polypeptide of SEQ ID NO. 6 through 11, 28, 29, 42 through 100, Table 1 or those C-terminal polypeptides identified in Table 4 or a fragment or an equivalent of each thereof;
(f) an isolated or recombinant polynucleotide encoding any one of (a) through (e) or an isolated or recombinant polynucleotide SEQ ID NO. 36 or an equivalent of each thereof, or a polynucleotide that hybridizes under stringent conditions to the polynucleotide, its equivalent or its complement;
(g) an antibody or antigen binding fragment that specifically recognizes or binds any one of (a) through (e), or an equivalent or fragment of each thereof;
(h) an isolated or recombinant polynucleotide encoding the antibody or antigen binding fragment of (g);
(i) an antigen presenting cell pulsed with any one of (a) through (e); and
(j) an antigen presenting cell transfected with one or more polynucleotides encoding any one of (a) through (e).

Subjects in need of such immune response or support include those at risk of or suffering from an infection that produces a microbial biofilm.

Also described herein are polynucleotides, polypeptides and antibodies, antigen binding fragments, and compositions for use in the above methods, non-limiting examples of which are discussed below.

An isolated or recombinant polypeptide comprising, or alternatively consisting essentially of, an amino acid sequence selected from SEQ ID NO. 1 to 5 or 12 to 27, 30 to 35, 101-348 or a DNA binding peptide identified in FIG. 9 is described.

An isolated or recombinant polypeptide comprising, or alternatively consisting essentially of, or yet further consisting of SEQ ID NO. 1 or 2, with the proviso that the polypeptide is none of SEQ ID NO. 6 to 11, 28, 29, or 42 through 100 is described.

An isolated or recombinant polypeptide comprising, or alternatively consisting essentially of, or yet further consisting of SEQ ID NO. 3, 4 or 5, with the proviso that the polypeptide is none of SEQ ID NO. 6 to 11, 28, 29, or 42 through 100 is described .

An isolated or recombinant polypeptide comprising, or alternatively consisting essentially of, or yet further consisting of, SEQ ID NO. 12, 14, 16, 18, 20, 22, 24, 26, 30 or 32, with the proviso that the polypeptide is none of SEQ ID NO. 6 to 11, 28, 29, or 42 through 100 is described.

An isolated or recombinant polypeptide comprising, or alternatively consisting essentially of, or yet further consisting of SEQ ID NO. 13, 15, 17, 19, 21, 23, 25, 27, 31 or 33, with the proviso that the polypeptide is none of SEQ ID NO. 6 to 11, 28, 29, or 42 through 100 is described.

An isolated or recombinant polypeptide comprising, or alternatively consisting essentially of, or yet further consisting of SEQ ID NO. 337, 338, 339, or 340, with the proviso that the polypeptide is none of SEQ ID NO, 6 to 11, 28, 29, or 42 through 100 is described.

An isolated or recombinant polypeptide comprising, or alternatively consisting essentially of, or yet further consisting of, SEQ ID NO. 12 and 13 or 14 and 15 or 16 and 17 or 18 and 19 or 20 and 21 or 22 and 23 or 24 and 25, or 26 and 27 or 30 and 31 or 32 and 33, with the proviso that the polypeptide is none of SEQ ID NO. 6 to 11, 28, 29, or 42 through 100 is described.

An isolated or recombinant polypeptide comprising, or alternatively consisting essentially of, or yet further consisting of, the C-terminal region containing at least 10, or alternatively at least 15, or alternatively at least 20, or alternatively at least 25, or alternatively at least 30, C-terminal amino acids of a polypeptide of the group of a DNABII polypeptide, an IHF polypeptide, an HU polypeptide, SEQ ID NO. 6 through 11, 28, 29 or those identified in Table 1, Table 2, the Arm fragment identified in Table 2, Table 4 or a fragment or an equivalent of each thereof, or a polypeptide of SEQ ID NOs.: 337 to 340, or an equivalent thereof is described.

An isolated or recombinant polypeptide or polynucleotide of the group of:
a polypeptide comprising SEQ ID NO. 12 and 13;
a polypeptide comprising SEQ ID NO. 14 and 15;
a polypeptide comprising SEQ ID NO. 16 and 17;
a polypeptide comprising SEQ ID NO. 18 and 19;
a polypeptide comprising SEQ ID NO. 20 and 21;
a polypeptide comprising SEQ ID NO. 23 and 24;
a polypeptide comprising SEQ ID NO. 25 and 26;
a polypeptide comprising SEQ ID NO. 30 and 31;
a polypeptide comprising SEQ ID NO. 32 and 33;
a polypeptide comprising SEQ ID NO. 34 and 35;
a polypeptide comprising SEQ ID NO. 337 and 338; or
a polypeptide comprising SEQ ID NO. 339 and 340;
a polynucleotide or polypeptide comprising SEQ ID NOS. 341 to 348;
with the proviso that the polypeptide is none of wild-type of any one of IHF alpha, IHF beta or SEQ ID NO. 6 to 11, 28, 29, or 42 through 100 is described.

An isolated or recombinant polynucleotide or polypeptide of the group of:
a polypeptide consisting essentially of SEQ ID NO. 12 and 13;
a polypeptide consisting essentially of SEQ ID NO. 14 and 15;
a polypeptide consisting essentially of SEQ ID NO. 16 and 17;
a polypeptide consisting essentially of SEQ ID NO. 18 and 19;
a polypeptide consisting essentially of SEQ ID NO. 20 and 21;
a polypeptide consisting essentially of SEQ ID NO. 23 and 24;
a polypeptide consisting essentially of SEQ ID NO. 25 and 26;
a polypeptide consisting essentially of SEQ ID NO. 30 and 31;
a polypeptide consisting essentially of SEQ ID NO. 32 and 33;
a polypeptide consisting essentially of SEQ ID NO. 34 and 35;
a polypeptide consisting essentially of SEQ ID NO. 337 and 338; or
a polypeptide consisting essentially of SEQ ID NO. 339 and 340;
a polynucleotide or polypeptide consisting essentially of any one of SEQ ID NOS . 341 to 348;
with the proviso that the polypeptide is none of wild-type of any one of IHF alpha, IHF beta or SEQ ID NO. 6 to 11, 28, 29, or 42 through 100 is described.

Also described are isolated or recombinant polypeptides comprising, or alternatively consisting essentially of or yet further consisting of, two or more, or three or more or four or more, or multiples of the above-identified isolated polypeptides, including fragments and equivalents thereof. Examples of such include isolated polypeptides comprising SEQ ID NO. 1 through 4 and/or 12 through 29, and/or 30 through 33, and/or 30 through 35 e.g., SEQ ID NO. 1 and 2, or alternatively 1 and 3 or alternatively 1 and 4, or alternatively 2 and 3, or alternatively SEQ ID NO. 1, 2 and 3 or alternatively, 2, 3 and 4, or alternatively 1, 3 and 4 or equivalent polypeptides, examples of which are shown in Table 2 and specifically the Arm fragment identified in Table 2 or an equivalent thereof, or a polypeptide of SEQ ID NOs.: 337 to 340, or an equivalent thereof. The polypeptides can be in any orientation, e.g., SEQ ID NO. 1, 2, and 3 or SEQ ID NO. 3, 2 and 1 or alternatively SEQ ID NO. 2, 1 and 3, or alternatively, 3, 1 and 2, or alternatively 11 and 12, or alternatively 1 and 12, or alternatively 2 and 12, or alternatively, 1 and 12, or alternatively 2 and 13, or alternatively 12, 16 and 1, or alternatively 1, 16 and 12.

An isolated or recombinant polypeptide comprising SEQ ID NO. 1 or 2 and 3 or 4 or a polypeptide or recombinant polypeptide comprising, or alternatively consisting essentially of, or yet further consisting of an amino acid corresponding to fragments of a DNABII protein such as the .beta.-3 and/or α-3 fragments of a Haemophilus influenzae IHF-α or IHF.beta. microorganism, non-limiting examples of which include SEQ ID NO. 12 through 27, or a fragment or an equivalent of each of the polypeptides, examples of which are shown in Tables 2 and 3 is described. Isolated wildtype polypeptides are specifically excluded, e.g. that the polypeptide is none of SEQ ID NO. 6 through 11 or a wildtype sequence identified in Table 1. In this case, SEQ ID NO. 1 or 2 or a polypeptide comprising, or alternatively consisting essentially of, or yet further consisting of an amino acid corresponding to the .beta.-3 and/or α-3 fragments of an IHF.alpha. or IHF.beta. microorganism, non-limiting examples of which include SEQ ID NO. 12 through 27 and 30 through 33 or an equivalent of each thereof is located upstream or amino terminus from SEQ ID NO. 3 or 4 or a fragment or an equivalent thereof. The isolated polypeptide comprises SEQ ID NO. 3 or 4 or a polypeptide comprising, or alternatively consisting essentially of, or yet further consisting of an amino acid corresponding to the .beta.-3 and/or .alpha.-3 fragments of an IHF.alpha. or IHF.beta. microorganism, non-limiting examples of which include SEQ ID NO. 12 through 27, or an equivalent thereof located upstream or amino terminus to SEQ ID. NO. 1 or 2 or an equivalent thereof.

A peptide linker can be added to the N-terminus or C-terminus of the polypeptide, fragment or equivalent thereof. The linker joins the polypeptides, e.g., SEQ ID NO. 1 to 4, 28, 29, 34, or 35 or 30 to 33, 34, or 35 or a polypeptide comprising, or alternatively consisting essentially of, or yet further consisting of an amino acid corresponding to the .beta.-3 and/or .alpha.-3 fragments of a Haemophilus influenza IHF.alpha. or IHF.beta. microorganism, non-limiting examples of which include SEQ ID NO. 12 through 27 or an equivalent of each thereof. A "linker" or "peptide linker" refers to a peptide sequence linked to either the N-terminus or the C-terminus of a polypeptide sequence. In one aspect, the linker is from about 1 to about 20 amino acid residues long or alternatively 2 to about 10, about 3 to about 5 amino acid residues long. An example of a peptide tinker is Gly-Pro-Ser-Leu-Lys-Leu (SEQ ID NO: 37).

Further described is a fragment or an equivalent of the isolated or recombinant polypeptide of any one of polypeptides identified above as well as an isolated or recombinant polypeptide comprising, or alternatively consisting essentially of, or yet further consisting of, two or more of the isolated or recombinant polypeptides identified above.

Yet further described is a polynucleotide that interferes with the binding of the microbial DNA with a polypeptide or fragment or equivalent thereof, e.g., SEQ ID 36, or a four-way junction polynucleotide resembling a Holliday junction, a 3 way junction polynucleotide resembling a replication fork, a polynucleotide that has inherent flexibility or bent polynucleotide; an isolated or recombinant polynucleotide encoding a polypeptide described above or an antibody or fragment thereof, which can be operatively linked to regulatory elements necessary for the expression and/or replication of the polynucleotide. The polynucleotide can be contained within a vector.

Also described is an isolated host cell comprising, or alternatively consisting essentially of, or yet further consisting of an isolated or recombinant polypeptide described above, a four-way junction polynucleotide resembling a Holliday junction, a 3 way junction polynucleotide resembling a replication fork, a polynucleotide that has inherent flexibility or bent polynucleotide; an isolated or recombinant polynucleotide as described above, or a vector as described above.

Further described is a fragment or an equivalent of the isolated or recombinant polypeptide of any one of polypeptides identified above as well as an isolated or recombinant polypeptide comprising, or alternatively consisting essentially of, or yet further consisting of, two or more of the isolated or recombinant polypeptides identified above.

The methods are performed with an antibody or antigen binding fragment that specifically recognizes and binds the isolated or recombinant polypeptide as described above, including a fragment or an equivalent of the polypeptide. Non-limiting examples of antibodies include a polyclonal antibody, a monoclonal antibody, a humanized antibody, a human antibody, an antibody derivative, a veneered antibody, a diabody, a chimeric antibody, an antibody derivative, a recombinant human antibody, or an antibody fragment. In a particular aspect, the antibody is a monoclonal antibody. Yet further provided is a hybridoma cell line that produces the monoclonal antibody.

The antibodies, polynucleotides, polypeptides, vectors or host cells can further comprise a detectable label or a carrier, such as a pharmaceutically acceptable carrier.

Compositions comprising a carrier and one or more of an isolated or recombinant peptide of the invention, an isolated or recombinant nucleotide of the invention, a vector of the invention, an isolated host cell of the invention, or an antibody of the invention are also provided. The carriers can be one or more of a solid support, a medical device like a stent or dental implant, or a liquid such as a pharmaceutically acceptable carrier. The compositions can further comprise an adjuvant, an antimicrobial or an antigenic peptide.

The compositions can further comprise additional biologically active agents. A non-limiting example of such is a antimicrobial agent such as other vaccine components (i.e., antigenic peptides) such as surface antigens, e.g. a Type IV Pilin protein (see Jurcisek and Bakaletz (2007) J. of Bacteriology 189(10):3868-3875).

A method for producing an antigenic peptide by growing or culturing a host cell comprising an isolated polynucleotide as described above under conditions that favor the expression of the polynucleotide is described. The polypeptide produced by this method can be isolating for further *in vitro* or *in vivo* use.

A kit is also described for diagnostic or therapeutic use comprising a composition as described above and instructions for use. A kit is also described to perform screens for new drugs and/or combination therapies as provided herein.

### SEQUENCE LISTING

SEQ. ID NO. 1
   A1-A2-A3-A4-A5-A6-A7-A8-A9
   wherein:
   A1 is V or I;
   A2 is any one of K, Q, E, A, V or Y;
   A3 is any one of K, L, I, V or F;
   A4 is any one of S, I, R or V;
   A5 is any one of G or S;
   A6 is F;
   A7 is G;
   A8 is any one of N or S or T or K; and
   A9 is F.
SEQ. ID NO. 2 is VKKSGFGNF
SEQ ID NO. 3 is B1-B2-B3-B4-B5-B5-B6-B7
   wherein:
   B1 is absent or any one of G or K;
   B2 is absent or any one of R, I or K;
   B3 is N or V;
   B4 is P or I;
   B5 is any one of K, Q, S or G;
   B6 is any one of T, K or S; and
   B7 is any one of G, K, Q or D.
SEQ. ID NO. 4 is NP(K/Q)TG
SEQ. ID NO. 5 GRNP(K/Q)TG
SEQ. ID NO. 6 Full Length Wild type (wt) 86-028NP *Haemophilus influenzae* IhfA; Genbank accession No.: AAX88425.1, last accessed March 21, 2011: 
MATITKLDIIEYLSDKYHLSK
QDTKNVVENFLEEIRLSLESGQDVKLSGFGNFELRDKSSRPGRNPKTGDVVPVSARR
VVTFKPGQKLRARVEKTK
SEQ. ID NO. 7 Full Length wt 86-028NP *Haemophilus influenzae* HU, Genbank accession No.: YP_248142.1, last accessed March 21, 2011: MRFVTIFINHAFNSSQVRLSFAQFLR QIRKDTFKESNFLFNRRYKFMNKTDLIDAIANAAELNKKQAKAALEATLDAITASLK EGEPVQLIGFGTFKVNERAARTGRNPQTGAEIQIAASKVPAFVSGKALKDAIK
SEQ. ID NO. 8 Full Length wt R2846 *Haemophilus influenzae* IhfA, Genbank accession No.: ADO96375, last accessed March 21, 2011: 
MATITKLDIIEYLSDKYHLSKQDTKNVVENFL
EEIRLSLESGQDVKLSGFGNFELRDKSSRPGRNPKTGDVVPVSARRVVTFKPGQKLR
ARVEKTK
SDQ. ID NO. 9 Full Length wt Rd *Haemophilus influenzae* IhfA; Genbank accession No.: AAC22959.1, last accessed March 21, 2011: MATITKLDIIEYLSDKYHLSKQDTK NVVENFLEEIRLSLESGQDVKLSGFGNFELRDKSSRPGRNPKTGDVVPVSARRVVTF KPGQKLRARVEKTK;
SEQ. ID NO. 10 Full Length wt *E. coli* K12 IhfA; Genbank accession No.: AAC74782.1, last accessed March 21, 2011: MALTKAEMSEYLFDKLGLSKRDAKELVELFFE EIRRALENGEQVKLSGFGNFDLRDKNQRPGRNPKTGEDIPITARRVVT FRPGQKLKSRVENASPKDE; DNA Genbank No. NC_000913
SEQ. ID NO. 11 Full Length wt *P. aeruginosa* PA 01 IhfA; Genbank accession No.: AAG06126.1, last accessed March 21, 2011: MGALTKAEIAERLYEELGLNKREA KELVELFFEEIRQALEHNEQVKLSGFGNFDLRDKRQRPGRNPKTGEEIPITARRVVTF RPGQKLKARVEAYAGTKS
SEQ. ID NOS. 12 and 13: β-3 and α-3 portions of (IHFβ) SEQ ID NO. 12: TFRPGQ and
SEQ ID NO. 13: KLKSRVENASPKDE
SEQ ID NOS. 14 and 15: β-3 and α-3 portions of (IHFβ) SEQ ID NO. 14: HFKPGK and SEQ ID NO. 15: ELRDRANIYG
SEQ ID NOS. 16 and 17: β-3 and α-3 portions of SEQ ID NOS. 6, SEQ ID NOS. 16: TFKPGQ and SEQ ID NO. 17: KLRARVEKTK
SEQ ID NOS. 18 and 19: β-3 and α-3 portions of 2019 *Haemophilus influenzae* IhfA, SEQ ID NO. 18: TFKPGQ and SEQ ID NO. 19: KLRARVENTK
SEQ ID NOS. 20 and 21: β-3 and α-3 portions of SEQ ID NO. 8, SEQ ID NO. 20: TFKPGQ and SEQ ID NO. 21: KLRARVEKTK
SEQ ID NOS. 22 and 23: β-3 and α-3 portions of SEQ ID NO. 9, SEQ ID NO. 22: TFKPGQ and SEQ ID NO. 23: KLRARVEKTK
SEQ ID NOS. 24 and 25: β-3 and α-3 portions of SEQ ID NO. 10, SEQ ID NO. 24: TFRPGQ and SEQ ID NO. 25: KLKSRVENASPKDE
SEQ ID NOS. 26 and 27: β-3 and α-3 portions of SEQ ID NO. 11, SEQ ID NO. 26: TFRPGQ and SEQ ID NO. 27: KLKARVEAYAGTKS
SEQ ID NO. 28: E. coli hupA, Genbank accession No.: AP_003818, Last accessed March 21, 2011: MNKTQLIDVIAEKAELSKTQAKAALESTLAAITESLKEGDAVQLVGFGTFK VNHRAERTGRNPQTGKEIKIAAANVPAFVSGKALKDAVK
SEQ ID NO. 29: E. coli hupB, Genbank accession No.: AP_001090.1, Last accessed March 21, 2011: MNKSQLIDKIAAGADISKAAAGRALDAIIASVTESLKEGDDVALVGFG TFAVKERAARTGRNPQTGKEITIAAAKVPSFRAGKALKDAVN
SEQ ID NOS. 30 and 31: β-3 and α-3 portions of SEQ ID NO. 28, SEQ ID NO. 30: AFVSGK and SEQ ID NO. 31: ALKDAVK
SEQ ID NOS. 32 and 33: β-3 and α-3 portions of SEQ ID NO. 29, SEQ ID NO. 32: SFRAGK and SEQ ID NO. 33: ALKDAVN
SEQ. ID NO. 34: C-terminal 20 amino acids of IHF α: TFRPGQKLKSRVENASPKDE
SEQ. ID NO. 35: C-terminal 20 amino acids of IHF β: KYVPHFKPGKELRDRANIYG
SEQ. ID NO. 36: DNABII binding consensus sequence: WATCAANNNNTTR wherein W is A or T, N is any base and R is a purine
SEQ. ID NO. 337: E. coli IHFalpha: GRNPKTGEDIPI
SEQ. ID NO. 338: E. coli IHFbeta: GRNPKTGDKVEL
SEQ. ID NO. 339: E. coli HUalpha: GRNPQTGKEIKI
SEQ. ID NO. 340: E. coli HUbeta: GRNPQTGKEITI

### DESCRIPTION OF TABLES

Table 1 is a non-limited summary of DNA binding proteins produced by gram(+) and gram (-) bacteria that can be used in the methods provided herein.

Table 2 is a sequence alignment of relevant portions of the DNA binding proteins of various embodiments of this invention. Bold letters indicate an exact match to consensus, light gray lettering indicates a conservative amino acid change, and lightly or darkly shaded sequences are highly conserved across species. Gray shaded undefined sequences at the amino and/or carboxy-terminal are undefined amino acids that do not share consenus sequences.. Table 2 is based on information previously published in Obeto et al. (1994) Biochimie 76:901-908. The fragment "ARM" is denoted at the bottom of the table and polypeptide fragment comprising, or consisting essentially of, or yet further consisting of these fragments or their equivalents have biological activity as noted herein.

Table 3 is a comparison of the 16 amino acid peptide motif to Liu et al. (2008) Cell Microbiol. 10(1):262-276.

Table 4 is a listing of α, β, and C-terminal portions of DNABII proteins from the indicated organism.

### BRIEF DESCRIPTION OF THE DRAWINGS

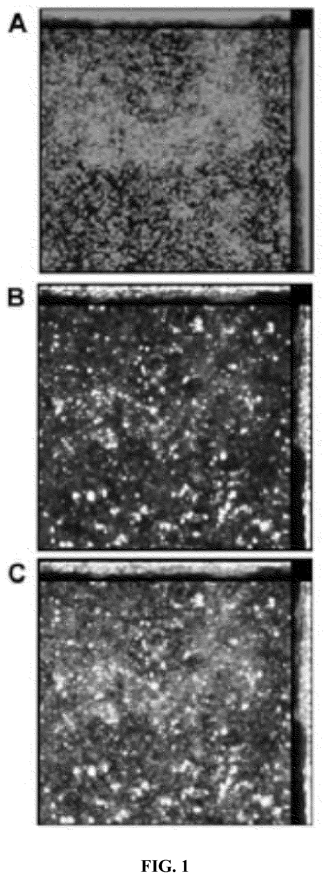

**FIGS. 1A-1C** show presence of both eDNA and IHF within a biofilm formed by *B. cenocepacia.* **FIG. 1A** - Unfixed twenty-four hr biofilm formed by *B. cenocepacia* stained with FilmTracer FM 1-43. **FIG. 1B** - unfixed 24 hr biofilm formed by *B. cenocepacia* and immunolabeled with monoclonal antiserum directed against dsDNA (white color in image). **FIG. 1C** - unfixed 24 hr. biofilm formed by *B. cenocepacia* labeled with both antiserum directed against dsDNA and rabbit anti-IHF serum to show the presence of IHF as well as dsDNA (white color) within the biofilm. Note robust biofilm formed by *B. cenocepacia* in FIG. 1A. The presence of eDNA that is particularly dense at the bottom of the biofilm can be seen in FIG. 1B. The 24-hr biofilm formed by *B. cenocepacia* is also rich in IHF as shown by the abundance in FIG. 1C. All images captured with a 63X objective.

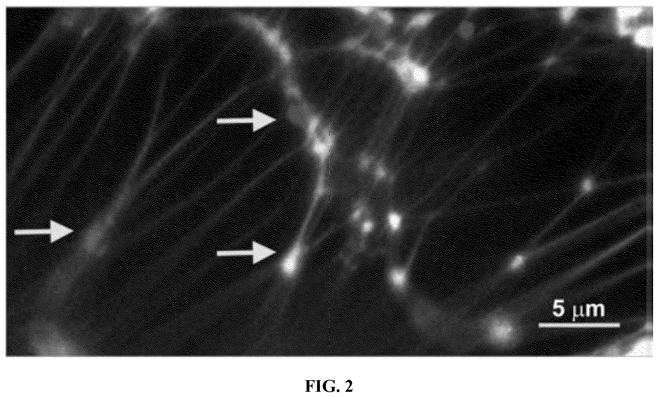

**FIG. 2** shows labeling of IHF and eDNA within sputum collected from a CF patient culture-positive for *B. cenocepacia.* Immunolabeled light micrograph demonstrates heavy labeling of both eDNA (white strands) and the DNABII protein IHF (punctate labeling; see arrows) within a sputum sample recovered from a CF patient infected with *B. cenocepacia.* As previously shown with nontypeable *Haemophilus influenzae*-formed biofilms, in those formed by *B. cenocepacia*, junctions where strands of bacterial eDNA cross label strongly for the presence of IHF, suggesting their role in maintaining the structural scaffolding of these biofilms. Scale bar equals 5 µm.

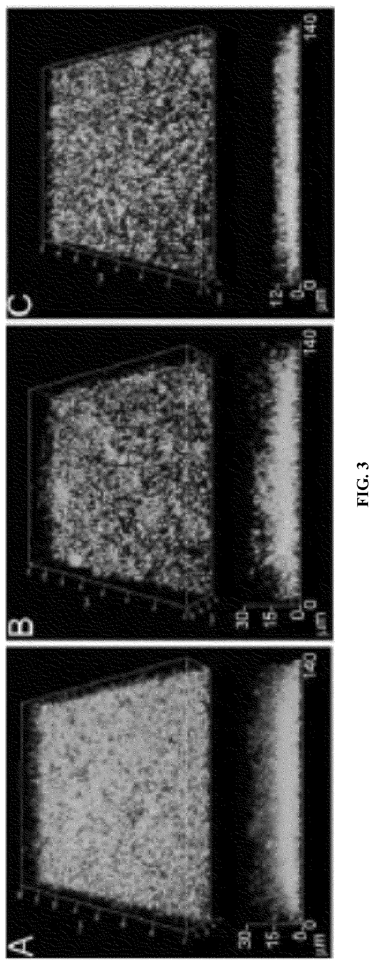

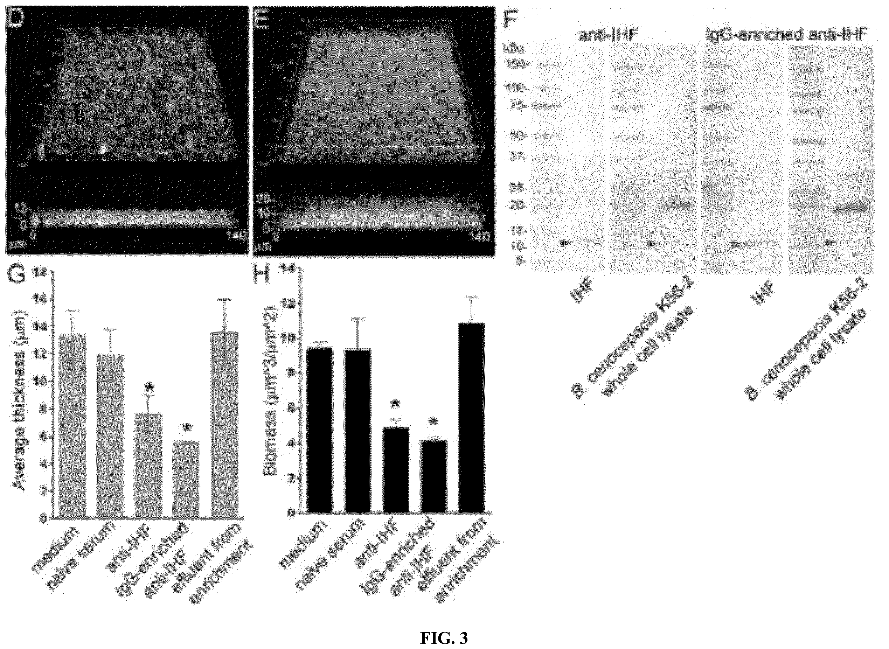

**FIGS. 3A-3H** show disruption of pre-formed *B. cenocepacia* biofilms by incubation with antiserum directed against IHF. *B. cenocepacia* biofilms after 24 hr. growth in a chamber slide, then treated for 16 hrs with: **FIG. 3A****-** sterile medium. **FIG. 3B** - naive rabbit serum. **FIG. 3C** -rabbit antiserum directed against isolated IHF. **FIG. 3D** -IgG enriched anti-IHF. **FIG. 3E** -serum effluent from enrichment column. **FIG. 3F** - Western blot showing recognition of antibody directed against IHF as well as IgG-enriched anti-IHF to purified IHF and IHF within *B. cenocepacia* whole cell lysate. Arrows indicate recognition of the monomer form of IHF in each blot. Moreover, each serum fraction recognized the di- and tri-meric forms of IHF within whole cell lysates of *B. cenocepacia* strain K56-2. **FIG. 3G** - plot of changes in average biofilm thickness ± SEM following each of the indicated treatments. **FIG. 3H** - plot of changes in biofilm biomass ± SEM following each of the indicated treatments. Note that statistically significant disruption of pre-formed *B. cenocepacia* biofilms is mediated only by rabbit anti-IHF serum as well as a fraction of that serum that was enriched for IgG when compared to treatment with sterile medium, naive rabbit serum or serum effluent from IgG enrichment column. Asterisks indicate statistical significance (p < 0.05) compared to sterile medium, naive serum and enrichment column effluent.

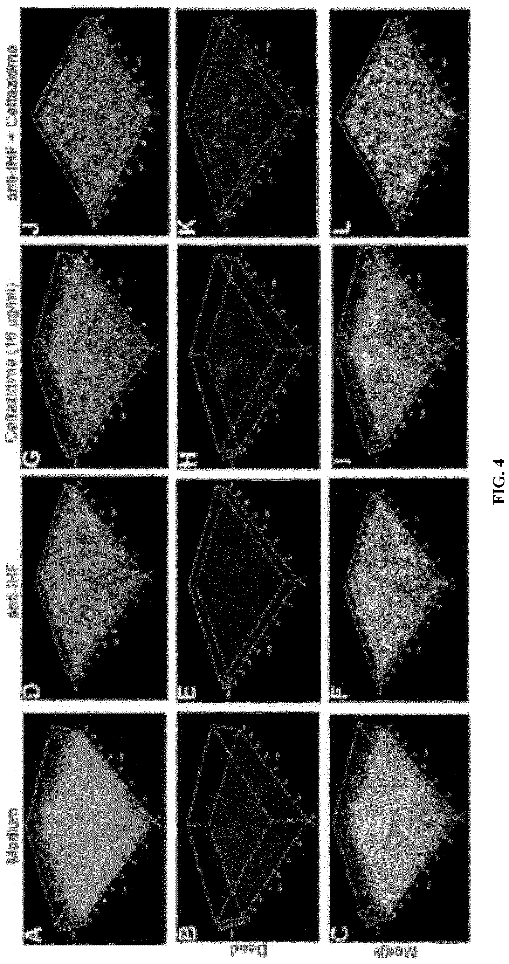

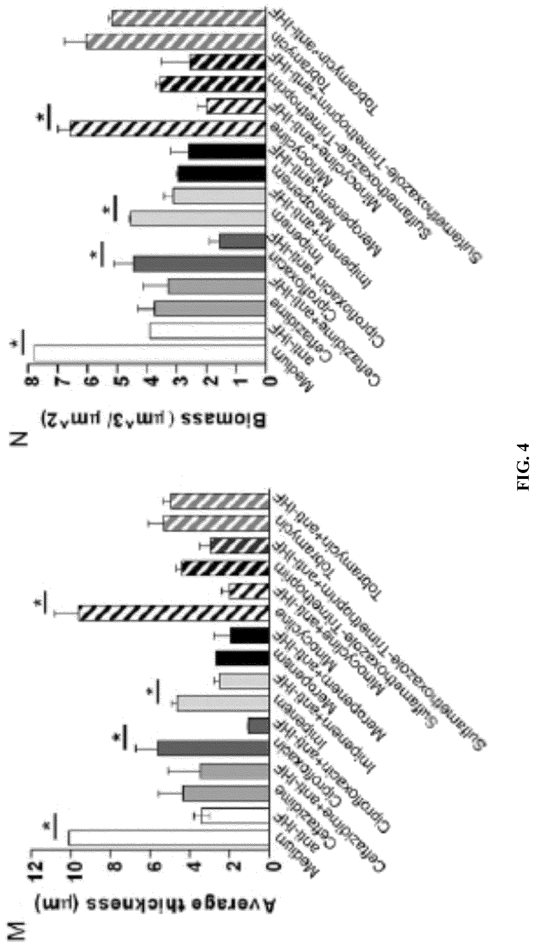

**FIGS. 4A-4N** show demonstration of the synergistic behavior of antibodies directed against IHF in combination with antimicrobials or antibiotics. **FIGS. 4A-4C** - An untreated *B. cenocepacia* biofilm. **FIGS. 4D-4F**- a *B. cenocepacia* biofilm after treatment with a 1:50 dilution of anti-IHF. **FIGS. 4G-4I** - a *B. cenocepacia* biofilm after treatment with the MIC of ceftazidime (16 µg/ml). **FIGS. 4J-4L** - a *B. cenocepacia* biofilm after treatment with a combination of anti-IHF plus ceftazidime at the noted MIC. Note marked reduction of biofilm height and notably increased killing of *B. cenocepacia* when treated with both anti-IHF and ceftazidime compared to treatment with antibiotic alone (indicated in second row of images; compare Panels H and K). **FIG. 4M** -average thickness of biofilms after treatment with antibiotic or antibiotic plus anti-IHF ± SEM. **FIG. 4N** - Biofilm biomass following incubation with antibiotic or antibiotic plus anti-IHF ± SEM. Asterisks indicate statistical significance between designated pairs (*p*< 0.05). Note significant reduction in both average biofilm thickness and biomass as mediated by a combination of anti-IHF serum plus ceftazidime, ciprofloxacin, imipenem and minocycline.

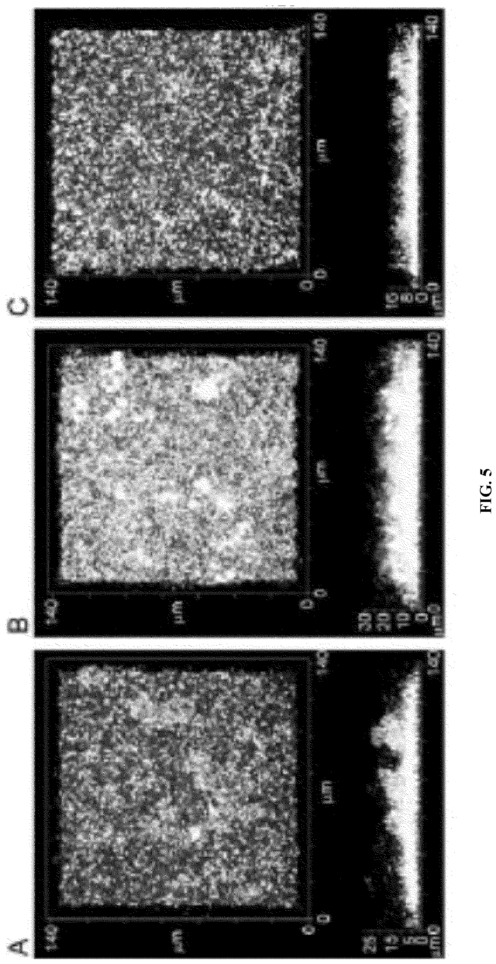

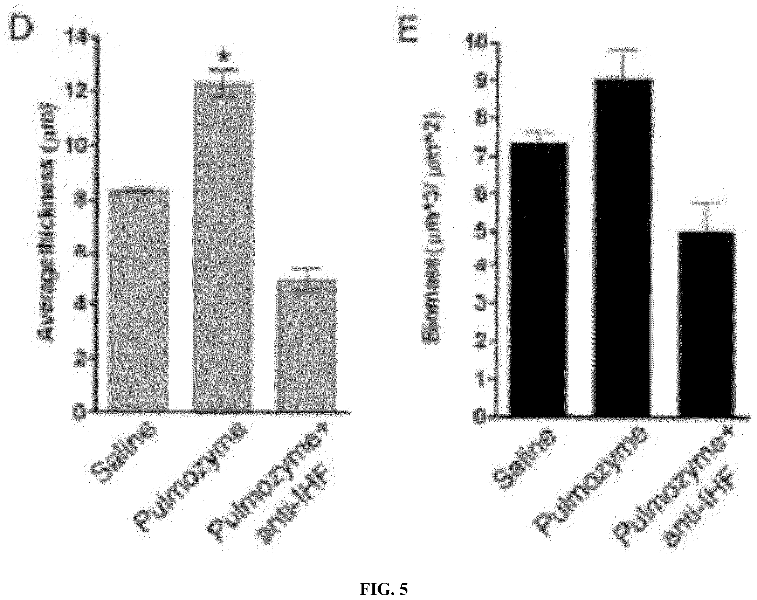

**FIGS. 5A-5E** show induction of a more robust *B. cenocepacia* biofilm following exposure to Pulmozyme (DNase). **FIG. 5A** - Treatment of a 24 hr *B. cenocepacia* biofilm with saline diluent alone. **FIG. 5B** - Treatment of a 24 hr. *B. cenocepacia* biofilm with Pulmozyme (DNase) induced the formation of a markedly denser and thicker biofilm than that treated with saline diluent alone (compare panels A and B). **FIG. 5C** - treatment of a 24 hr *B. cenocepacia* biofilm with both Pulmozyme and anti-IHF. Biofilms were stained for viability and pseudocolored white (live cells) and dark (dead cells) and demonstrate minimal bacterial death upon any treatment. Mean relative biofilm thickness ± SD and biomass ± SEM are depicted graphically in **FIGS. 5D & 5E****.** Asterisk indicates significantly thicker biofilm after exposure to Pulmozyme compared to treatment with saline diluent (*p*< 0.05).

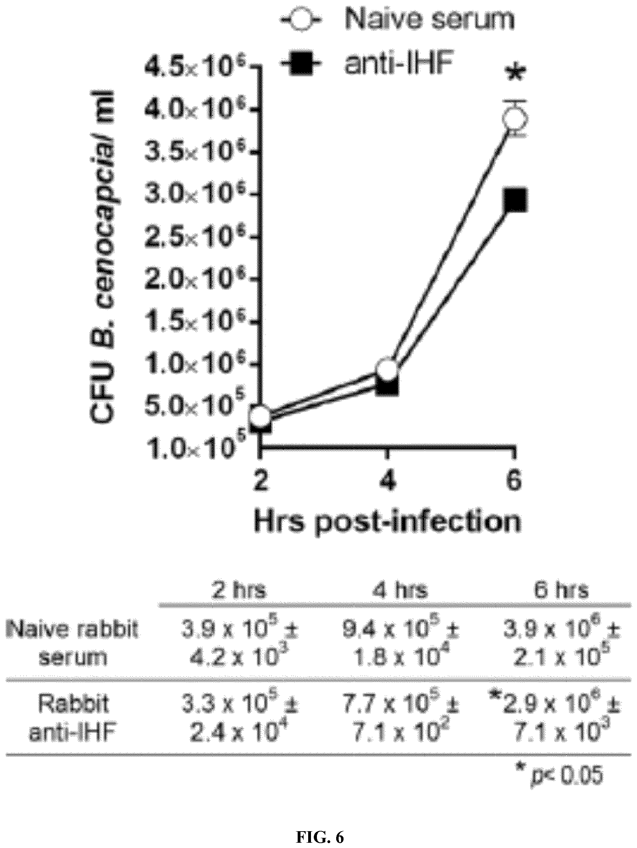

**FIG. 6** shows pre-treatment of *B. cenocepacia* with anti-IHF induced significantly reduced survival in murine CF macrophages. Significantly fewer *B. cenocepacia* were detected 6 hrs after pre- treatment of the bacteria with anti-IHF compared to naive serum (* *p* < 0.05). Bacterial CFU/ml for each time point ± SD are also shown.

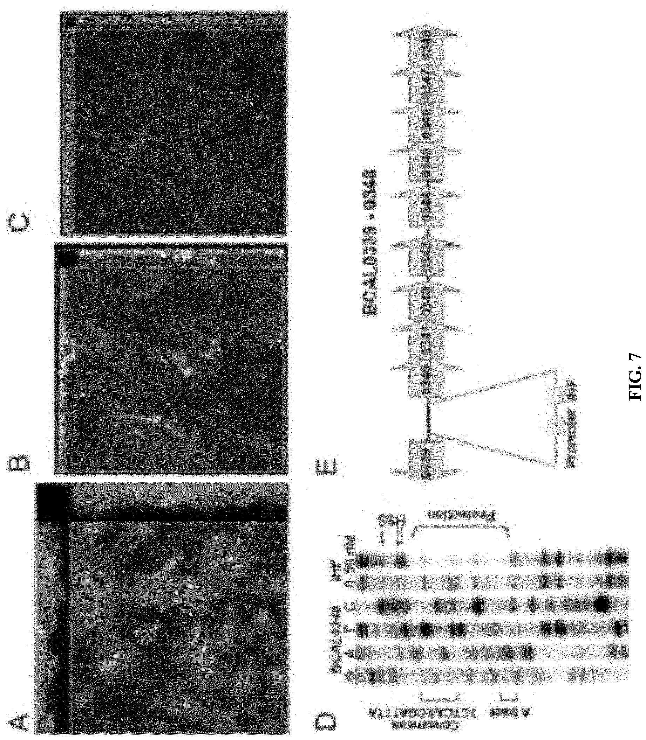

**FIGS. 7A-7E** show expression of a robust *B. cenocepacia* biofilm that incorporates IHF was dependent upon an active T6SS. **FIG. 7A** - Biofilm formed by the parental isolate (*B. cenocepacia* strain K56-2). **FIG. 7B** - biofilm formed by the type III secretion system mutant (strain JRL2) stained with propidium iodide. **FIG. 7C** - biofilm formed by the type VI secretion system mutant (strain DFA2) stained with propidium iodide. All biofilms were labeled for the presence of a DNABII protein (IHF). Whereas biofilms formed by either secretion system mutant were notably less robust than that formed by the parental isolate, note marked reduction in labeling of biofilm formed by the T6SS mutant (see FIG. 7C), which suggested that the T6SS mutant was compromised in its ability to incorporate IHF into biofilms formed under these conditions. **FIG. 7D** - IHF bound DNase footprint of the intergenic space (386 bp) between BCAL0339 and BCAL0340, part of the T6SS gene cluster for *B. cenocepacia.* The IHF footprint covers the region from 25 bp to 52 bp upstream of the BACL0340 start codon while a putative promoter was found 75 bp to 104 bp upstream of the start codon (**FIG**. **7E**). HSS: hyper-sensitive site indicative of DNA bending. This observation suggested that IHF might self-regulate its own release as well as perhaps that of eDNA that is incorporated into the biofilm matrix.

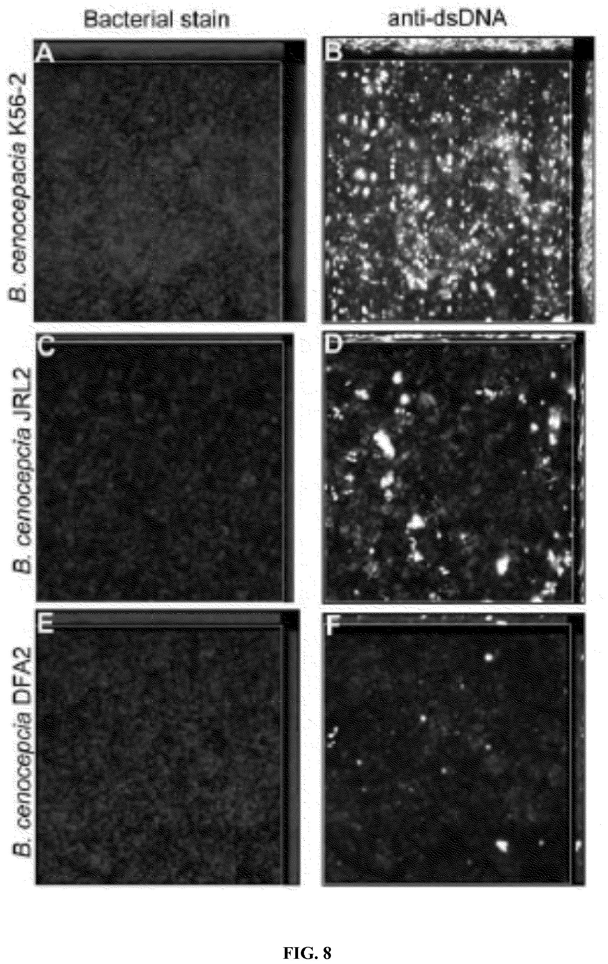

**FIGS. 8A-8F** show demonstration of relative eDNA content of biofilms formed in chamber slides by either the parental isolate K56-2, its T3SS mutant or its T6SS mutant. Biofilms formed by either the parental *B. cenocepacia* strain or its T3SS and T6SS mutants followed by staining with FilmTracer FM 1-43 can be seen in FIGS. 8A, 8C & 8E, respectively. Relative eDNA content of each unfixed biofilm can be ascertained via immunolabeling of each biofilm with a monoclonal antibody directed against dsDNA as in FIGS. 8B, 8D & 8F.
**FIG. 9A** is a map indicating the amino acid residues of IHF that interact with or bind to another IHF in an IHF-IHF dimmer (indicated by triangles at the upper level) or interact with or bind DNA (indicated by triangles at the lower level). The peptide is divided by the short vertical bars into regions containing 3 amino acids. **FIG. 9B** graphically depicts the interaction of microbial DNA with an IHF.

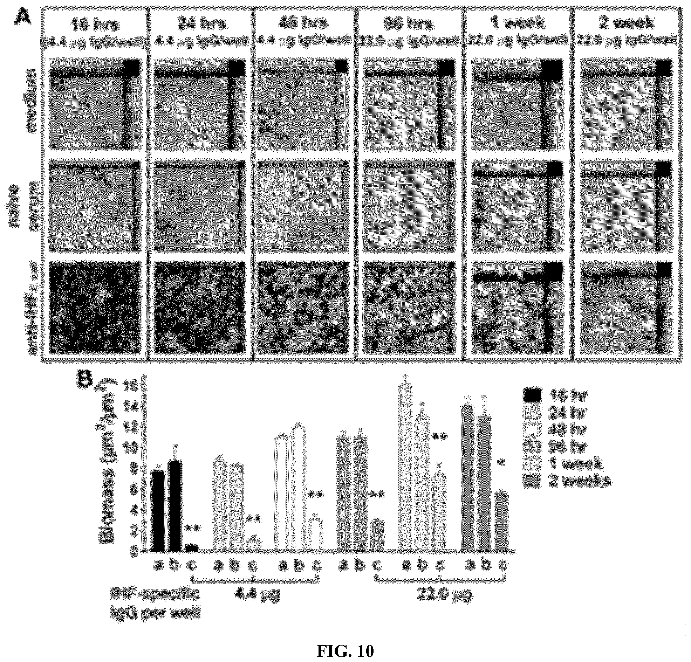

**FIGS. 10A-10B** show disruption of NTHI biofilms by anti-IHF_{*E*. *coli*}. (**A**) Representative images of NTHI biofilms and (**B**) calculated mean biomass. a, medium; b, naive serum; c, anti-IHF_{*E*. *coli*}. Anti-IHF_{*E*. *coli*} significantly disrupted NTHI biofilms established for 16 hr to 2 weeks, compared to naive serum or medium. Data are expressed as the mean ± SEM of three independent assays. **p*<0.05; ***p*<0.01 compared to respective naive serum treatment, one way ANOVA.

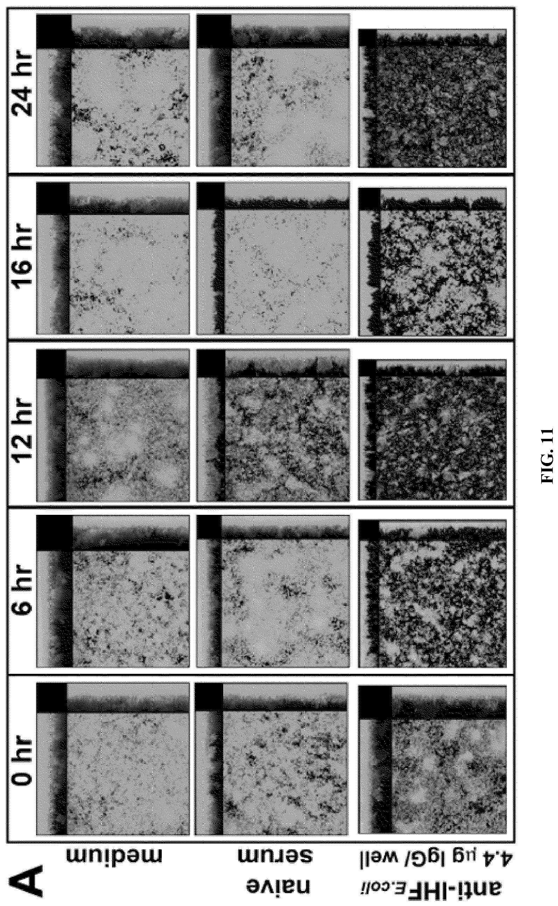

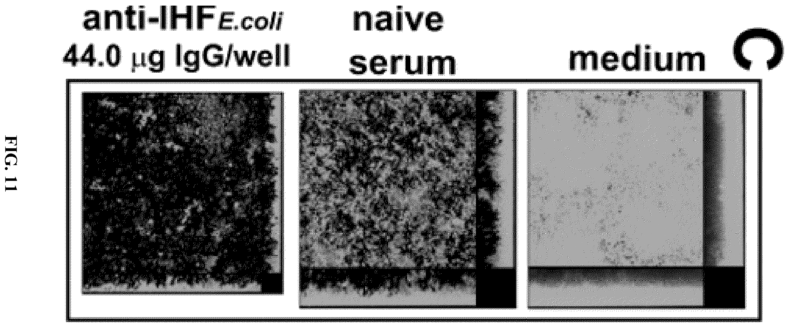

**FIGS. 11A-11C** shows kinetics of biofilm disruption by anti-IHF_{*E*. *coli*}. (**A**) Representative images of NTHI biofilms and (**B**) calculated mean biomass. a, medium; b, naive serum; c, anti-IHF_{*E*. *coli*}. Reduction in biomass mediated by anti-IHF_{*E*. *coli*} was maximal at 6 hr and sustained for 24 hr. (**C**) 24 hr biofilm treated with medium, a 1:5 dilution of naive serum or anti-IHF*_{E. coli}*. Treatment with a greater concentration of anti-IHF*_{E. coli}* eradicated the biofilm, leaving a monolayer of bacteria. Data are expressed as the mean ± SEM of three independent assays. **p*<0.01 compared to respective naive serum treatment, one way ANOVA.

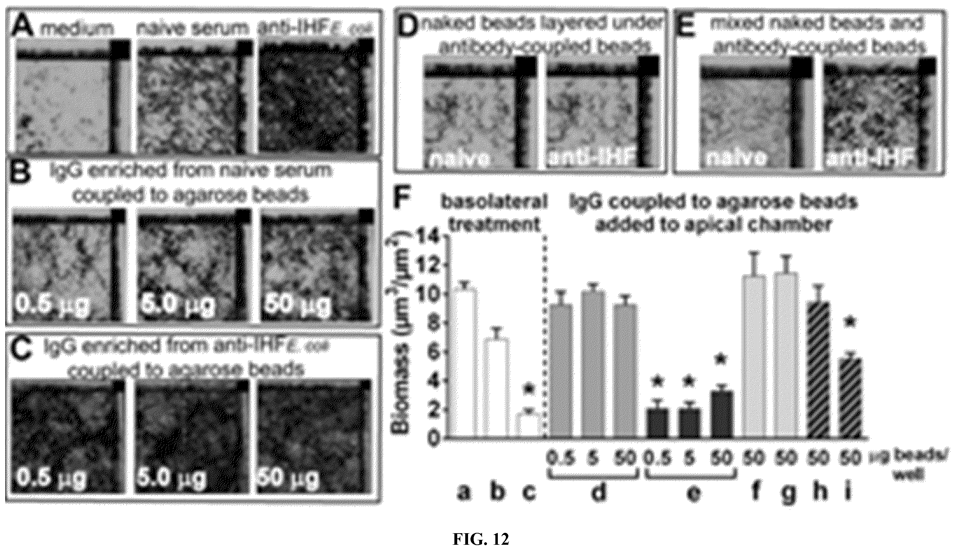

**FIGS. 12A-12F** show that direct contact between anti-IHF_{*E*. *coli*} and biofilm was not required to mediate disruption. (**A**) Representative images of biofilms treated basolaterally with medium or serum. (**B-C**) Biofilms treated apically by placement of antibody coupled to agarose beads into a transwell, (**D**) NTHI biofilms after naked beads were layered under antibody-coupled beads apically, (**E**) biofilms after mixing of naked and antibody-coupled beads. (**F**) Biomass values after incubation with: a, medium; b, naive serum; c, anti-IHF*_{E. coli}*; d, coupled IgG-enriched naive serum; e, coupled IgG-enriched anti-IHF*_{E. coli}*; f, naked beads layered under coupled IgG-enriched naive serum; g, naked beads layered under coupled IgG-enriched anti-IHF*_{E. coli}*; h, mixed naked and coupled IgG-enriched naive serum; i, mixed naked and coupled IgG-enriched anti-IHF*_{E. coli}*. Data are expressed as the mean + SEM of three independent assays. **p*<0.05 compared to respective naive serum or IgG-enriched naive serum conjugated to agarose beads treatment, one way ANOVA.

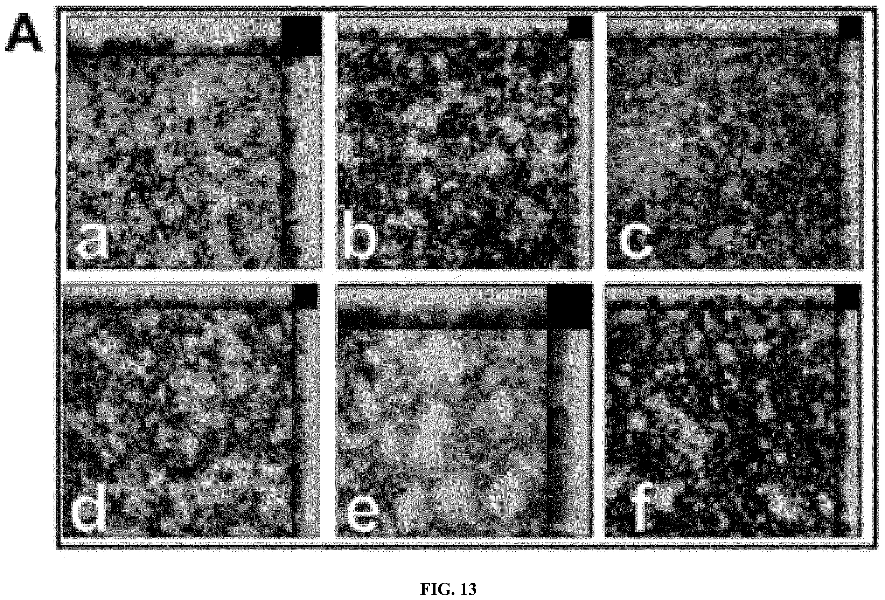

**FIGS. 13A-13B** show adsorption of anti-IHF-specific antibody. (**A**) Representative images of biofilms incubated with IHF-adsorbed serum. (**B**) Changes in biofilm biomass and mean thickness, a, naive serum; b, anti-IHF*_{E. coli}*. Anti-IHF*_{E. coli}* (4.4 µg) adsorbed with: c, 0 µg IHF_{*E*. *coli*}; d, 2.2 µg IHF_{*E*. *coli*}; e, 4.4 µg IHF_{*E*. *coli*}; f, 4.4 µg rsPilA. Adsorption of IHF-specific antibody counteracted biofilm disruption. Data are expressed as the mean ± SEM of three independent assays. **p*<0.05 compared to naive serum, one way ANOVA.

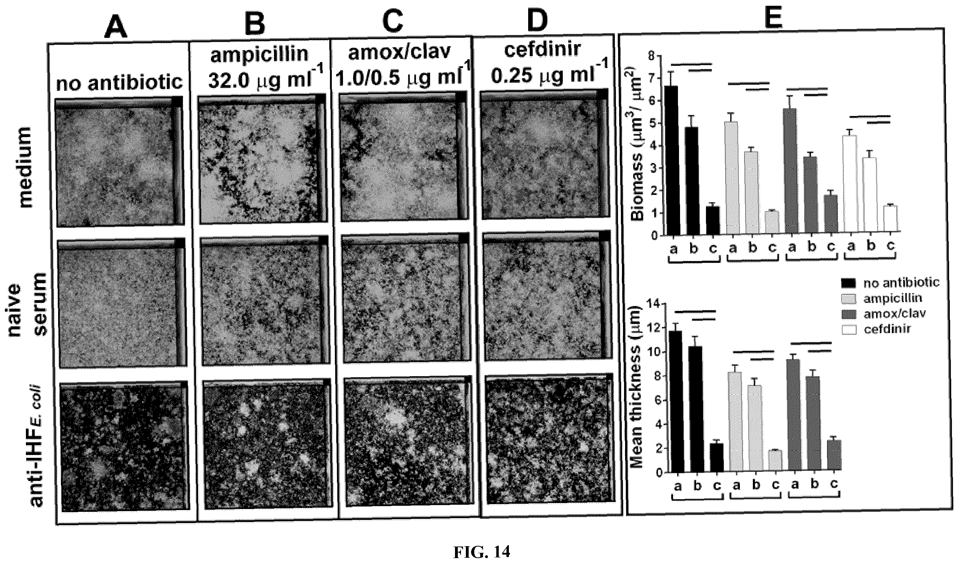

**FIGS. 14A-14E** show that anti-IHF*_{E. coli}* acted synergistically with antibiotics. (**A-D**) Representative images biofilms treated with medium, antibiotic at the MIC₉₀ or antiserum. (**E**) Biomass and mean thickness of treated biofilms. a, medium; b, naive serum; c, anti-IHF_{*E*. *coli*}. Incubation of NTHI biofilms with anti-IHF_{*E*. *coli*} plus antibiotic markedly altered biofilm architecture, significantly reduced biofilm biomass and mean thickness and negatively impacted viability (note yellow color of biofilms). Data are expressed as the mean ± SEM of three independent assays. Bars indicate *p*<0.05, one way ANOVA.

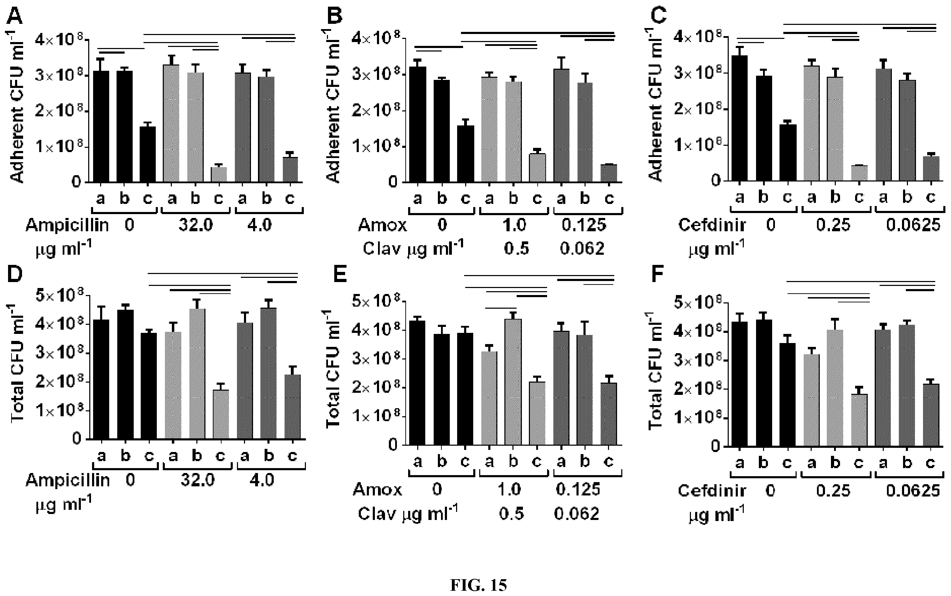

**FIGS. 15A-15F** shows that bacteria newly released from the biofilm by treatment with anti-IHF_{*E*. *coli*} showed enhanced sensitivity to antibiotics. Biofilms were incubated with ampicillin (**A&D**), amoxicillin-clavulanate (**B&E**), or cefdinir (**C&F**) in the absence or presence of a 1:50 dilution of anti-IHF_{*E*. *coli*} or naive serum. (**A-C**) CFU NTHI adherent within the biofilms. (**D-F**) Sum of the planktonic and adherent NTHI. a, medium alone; b, naive serum; c, anti-IHF*_{E. coli}*. Data are expressed as the mean ± SEM of three independent assays. Bars indicate *p*< 0.05, one way ANOVA.

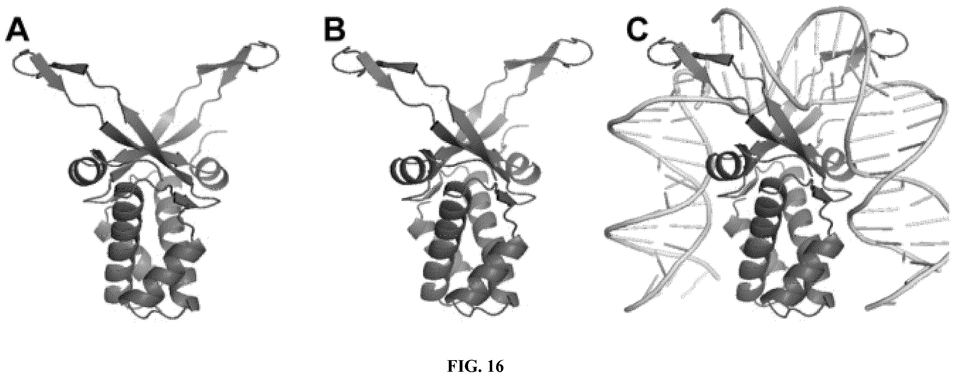

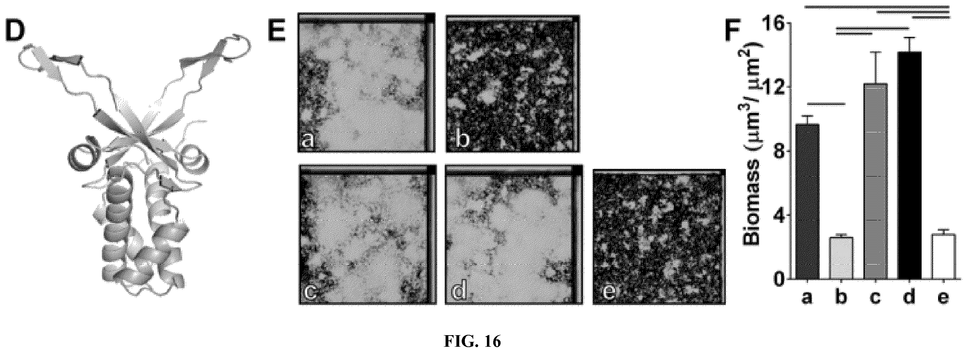

**FIGS. 16A-16F** show epitope mapping IHF_{NTHI} and design of a minimal IHF_{NTHI}-targeted peptide. 3-D model depicting reactivity of (**A**) chinchilla anti-IHF_{*E*. *coli*} and (**B**) chinchilla anti-IHF*_{E. coli}*- complexed to DNA to synthetic peptides representing IHF_{NTHI}. Regions with reactivity are indicated in grey, nonreactive regions in black. (**C**) 3-D model of DNA bound to IHF to show occlusion of tip-binding regions. (**D**) Localization of IhfA-3_{NTHI} (yellow) and IhfA-5 _{NTHI} (green) within IHF model. (**E**) Representative images and (**F**) mean biomass of biofilms after incubation with chinchilla serum. Data are expressed as the mean ± SEM of three independent assays. Bars indicate p< 0.05, one way ANOVA. a, naive serum; b, anti-IHF*_{E. coli}*; c, anti-IHF_{*E*. *coli*} complexed to DNA; d, anti-IhfA-3_{NTHI}; e, anti-IhfA-5_{NTHI}..

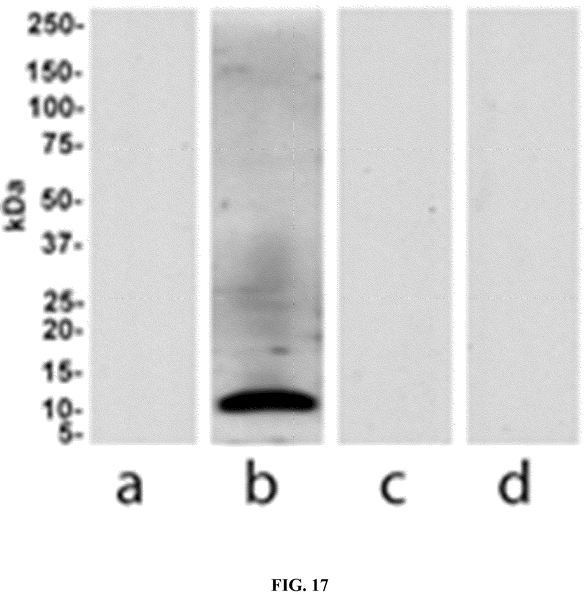

**FIG. 17** shows IgG-enriched anti-IHFE. coli bound to agarose beads and placed into the apical chamber of a transwell did not diffuse into the basolateral chamber as determined by Western blot against purified IHFE. coli. Medium from the basolateral chamber was collected 24 hr after treatment with: a, naive serum added basolaterally; b, anti-IHFE. coli added basolaterally; c, IgG-enriched naive serum tethered to agarose beads added apically; d, IgG-enriched anti-IHFE. coli tethered to agarose beads added apically.

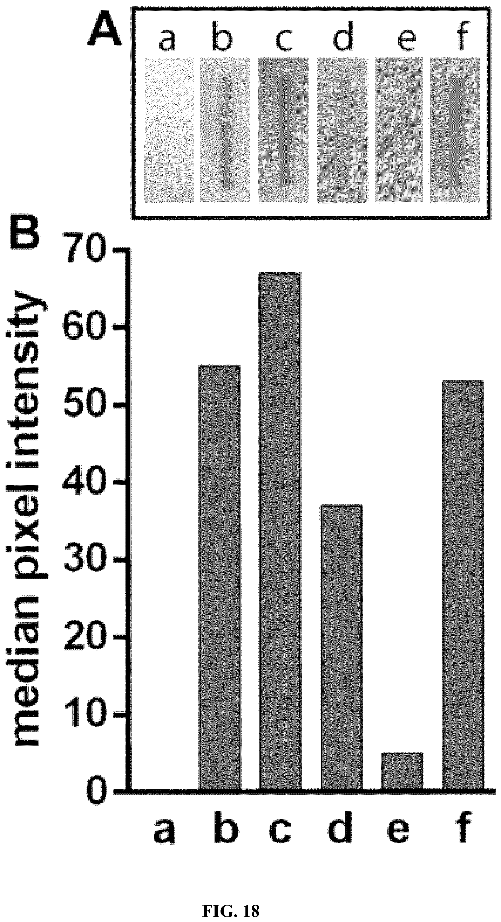

**FIGS. 18A-18B** show adsorption of anti-IHF antibodies. (**A**) Band intensity was reduced by incubation of anti-IHF*_{E. coli}* with purified IHF_{*E*. *coli*} shown by slot blot and (**B**) quantitated by densitometry. a, naive serum; b, anti-IHF_{*E*. *coli*}; c, anti-IHF_{*E*. *coli*} adsorbed with 0 µg IHF_{*E*. *coli*}; d, anti-IHF_{*E*. *coli*} adsorbed with 2.2 µg IHF_{*E*. *coli*}; e, anti-IHF_{*E*. *coli*} adsorbed with 4.4 µg IHF_{*E*. *coli*}; f, anti-IHF*_{E. coli}* adsorbed with 4.4 µg rsPilA.

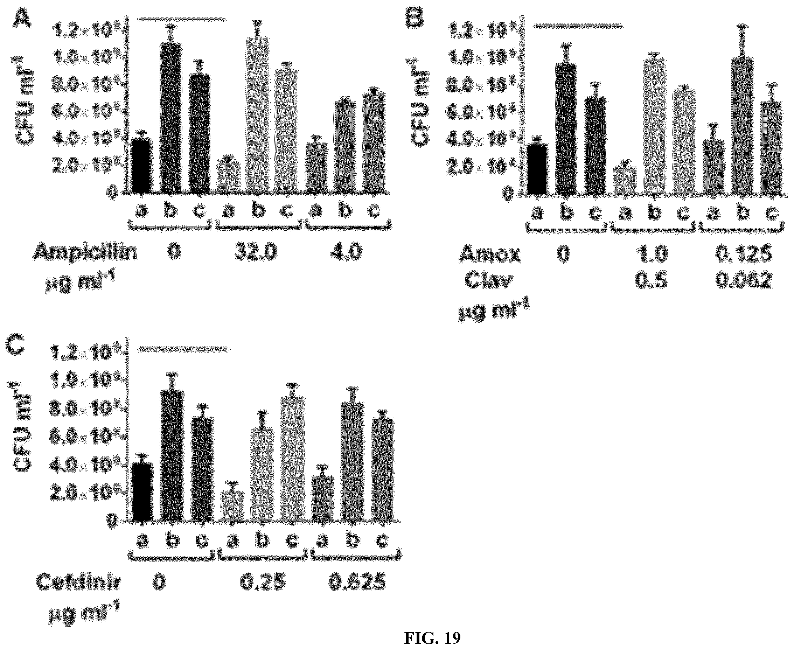

**FIGS. 19A-19C** show treatment with anti-IHF_{*E*. *coli*} did not increase the sensitivity of planktonic cultures of NTHI to antibiotics. (**A-C**) Broth cultures of NTHI were incubated with ampicillin, amoxicillin-clavulanate, or cefdinir in the absence or presence of anti-IHF*_{E. coli}* or naive serum. a, medium alone; b, naive serum; c, anti-IHF_{*E*. *coli*}. Data represent mean ± SEM of three independent assays. Bars indicate *p* < 0.05, one way ANOVA.

### DETAILED DESCRIPTION

### Definitions

Unless defined otherwise, all technical and scientific terms used herein have the same meanings as commonly understood by one of ordinary skill in the art to which this this invention belongs.

The scope of the invention is defined by the appended claims.

The practice of the present invention will employ, unless otherwise indicated, conventional techniques of tissue culture, immunology, molecular biology, microbiology, cell biology and recombinant DNA, which are within the skill of the art. See, e.g., Sambrook and Russell eds. (2001) Molecular Cloning: A Laboratory Manual, 3rd edition; the series Ausubel et al. eds. (2007) Current Protocols in Molecular Biology; the series Methods in Enzymology (Academic Press, Inc., N.Y.); MacPherson et al. (1991) PCR 1: A Practical Approach (IRL Press at Oxford University Press); MacPherson et al. (1995) PCR 2: A Practical Approach; Harlow and Lane eds. (1999) Antibodies, A Laboratory Manual; Freshney (2005) Culture of Animal Cells: A Manual of Basic Technique, 5th edition; Gait ed. (1984) Oligonucleotide Synthesis; U.S. Patent No. 4,683,195; Hames and Higgins eds. (1984) Nucleic Acid Hybridization; Anderson (1999) Nucleic Acid Hybridization; Hames and Higgins eds. (1984) Transcription and Translation; Immobilized Cells and Enzymes (IRL Press (1986)); Perbal (1984) A Practical Guide to Molecular Cloning; Miller and Calos eds. (1987) Gene Transfer Vectors for Mammalian Cells (Cold Spring Harbor Laboratory); Makrides ed. (2003) Gene Transfer and Expression in Mammalian Cells; Mayer and Walker eds. (1987) Immunochemical Methods in Cell and Molecular Biology (Academic Press, London); and Herzenberg et al. eds (1996) Weir's Handbook of Experimental Immunology.

All numerical designations, e.g., pH, temperature, time, concentration, and molecular weight, including ranges, are approximations which are varied (+) or (-) by increments of 1.0 or 0.1, as appropriate or alternatively by a variation of +/- 15 %, or alternatively 10% or alternatively 5% or alternatively 2%. It is to be understood, although not always explicitly stated, that all numerical designations are preceded by the term "about". It also is to be understood, although not always explicitly stated, that the reagents described herein are merely exemplary and that equivalents of such are known in the art.

As used in the specification and claims, the singular form "a", "an" and "the" include plural references unless the context clearly dictates otherwise. For example, the term "a polypeptide" includes a plurality of polypeptides, including mixtures thereof.

As used herein, the term "comprising" is intended to mean that the compositions and methods include the recited elements, but do not exclude others. "Consisting essentially of' when used to define compositions and methods, shall mean excluding other elements of any essential significance to the combination for the intended use. Thus, a composition consisting essentially of the elements as defined herein would not exclude trace contaminants from the isolation and purification method and pharmaceutically acceptable carriers, such as phosphate buffered saline, preservatives, and the like. "Consisting of' shall mean excluding more than trace elements of other ingredients and substantial method steps for administering the compositions of this invention.

A "biofilm" intends a thin layer of microorganisms that adhere to the surface of a structure, that may be organic or inorganic, together with the polymers such as DNA that they secrete. They are very resistant to microbiotics and antimicrobial agents. They live on gingival tissues, teeth and restorations, causing caries and periodontal disease, also known as periodontal plaque disease. They also cause chronic middle ear infections. Biofilms can also form on the surface of dental implants, stents, catheter lines and contact lenses. They grow on pacemakers, heart valve replacements, artificial joints and other surgical implants. The Centers for Disease Control) estimate that over 65% of nosocomial (hospital-acquired) infections are caused by biofilms. Fungal biofilms also frequently contaminate medical devices. They cause chronic vaginal infections and lead to life-threatening systemic infections in people with hobbled immune systems. Biofilms also are involved in numerous diseases. For instance, cystic fibrosis patients have *Pseudomonas* and *B. cenocepacia* infections that often result in antibiotic resistant biofilms.

The term "inhibiting, competing or titrating" intends a reduction or prevention in the formation of the DNA/protein matrix that is a component of a microbial biofilm.

A "DNA BII polypeptide or protein" intends a DNA binding protein or polypeptide that is composed of DNA-binding domains and thus have a specific or general affinity for microbial DNA. In one aspect, they bind DNA in the minor grove. A non-limiting example of a DNA BII protein is an integration host factor (IHF) protein. Other DNA binding proteins that may be associated with the biofilm include DPS (Genbank Accession No.: CAA49169), H-NS (Genbank Accession No.: CAA47740), Hfq (Genbank Accession No.: ACE63256), CbpA (Genbank Accession No.: BAA03950) and CbpB (Genbank Accession No.: NP_418813).

An "integration host factor" protein is a bacterial protein that is used by bacteriophages to incorporate their DNA into the host bacteria. They also bind extracellular microbial DNA.

"HMGB1 is an high mobility group box (HMGB) 1 protein that is reported to bind to and distort the minor groove of DNA and is an example of an interfering agent. Recombinant or isolated protein and polypeptide is commercially available from Atgenglobal, ProSpecBio, Protein1 and Abnova.

"HU" refers to a class of heterodimeric proteins typically associate with *E. coli.* HU proteins are known to bind DNA junctions. Related proteins have been isolated from other microorganisms. The complete amino acid sequence of *E. coli* HU was reported by Laine et al. (1980) Eur. J. Biochem. 103(3):447-481. Antibodies to the HU protein are commercially available from Abcam.

"HU" or "histone-like protein from *E.coli* strain U93" refers to a class of heterodimeric proteins typically associate with *E. coli.* HU proteins are known to bind DNA junctions. Related proteins have been isolated from other microorganisms. The complete amino acid sequence of *E. coli* HU was reported by Laine et al. (1980) Eur. J. Biochem, 103(3)447-481. Antibodies to the HU protein are commercially available from Abeam. The genes that encode the HU protein subunits in *E. coli* are hupA and hupB corresponding to SEQ ID Nos: 28 and 29, respectively. Homologs for these genes are found in other organisms, and peptides corresponding to these genes from other organisms can be found in Table 4.

"Microbial DNA" intends single or double stranded DNA from a microorganism that produces a biofilm.

"Inhibiting, preventing or breaking down" a biofilm intends the prophylactic or therapeutic reduction in the structure of a biofilm.

An "interfering agent" intends an agent that any one or more of competes, inhibits, prevents, titrates or occludes a DNA BII polypeptide such as IHF to a microbial DNA or also breaks down a microbial biofilm. It can be any one or more of a chemical or biological molecule. For example, IHF can specifically bind, bend or distorted DNA structures such as DNA containing four-way junctions, cis-platinum adducts, DNA loop or base bulges. Examples of such agents, without limitation, include (1) small molecules that inhibit the DNA-binding activity of IHF, (2) small molecules such as polyamines and spermine that compete with IHF in DNA binding, (3) polypeptides such as peptide fragments of IHF that compete with IHF in DNA binding, (4) antibodies or fragments thereof directed to IHF, or (5) a four-way or bent polynucleotides or other types of polynucleotides containing bent or distorted DNA structures that compete in IHF-binding. A "small molecule that inhibits the binding of an IHF to a nucleic acid" refers to (1) or (2) above and include those that bind DNA in the minor grove, i.e., minor groove binding molecules. A "four-way polynucleotide" intends a polynucleotide that contains a four-way junction, also known as the Holliday junction, between four strands of DNA.

A "bent polynucleotide" intends a double strand polynucleotide that contains a small loop on one strand which does not pair with the other strand. In some embodiments, the loop is from 1 base to about 20 bases long, or alternatively from 2 bases to about 15 bases long, or alternatively from about 3 bases to about 12 bases long, or alternatively from about 4 bases to about 10 bases long, or alternatively has about 4, 5, or 6, or 7, or 8, or 9, or 10 bases.

"Polypeptides that compete with IHF in DNA binding" intend proteins or peptides that occlude or compete with IHF in binding bent or distorted DNA structures but do not form a biofilm with the DNA. Examples, without limitation, include fragments of IHF that include one or more DNA binding domains of the IHF, or the biological equivalents thereof. DNA binding domains are shown in Figure 9.

A "subject" of diagnosis or treatment is a cell or an animal such as a mammal, or a human. Non-human animals subject to diagnosis or treatment and are those subject to infections or animal models, for example, simians, murines, such as, rats, mice, chinchilla, canine, such as dogs, leporids, such as rabbits, livestock, sport animals, and pets.

The term "protein", "peptide" and "polypeptide" are used interchangeably and in their broadest sense to refer to a compound of two or more subunit amino acids, amino acid analogs or peptidomimetics. The subunits may be linked by peptide bonds. In another embodiment, the subunit may be linked by other bonds, e.g., ester, ether, etc. A protein or peptide must contain at least two amino acids and no limitation is placed on the maximum number of amino acids which may comprise a protein's or peptide's sequence. As used herein the term "amino acid" refers to either natural and/or unnatural or synthetic amino acids, including glycine and both the D and L optical isomers, amino acid analogs and peptidomimetics.

A "c-terminal polypeptide" intends the c-terminal half of a polypeptide. As an example, for polypeptides containing 90 amino acids, the c-terminal polypeptide would comprise amino acids 46 through 90. In another aspect, the term intends the c-terminal 20 amino acids from the carboxy terminus.

The terms "polynucleotide" and "oligonucleotide" are used interchangeably and refer to a polymeric form of nucleotides of any length, either deoxyribonucleotides or ribonucleotides or analogs thereof. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, RNAi, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any embodiment of this invention that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for thymine when the polynucleotide is RNA. Thus, the term "polynucleotide sequence" is the alphabetical representation of a polynucleotide molecule. This alphabetical representation can be input into databases in a computer having a central processing unit and used for bioinformatics applications such as functional genomics and homology searching.

The term "isolated" or "recombinant" as used herein with respect to nucleic acids, such as DNA or RNA, refers to molecules separated from other DNAs or RNAs, respectively that are present in the natural source of the macromolecule as well as polypeptides. The term "isolated or recombinant nucleic acid" is meant to include nucleic acid fragments which are not naturally occurring as fragments and would not be found in the natural state. The term "isolated" is also used herein to refer to polynucleotides, polypeptides and proteins that are isolated from other cellular proteins and is meant to encompass both purified and recombinant polypeptides. In other embodiments, the term "isolated or recombinant" means separated from constituents, cellular and otherwise, in which the cell, tissue, polynucleotide, peptide, polypeptide, protein, antibody or fragment(s) thereof, which are normally associated in nature. For example, an isolated cell is a cell that is separated from tissue or cells of dissimilar phenotype or genotype. An isolated polynucleotide is separated from the 3' and 5' contiguous nucleotides with which it is normally associated in its native or natural environment, e.g., on the chromosome. As is apparent to those of skill in the art, a non-naturally occurring polynucleotide, peptide, polypeptide, protein, antibody or fragment(s) thereof, does not require "isolation" to distinguish it from its naturally occurring counterpart.

It is to be inferred without explicit recitation and unless otherwise intended, that when the present invention relates to a polypeptide, protein, polynucleotide or antibody, an equivalent or a biologically equivalent of such is intended within the scope of this invention. As used herein, the term "biological equivalent thereof' is intended to be synonymous with "equivalent thereof' when referring to a reference protein, antibody, polypeptide or nucleic acid, intends those having minimal homology or sequence identity while still maintaining desired structure or functionality. Unless specifically recited herein, it is contemplated that any polynucleotide, polypeptide or protein mentioned herein also includes equivalents thereof. For example, an equivalent intends at least about 60%, or alternatively at least about 65%, or alternatively at least about 70%, or alternatively at least about 75%, or alternatively at least about 80 %, or alternatively about least about 85 %, or alternatively at least about 90 %, or alternatively at least about 95 %, or alternatively 98 % percent homology or sequence identity and exhibits substantially equivalent biological activity to the reference protein, polypeptide or nucleic acid. Examples of biologically equivalent polypeptides are provided in Table 2, and the Arm fragments identified in Table 2, which identify conservative amino acid substitutions to the preferred amino acid sequences.

A polynucleotide or polynucleotide region (or a polypeptide or polypeptide region) having a certain percentage (for example, 60%, 65%, 70%, 75%, 80%, 85%, 90%, or 95%) of "sequence identity" to another sequence means that, when aligned, that percentage of bases (or amino acids) are the same in comparing the two sequences. The alignment and the percent homology or sequence identity can be determined using software programs known in the art, for example those described in Current Protocols in Molecular Biology (Ausubel et al., eds. 1987) Supplement 30, section 7.7.18, Table 7.7.1. Preferably, default parameters are used for alignment. A preferred alignment program is BLAST, using default parameters. In particular, preferred programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the following Internet address: ncbi.nlm.nih.gov/cgi-bin/BLAST.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present invention.

"Homology" or "identity" or "similarity" can also refer to two nucleic acid molecules that hybridize under stringent conditions.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Examples of stringent hybridization conditions include: incubation temperatures of about 25° C. to about 37° C.; hybridization buffer concentrations of about 6×SSC to about 10.times.SSC; formamide concentrations of about 0% to about 25%; and wash solutions from about 4×SSC to about 8×SSC. Examples of moderate hybridization conditions include: incubation temperatures of about 40° C. to about 50° C.; buffer concentrations of about 9×SSC to about 2×SSC; formamide concentrations of about 30% to about 50%; and wash solutions of about 5×SSC to about 2×SSC. Examples of high stringency conditions include: incubation temperatures of about 55° C. to about 68° C.; buffer concentrations of about 1×SSC to about 0.1×SSC; formamide concentrations of about 55% to about 75%; and wash solutions of about 1×SSC, 0.1×SSC, or deionized water. In general, hybridization incubation times are from 5 minutes to 24 hours, with 1, 2, or more washing steps, and wash incubation times are about 1, 2, or 15 minutes. SSC is 0.15 M NaCl and 15 mM citrate buffer. It is understood that equivalents of SSC using other buffer systems can be employed.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in an eukaryotic cell.

The term "encode" as it is applied to polynucleotides refers to a polynucleotide which is said to "encode" a polypeptide if, in its native state or when manipulated by methods well known to those skilled in the art, it can be transcribed and/or translated to produce the mRNA for the polypeptide and/or a fragment thereof. The antisense strand is the complement of such a nucleic acid, and the encoding sequence can be deduced therefrom.

As used herein, the terms "treating," "treatment" and the like are used herein to mean obtaining a desired pharmacologic and/or physiologic effect. The effect may be prophylactic in terms of completely or partially preventing a disorder or sign or symptom thereof, and/or may be therapeutic in terms of a partial or complete cure for a disorder and/or adverse effect attributable to the disorder.

To prevent intends to prevent a disorder or effect *in vitro* or *in vivo* in a system or subject that is predisposed to the disorder or effect. An example of such is preventing the formation of a biofilm in a system that is infected with a microorganism known to produce one.

A "composition" is intended to mean a combination of active agent and another compound or composition, inert (for example, a detectable agent or label) or active, such as an adjuvant.

A "pharmaceutical composition" is intended to include the combination of an active agent with a carrier, inert or active, making the composition suitable for diagnostic or therapeutic use *in vitro*, *in vivo* or *ex vivo.*

"Pharmaceutically acceptable carriers" refers to any diluents, excipients, polymers, micelles, lipsomes, vectors, plasmids, or carriers that may be used in the compositions of the invention. Pharmaceutically acceptable carriers include ion exchangers, alumina, aluminum stearate, lecithin, serum proteins, such as human serum albumin, buffer substances, such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Suitable pharmaceutical carriers are described in Remington's Pharmaceutical Sciences, Mack Publishing Company, a standard reference text in this field. They are preferably selected with respect to the intended form of administration, that is, oral tablets, capsules, elixirs, syrups and the like, and consistent with conventional pharmaceutical practices.

Pharmaceutical compositions suitable for injectable use can include sterile aqueous solutions (where the components are water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.), or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

Oral compositions generally include an inert diluent or an edible carrier. For the purpose of oral therapeutic administration, the active compound can be incorporated with excipients and used in the form of tablets, troches, or capsules, *e*.*g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser which contains a suitable propellant, *e.g*., a gas such as carbon dioxide, or a nebulizer.

A "biologically active agent" or an active agent of this invention intends one or more of an isolated or recombinant polypeptide, an isolated or recombinant polynucleotide, a vector, an isolated host cell, or an antibody, as well as compositions comprising one or more of same.

"Administration" can be effected in one dose, continuously or intermittently throughout the course of treatment. Methods of determining the most effective means and dosage of administration are known to those of skill in the art and will vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents are known in the art. Route of administration can also be determined and method of determining the most effective route of administration are known to those of skill in the art and will vary with the composition used for treatment, the purpose of the treatment, the health condition or disease stage of the subject being treated, and target cell or tissue. Non-limiting examples of route of administration include oral administration, nasal administration, injection, and topical application.

An agent of the present invention can be administered for therapy by any suitable route of administration. It will also be appreciated that the preferred route will vary with the condition and age of the recipient, and the disease being treated.

The term "effective amount" refers to a quantity sufficient to achieve a desired effect. In the context of therapeutic or prophylactic applications, the effective amount will depend on the type and severity of the condition at issue and the characteristics of the individual subject, such as general health, age, sex, body weight, and tolerance to pharmaceutical compositions. In the context of an immunogenic composition, in some embodiments the effective amount is the amount sufficient to result in a protective response against a pathogen. In other embodiments, the effective amount of an immunogenic composition is the amount sufficient to result in antibody generation against the antigen. In some embodiments, the effective amount is the amount required to confer passive immunity on a subject in need thereof. With respect to immunogenic compositions, in some embodiments the effective amount will depend on the intended use, the degree of immunogenicity of a particular antigenic compound, and the health/responsiveness of the subject's immune system, in addition to the factors described above. The skilled artisan will be able to determine appropriate amounts depending on these and other factors.

In the case of an *in vitro* application, in some embodiments the effective amount will depend on the size and nature of the application in question. It will also depend on the nature and sensitivity of the *in vitro* target and the methods in use. The skilled artisan will be able to determine the effective amount based on these and other considerations. The effective amount may comprise one or more administrations of a composition depending on the embodiment.

The term "conjugated moiety" refers to a moiety that can be added to an isolated chimeric polypeptide by forming a covalent bond with a residue of chimeric polypeptide. The moiety may bond directly to a residue of the chimeric polypeptide or may form a covalent bond with a linker which in turn forms a covalent bond with a residue of the chimeric polypeptide.

A "peptide conjugate" refers to the association by covalent or non-covalent bonding of one or more polypeptides and another chemical or biological compound. In a non-limiting example, the "conjugation" of a polypeptide with a chemical compound results in improved stability or efficacy of the polypeptide for its intended purpose. In one embodiment, a peptide is conjugated to a carrier, wherein the carrier is a liposome, a micelle, or a pharmaceutically acceptable polymer.

"Liposomes" are microscopic vesicles consisting of concentric lipid bilayers. Structurally, liposomes range in size and shape from long tubes to spheres, with dimensions from a few hundred Angstroms to fractions of a millimeter. Vesicle-forming lipids are selected to achieve a specified degree of fluidity or rigidity of the final complex providing the lipid composition of the outer layer. These are neutral (cholesterol) or bipolar and include phospholipids, such as phosphatidylcholine (PC), phosphatidylethanolamine (PE), phosphatidylinositol (PI), and sphingomyelin (SM) and other types of bipolar lipids including but not limited to dioleoylphosphatidylethanolamine (DOPE), with a hydrocarbon chain length in the range of 14-22, and saturated or with one or more double C=C bonds. Examples of lipids capable of producing a stable liposome, alone, or in combination with other lipid components are phospholipids, such as hydrogenated soy phosphatidylcholine (HSPC), lecithin, phosphatidylethanolamine, lysolecithin, lysophosphatidylethanol- amine, phosphatidylserine, phosphatidylinositol, sphingomyelin, cephalin, cardiolipin, phosphatidic acid, cerebrosides, distearoylphosphatidylethan- olamine (DSPE), dioleoylphosphatidylcholine (DOPC), dipalmitoylphosphatidylcholine (DPPC), palmitoyloleoylphosphatidylcholine (POPC), palmitoyloleoylphosphatidylethanolamine (POPE) and dioleoylphosphatidylethanolamine 4-(N-maleimido-methyl)cyclohexane-1-carboxylate (DOPE-mal). Additional non-phosphorous containing lipids that can become incorporated into liposomes include stearylamine, dodecylamine, hexadecylamine, isopropyl myristate, triethanolamine-lauryl sulfate, alkyl-aryl sulfate, acetyl palmitate, glycerol ricinoleate, hexadecyl stereate, amphoteric acrylic polymers, polyethyloxylated fatty acid amides, and the cationic lipids mentioned above (DDAB, DODAC, DMRIE, DMTAP, DOGS, DOTAP (DOTMA), DOSPA, DPTAP, DSTAP, DC-Chol). Negatively charged lipids include phosphatidic acid (PA), dipalmitoylphosphatidylglycerol (DPPG), dioleoylphosphatidylglycerol and (DOPG), dicetylphosphate that are able to form vesicles. Typically, liposomes can be divided into three categories based on their overall size and the nature of the lamellar structure. The three classifications, as developed by the New York Academy Sciences Meeting, "Liposomes and Their Use in Biology and Medicine," December 1977, are multi-lamellar vesicles (MLVs), small uni-lamellar vesicles (SUVs) and large uni-lamellar vesicles (LUVs). The biological active agents can be encapsulated in such for administration in accordance with the methods described herein.

A "micelle" is an aggregate of surfactant molecules dispersed in a liquid colloid. A typical micelle in aqueous solution forms an aggregate with the hydrophilic "head" regions in contact with surrounding solvent, sequestering the hydrophobic tail regions in the micelle center. This type of micelle is known as a normal phase micelle (oil-in-water micelle). Inverse micelles have the head groups at the center with the tails extending out (water-in-oil micelle). Micelles can be used to attach a polynucleotide, polypeptide, antibody or composition described herein to facilitate efficient delivery to the target cell or tissue.

The phrase "pharmaceutically acceptable polymer" refers to the group of compounds which can be conjugated to one or more polypeptides described here. It is contemplated that the conjugation of a polymer to the polypeptide is capable of extending the half-life of the polypeptide *in vivo* and *in vitro.* Non-limiting examples include polyethylene glycols, polyvinylpyrrolidones, polyvinylalcohols, cellulose derivatives, polyacrylates, polymethacrylates, sugars, polyols and mixtures thereof. The biological active agents can be conjugated to a pharmaceutically acceptable polymer for administration in accordance with the methods described herein.

A "gene delivery vehicle" is defined as any molecule that can carry inserted polynucleotides into a host cell. Examples of gene delivery vehicles are liposomes, micelles biocompatible polymers, including natural polymers and synthetic polymers; lipoproteins; polypeptides; polysaccharides; lipopolysaccharides; artificial viral envelopes; metal particles; and bacteria, or viruses, such as baculovirus, adenovirus and retrovirus, bacteriophage, cosmid, plasmid, fungal vectors and other recombination vehicles typically used in the art which have been described for expression in a variety of eukaryotic and prokaryotic hosts, and may be used for gene therapy as well as for simple protein expression.

A polynucleotide of this invention can be delivered to a cell or tissue using a gene delivery vehicle. "Gene delivery," "gene transfer," "transducing," and the like as used herein, are terms referring to the introduction of an exogenous polynucleotide (sometimes referred to as a "transgene") into a host cell, irrespective of the method used for the introduction. Such methods include a variety of well-known techniques such as vector-mediated gene transfer (by, e.g., viral infection/transfection, or various other protein-based or lipid-based gene delivery complexes) as well as techniques facilitating the delivery of "naked" polynucleotides (such as electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome. A number of vectors are known to be capable of mediating transfer of genes to mammalian cells, as is known in the art and described herein.

A "plasmid" is an extra-chromosomal DNA molecule separate from the chromosomal DNA which is capable of replicating independently of the chromosomal DNA. In many cases, it is circular and double-stranded. Plasmids provide a mechanism for horizontal gene transfer within a population of microbes and typically provide a selective advantage under a given environmental state. Plasmids may carry genes that provide resistance to naturally occurring antibiotics in a competitive environmental niche, or alternatively the proteins produced may act as toxins under similar circumstances.

"Plasmids" used in genetic engineering are called "plasmid vectors". Many plasmids are commercially available for such uses. The gene to be replicated is inserted into copies of a plasmid containing genes that make cells resistant to particular antibiotics and a multiple cloning site (MCS, or polylinker), which is a short region containing several commonly used restriction sites allowing the easy insertion of DNA fragments at this location. Another major use of plasmids is to make large amounts of proteins. In this case, researchers grow bacteria containing a plasmid harboring the gene of interest. Just as the bacteria produces proteins to confer its antibiotic resistance, it can also be induced to produce large amounts of proteins from the inserted gene. This is a cheap and easy way of mass-producing a gene or the protein it then codes for.

A "yeast artificial chromosome" or "YAC" refers to a vector used to clone large DNA fragments (larger than 100 kb and up to 3000 kb). It is an artificially constructed chromosome and contains the telomeric, centromeric, and replication origin sequences needed for replication and preservation in yeast cells. Built using an initial circular plasmid, they are linearized by using restriction enzymes, and then DNA ligase can add a sequence or gene of interest within the linear molecule by the use of cohesive ends. Yeast expression vectors, such as YACs, YIps (yeast integrating plasmid), and YEps (yeast episomal plasmid), are extremely useful as one can get eukaryotic protein products with posttranslational modifications as yeasts are themselves eukaryotic cells, however YACs have been found to be more unstable than BACs, producing chimeric effects.

A "viral vector" is defined as a recombinantly produced virus or viral particle that comprises a polynucleotide to be delivered into a host cell, either *in vivo*, *ex vivo* or *in vitro.* Examples of viral vectors include retroviral vectors, adenovirus vectors, adeno-associated virus vectors, alphavirus vectors and the like. Infectious tobacco mosaic virus (TMV)-based vectors can be used to manufacturer proteins and have been reported to express Griffithsin in tobacco leaves (O'Keefe et al. (2009) Proc. Nat. Acad. Sci. USA 106(15):6099-6104). Alphavirus vectors, such as Semliki Forest virus-based vectors and Sindbis virus-based vectors, have also been developed for use in gene therapy and immunotherapy. See, Schlesinger & Dubensky (1999) Curr. Opin. Biotechnol. 5:434-439 and Ying et al. (1999) Nat. Med. 5(7):823-827. In aspects where gene transfer is mediated by a retroviral vector, a vector construct refers to the polynucleotide comprising the retroviral genome or part thereof, and a therapeutic gene.

As used herein, "retroviral mediated gene transfer" or "retroviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, retroviral vector refers to a viral particle capable of introducing exogenous nucleic acid into a cell through a viral or viral-like entry mechanism.

Retroviruses carry their genetic information in the form of RNA; however, once the virus infects a cell, the RNA is reverse-transcribed into the DNA form which integrates into the genomic DNA of the infected cell. The integrated DNA form is called a provirus.

In aspects where gene transfer is mediated by a DNA viral vector, such as an adenovirus (Ad) or adeno-associated virus (AAV), a vector construct refers to the polynucleotide comprising the viral genome or part thereof, and a transgene. Adenoviruses (Ads) are a relatively well characterized, homogenous group of viruses, including over 50 serotypes. See, e.g., International PCT Publication No. WO 95/27071. Ads do not require integration into the host cell genome. Recombinant Ad derived vectors, particularly those that reduce the potential for recombination and generation of wild-type virus, have also been constructed. See, International PCT Publication Nos. WO 95/00655 and WO 95/11984. Wild-type AAV has high infectivity and specificity integrating into the host cell's genome. See, Hermonat & Muzyczka (1984) Proc. Natl. Acad. Sci. USA 81:6466-6470 and Lebkowski et al. (1988) Mol. Cell. Biol. 8:3988-3996.

Vectors that contain both a promoter and a cloning site into which a polynucleotide can be operatively linked are well known in the art. Such vectors are capable of transcribing RNA *in vitro* or *in vivo*, and are commercially available from sources such as Stratagene (La Jolla, CA) and Promega Biotech (Madison, WI). In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

Gene delivery vehicles also include DNA/liposome complexes, micelles and targeted viral protein-DNA complexes. Liposomes that also comprise a targeting antibody or fragment thereof can be used in the methods of this invention. In addition to the delivery of polynucleotides to a cell or cell population, direct introduction of the proteins described herein to the cell or cell population can be done by the non-limiting technique of protein transfection, alternatively culturing conditions that can enhance the expression and/or promote the activity of the proteins of this invention are other non-limiting techniques.

As used herein the term "eDNA" refers to extracellular DNA found as a component to pathogenic, e.g., bacterial, biofilms.

As used herein the terms "antibodies" and "immunoglobulin" include antibodies or immunoglobulins of any isotype, fragments of antibodies which retain specific binding to antigen, including, but not limited to, Fab, Fab', F(ab)₂, Fv, scFv, dsFv, Fd fragments, dAb, VH, VL, VhH, and V-NAR domains; minibodies, diabodies, triabodies, tetrabodies and kappa bodies; multispecific antibody fragments formed from antibody fragments and one or more isolated CDRs or a functional paratope; chimeric antibodies, humanized antibodies, single-chain antibodies, and fusion proteins comprising an antigen-binding portion of an antibody and a non-antibody protein. The variable regions of the heavy and light chains of the immunoglobulin molecule contain a binding domain that interacts with an antigen. The constant regions of the antibodies (Abs) may mediate the binding of the immunoglobulin to host tissues.

The term "antibody" herein is used in the broadest sense and specifically includes full-length monoclonal antibodies, polyclonal antibodies, human antibodies, humanized antibodies, human monoclonal antibody, recombinant human antibody, chimeric antibodies, antibody derivative, multispecific antibodies (e.g., bispecific antibodies), and antibody fragments, antigen binding fragment, so long as they exhibit the desired biological activity.

As used herein, "monoclonal antibody" refers to an antibody obtained from a substantially homogeneous antibody population. Monoclonal antibodies are highly specific, as each monoclonal antibody is directed against a single determinant on the antigen. The antibodies may be detectably labeled, e.g., with a radioisotope, an enzyme which generates a detectable product, a fluorescent protein, and the like. The antibodies may be further conjugated to other moieties, such as members of specific binding pairs, e.g., biotin (member of biotin-avidin specific binding pair), and the like. The antibodies may also be bound to a solid support, including, but not limited to, polystyrene plates or beads, and the like.

Monoclonal antibodies may be generated using hybridoma techniques or recombinant DNA methods known in the art. Alternative techniques for generating or selecting antibodies include *in vitro* exposure of lymphocytes to antigens of interest, and screening of antibody display libraries in cells, phage, or similar systems.

The term "human antibody" as used herein, is intended to include antibodies having variable and constant regions derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo*). However, the term "human antibody" as used herein, is not intended to include antibodies in which CDR sequences derived from the germline of another mammalian species, such as a mouse, have been grafted onto human framework sequences. Thus, as used herein, the term "human antibody" refers to an antibody in which substantially every part of the protein (e.g., CDR, framework, C_{L}, C_{H} domains (e.g., C_{H1}, C_{H2}, C_{H3}), hinge, (VL, VH)) is substantially non-immunogenic in humans, with only minor sequence changes or variations. Similarly, antibodies designated primate (monkey, baboon, chimpanzee, etc.), rodent (mouse, rat, rabbit, guinea pig, hamster, and the like) and other mammals designate such species, sub-genus, genus, sub-family, family specific antibodies. Further, chimeric antibodies include any combination of the above. Such changes or variations optionally and preferably retain or reduce the immunogenicity in humans or other species relative to non-modified antibodies. Thus, a human antibody is distinct from a chimeric or humanized antibody. It is pointed out that a human antibody can be produced by a non-human animal or prokaryotic or eukaryotic cell that is capable of expressing functionally rearranged human immunoglobulin (e.g., heavy chain and/or light chain) genes. Further, when a human antibody is a single chain antibody, it can comprise a linker peptide that is not found in native human antibodies. For example, an Fv can comprise a linker peptide, such as two to about eight glycine or other amino acid residues, which connects the variable region of the heavy chain and the variable region of the light chain. Such linker peptides are considered to be of human origin.

As used herein, a human antibody is "derived from" a particular germline sequence if the antibody is obtained from a system using human immunoglobulin sequences, e.g., by immunizing a transgenic mouse carrying human immunoglobulin genes or by screening a human immunoglobulin gene library. A human antibody that is "derived from" a human germline immunoglobulin sequence can be identified as such by comparing the amino acid sequence of the human antibody to the amino acid sequence of human germline immunoglobulins. A selected human antibody typically is at least 90% identical in amino acids sequence to an amino acid sequence encoded by a human germline immunoglobulin gene and contains amino acid residues that identify the human antibody as being human when compared to the germline immunoglobulin amino acid sequences of other species (e.g., murine germline sequences). In certain cases, a human antibody may be at least 95%, or even at least 96%, 97%, 98%, or 99% identical in amino acid sequence to the amino acid sequence encoded by the germline immunoglobulin gene. Typically, a human antibody derived from a particular human germline sequence will display no more than 10 amino acid differences from the amino acid sequence encoded by the human germline immunoglobulin gene. In certain cases, the human antibody may display no more than 5, or even no more than 4, 3, 2, or 1 amino acid difference from the amino acid sequence encoded by the germline immunoglobulin gene.

A "human monoclonal antibody" refers to antibodies displaying a single binding specificity which have variable and constant regions derived from human germline immunoglobulin sequences. The term also intends recombinant human antibodies. Methods to making these antibodies are described herein.

The term "recombinant human antibody", as used herein, includes all human antibodies that are prepared, expressed, created or isolated by recombinant means, such as antibodies isolated from an animal (e.g., a mouse) that is transgenic or transchromosomal for human immunoglobulin genes or a hybridoma prepared therefrom, antibodies isolated from a host cell transformed to express the antibody, e.g., from a transfectoma, antibodies isolated from a recombinant, combinatorial human antibody library, and antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.* Methods to making these antibodies are described herein.

As used herein, chimeric antibodies are antibodies whose light and heavy chain genes have been constructed, typically by genetic engineering, from antibody variable and constant region genes belonging to different species.

As used herein, the term "humanized antibody" or "humanized immunoglobulin" refers to a human/non-human chimeric antibody that contains a minimal sequence derived from non-human immunoglobulin. For the most part, humanized antibodies are human immunoglobulins (recipient antibody) in which residues from a variable region of the recipient are replaced by residues from a variable region of a non-human species (donor antibody) such as mouse, rat, rabbit, or non-human primate having the desired specificity, affinity and capacity. Humanized antibodies may comprise residues that are not found in the recipient antibody or in the donor antibody. The humanized antibody can optionally also comprise at least a portion of an immunoglobulin constant region (Fc), typically that of a human immunoglobulin. a non-human antibody containing one or more amino acids in a framework region, a constant region or a CDR, that have been substituted with a correspondingly positioned amino acid from a human antibody. In general, humanized antibodies are expected to produce a reduced immune response in a human host, as compared to a non-humanized version of the same antibody. The humanized antibodies may have conservative amino acid substitutions which have substantially no effect on antigen binding or other antibody functions. Conservative substitutions groupings include:glycine-alanine, valine-leucine-isoleucine, phenylalanine-tyrosine, lysine-arginine, alanine-valine, serine-threonine and asparagine-glutamine.

As used herein, the term "antibody derivative", comprises a full-length antibody or a fragment of an antibody, wherein one or more of the amino acids are chemically modified by alkylation, pegylation, acylation, ester formation or amide formation or the like, e.g., for linking the antibody to a second molecule. This includes, but is not limited to, pegylated antibodies, cysteine-pegylated antibodies, and variants thereof.

As used herein, the term "immunoconjugate" comprises an antibody or an antibody derivative associated with or linked to a second agent, such as a cytotoxic agent, a detectable agent, a fluorescent label, a radioactive agent, a targeting agent, a human antibody, a humanized antibody, a chimeric antibody, a synthetic antibody, a semisynthetic antibody, or a multispecific antibody.

As used herein, the term "detectable label" intends a directly or indirectly detectable compound or composition that is conjugated directly or indirectly to the composition to be detected, e.g., N-terminal histadine tags (N-His), magnetically active isotopes, e.g., ¹¹⁵Sn, ¹¹⁷Sn and ¹¹⁹Sn, a non-radioactive isotopes such as ¹³C and ¹⁵N, polynucleotide or protein such as an antibody so as to generate a "labeled" composition. The term also includes sequences conjugated to the polynucleotide that will provide a signal upon expression of the inserted sequences, such as green fluorescent protein (GFP) and the like. The label may be detectable by itself (e.g. radioisotope labels or fluorescent labels) or, in the case of an enzymatic label, may catalyze chemical alteration of a substrate compound or composition which is detectable. The labels can be suitable for small scale detection or more suitable for high-throughput screening. As such, suitable labels include, but are not limited to magnetically active isotopes, non-radioactive isotopes, radioisotopes, fluorochromes, luminescent compounds, dyes, and proteins, including enzymes. The label may be simply detected or it may be quantified. A response that is simply detected generally comprises a response whose existence merely is confirmed, whereas a response that is quantified generally comprises a response having a quantifiable (e.g., numerically reportable) value such as an intensity, polarization, and/or other property. In luminescence or fluorescence assays, the detectable response may be generated directly using a luminophore or fluorophore associated with an assay component actually involved in binding, or indirectly using a luminophore or fluorophore associated with another (e.g., reporter or indicator) component.

Examples of luminescent labels that produce signals include, but are not limited to bioluminescence and chemiluminescence. Detectable luminescence response generally comprises a change in, or an occurrence of, a luminescence signal. Suitable methods and luminophores for luminescently labeling assay components are known in the art and described for example in Haugland, Richard P. (1996) Handbook of Fluorescent Probes and Research Chemicals (6th ed.). Examples of luminescent probes include, but are not limited to, aequorin and luciferases.

Examples of suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade Blue™, and Texas Red. Other suitable optical dyes are described in the Haugland, Richard P. (1996) Handbook of Fluorescent Probes and Research Chemicals (6th ed.).

In another aspect, the fluorescent label is functionalized to facilitate covalent attachment to a cellular component present in or on the surface of the cell or tissue such as a cell surface marker. Suitable functional groups, including, but not are limited to, isothiocyanate groups, amino groups, haloacetyl groups, maleimides, succinimidyl esters, and sulfonyl halides, all of which may be used to attach the fluorescent label to a second molecule. The choice of the functional group of the fluorescent label will depend on the site of attachment to either a linker, the agent, the marker, or the second labeling agent.

Examples of suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade Blue®, and Texas Red®. Other suitable optical dyes are described in the Haugland, Richard P. (1996) Handbook of Fluorescent Probes and Research Chemicals (6th ed.).

In another aspect, the fluorescent label is functionalized to facilitate covalent attachment to a cellular component present in or on the surface of the cell or tissue such as a cell surface marker. Suitable functional groups, including, but not are limited to, isothiocyanate groups, amino groups, haloacetyl groups, maleimides, succinimidyl esters, and sulfonyl halides, all of which may be used to attach the fluorescent label to a second molecule. The choice of the functional group of the fluorescent label will depend on the site of attachment to either a linker, the agent, the marker, or the second labeling agent.

"Eukaryotic cells" comprise all of the life kingdoms except monera. They can be easily distinguished through a membrane-bound nucleus. Animals, plants, fungi, and protists are eukaryotes or organisms whose cells are organized into complex structures by internal membranes and a cytoskeleton. The most characteristic membrane-bound structure is the nucleus. Unless specifically recited, the term "host" includes a eukaryotic host, including, for example, yeast, higher plant, insect and mammalian cells. Non-limiting examples of eukaryotic cells or hosts include simian, bovine, porcine, murine, rat, avian, reptilian and human.

"Prokaryotic cells" that usually lack a nucleus or any other membrane-bound organelles and are divided into two domains, bacteria and archaea. Additionally, instead of having chromosomal DNA, these cells' genetic information is in a circular loop called a plasmid. Bacterial cells are very small, roughly the size of an animal mitochondrion (about 1-2µm in diameter and 10 µm long). Prokaryotic cells feature three major shapes: rod shaped, spherical, and spiral. Instead of going through elaborate replication processes like eukaryotes, bacterial cells divide by binary fission. Examples include but are not limited to *bacillus* bacteria, *E. coli* bacterium, and *Salmonella* bacterium.

A "native" or "natural" antigen is a polypeptide, protein or a fragment which contains an epitope, which has been isolated from a natural biological source, and which can specifically bind to an antigen receptor, in particular a T cell antigen receptor (TCR), in a subject.

The terms "antigen" and "antigenic" refer to molecules with the capacity to be recognized by an antibody or otherwise act as a member of an antibody-ligand pair. "Specific binding" refers to the interaction of an antigen with the variable regions of immunoglobulin heavy and light chains. Antibody-antigen binding may occur *in vivo* or *in vitro.* The skilled artisan will understand that macromolecules, including proteins, nucleic acids, fatty acids, lipids, lipopolysaccharides and polysaccharides have the potential to act as an antigen. The skilled artisan will further understand that nucleic acids encoding a protein with the potential to act as an antibody ligand necessarily encode an antigen. The artisan will further understand that antigens are not limited to full-length molecules, but can also include partial molecules. The term "antigenic" is an adjectival reference to molecules having the properties of an antigen. The term encompasses substances which are immunogenic, i.e., immunogens, as well as substances which induce immunological unresponsiveness, or anergy, i.e., anergens.

An "altered antigen" is one having a primary sequence that is different from that of the corresponding wild-type antigen. Altered antigens can be made by synthetic or recombinant methods and include, but are not limited to, antigenic peptides that are differentially modified during or after translation, e.g., by phosphorylation, glycosylation, cross-linking, acylation, proteolytic cleavage, linkage to an antibody molecule, membrane molecule or other ligand. (Ferguson et al. (1988) Ann. Rev. Biochem. 57:285-320). A synthetic or altered antigen of the invention is intended to bind to the same TCR as the natural epitope.

A "self-antigen" also referred to herein as a native or wild-type antigen is an antigenic peptide that induces little or no immune response in the subject due to self-tolerance to the antigen. An example of a self-antigen is the melanoma specific antigen gp100.

The terms "major histocompatibility complex" or "MHC" refers to a complex of genes encoding cell-surface molecules that are required for antigen presentation to T cells and for rapid graft rejection. In humans, the MHC is also known as the "human leukocyte antigen" or "HLA" complex. The proteins encoded by the MHC are known as "MHC molecules" and are classified into class I and class II MHC molecules. Class I MHC includes membrane heterodimeric proteins made up of an α chain encoded in the MHC noncovalently linked with the β2-microglobulin. Class I MHC molecules are expressed by nearly all nucleated cells and have been shown to function in antigen presentation to CD8⁺ T cells. Class I molecules include HLA-A, B, and C in humans. Class II MHC molecules also include membrane heterodimeric proteins consisting of noncovalently associated α and β chains. Class II MHC molecules are known to function in CD4⁺ T cells and, in humans, include HLA-DP, -DQ, and DR. In a preferred embodiment, invention compositions and ligands can complex with MHC molecules of any HLA type. Those of skill in the art are familiar with the serotypes and genotypes of the HLA. See: bimas.dcrt.nih.gov/cgi-bin/molbio/hla coefficient viewing page. Rammensee H. G., Bachmann J., and Stevanovic S. MHC Ligands and Peptide Motifs (1997) Chapman & Hall Publishers; Schreuder G. M. Th. et al. The HLA dictionary (1999) Tissue Antigens 54:409-437.

"Immune response" broadly refers to the antigen-specific responses of lymphocytes to foreign substances. The terms "immunogen" and "immunogenic" refer to molecules with the capacity to elicit an immune response. The response may involve antibody production or the activation of immune cells. The response may occur *in vivo* or *in vitro.* The skilled artisan will understand that a variety of macromolecules, including proteins, nucleic acids, fatty acids, lipids, lipopolysaccharides and polysaccharides have the potential to be immunogenic. The skilled artisan will further understand that nucleic acids encoding a molecule capable of eliciting an immune response necessarily encode an immunogen. The artisan will further understand that immunogens are not limited to full-length molecules, but may include partial molecules.

The term "passive immunity" refers to the transfer of immunity from one subject to another through the transfer of antibodies. Passive immunity may occur naturally, as when maternal antibodies are transferred to a fetus. Passive immunity may also occur artificially as when antibody compositions are administered to non-immune subjects. Antibody donors and recipients may be human or non-human subjects. Antibodies may be polyclonal or monoclonal, may be generated *in vitro* or *in vivo*, and may be purified, partially purified, or unpurified depending on the embodiment. In some embodiments described herein, passive immunity is conferred on an a subject in need thereof through the administration of antibodies or antigen binding fragments that specifically recognize or bind to a particular antigen. In some embodiments, passive immunity is conferred through the administration of an isolated or recombinant polynucleotide encoding an antibody or antigen binding fragment that specifically recognizes or binds to a particular antigen.

In the context of this invention, a "ligand" is a polypeptide. In one aspect, the term "ligand" as used herein refers to any molecule that binds to a specific site on another molecule. In other words, the ligand confers the specificity of the protein in a reaction with an immune effector cell or an antibody to a protein or DNA to a protein. In one aspect it is the ligand site within the protein that combines directly with the complementary binding site on the immune effector cell.

As used herein, "solid phase support" or "solid support", used interchangeably, is not limited to a specific type of support. Rather a large number of supports are available and are known to one of ordinary skill in the art. Solid phase supports include silica gels, resins, derivatized plastic films, glass beads, cotton, plastic beads, alumina gels. As used herein, "solid support" also includes synthetic antigen-presenting matrices, cells, and liposomes. A suitable solid phase support may be selected on the basis of desired end use and suitability for various protocols. For example, for peptide synthesis, solid phase support may refer to resins such as polystyrene (e.g., PAM-resin obtained from Bachem Inc., Peninsula Laboratories, etc.), POLYHIPE.RTM. resin (obtained from Aminotech, Canada), polyamide resin (obtained from Peninsula Laboratories), polystyrene resin grafted with polyethylene glycol (TentaGel.RTM., Rapp Polymere, Tubingen, Germany) or polydimethylacrylamide resin (obtained from Milligen/Biosearch, Calif.).

An example of a solid phase support include glass, polystyrene, polypropylene, polyethylene, dextran, nylon, amylases, natural and modified celluloses, polyacrylamides, gabbros, and magnetite. The nature of the carrier can be either soluble to some extent or insoluble. The support material may have virtually any possible structural configuration so long as the coupled molecule is capable of binding to a polynucleotide, polypeptide or antibody. Thus, the support configuration may be spherical, as in a bead, or cylindrical, as in the inside surface of a test tube, or the external surface of a rod. Alternatively, the surface may be flat such as a sheet, test strip, etc. or alternatively polystyrene beads. Those skilled in the art will know many other suitable carriers for binding antibody or antigen, or will be able to ascertain the same by use of routine experimentation.

### Modes For Carrying Out The Disclosure

Cystic fibrosis (CF) is the most common lethal inherited genetic disorder affection Caucasians. Even with medical advances, CF is life-shortening with patients typically surviving only to age 38. Infection of the CF lung by *Burkholderia cenocepacia (B. cenocepacia)* presents exceptional challenges to medical management of these patients as clinically this microbe is resistant to virtually all antibiotics, is highly transmissible and infection of CF patients with this microbe renders them ineligible for lung transplant, often the last lifesaving option. The inventors have targeted two abundant components of the *B. cenocepacia* biofilm for immune intervention: extracellular DNA and DNABII proteins, the latter of which are bacterial nucleic acid binding proteins. Treatment of *B. cenocepacia* biofilms with antiserum directed at one of these DNABII proteins (integration host factor or IHF) resulted in significant disruption of the biofilm. Moreover, when anti-IHF mediated destabilization of a *B. cenocepacia* biofilm was combined with exposure to traditional antibiotics, *B. cenocepacia* resident within the biofilm and thereby typically highly resistant to the action of antibiotics, were now rendered susceptible to killing. Pre-incubation of *B. cenocepacia* with anti-IHF serum prior to exposure to murine CF macrophages, which are normally unable to effectively degrade ingested *B. cenocepacia*, resulted in a statistically significant increase in killing of phagocytized *B. cenocepacia.* Collectively, these findings show that targeting DNABII proteins is a novel approach for treatment of CF patients, particularly those whose lungs are infected with *B. cenocepacia.*

### Diagnostic and Therapeutic Methods

This disclosure provides a method for inhibiting, competing or titrating a biofilm produced by *Burkholderia*, the method comprising, or alternatively consisting essentially of, or yet consisting of, contacting the biofilm with an effective amount of an anti- integration host factor (anti-IHF) antibody, thereby inhibiting, competing or titrating the biofilm. The method can be performed *in vitro* or *in vivo.* In one aspect, the contacting of the anti-IHF antibody is performed in the absence of a DNase treatment. In one aspect, the DNAse treatment that is excluded from therapy, comprises an enzyme that catalyzes the cleavage of phosphodiester linkages in the DNA backbone. Three non-limiting examples of DNase enzymes that are excluded from therapy are known to target not only cruciform structures, but also a variety of secondary structure of DNA include DNAse I, T4 EndoVII and T7 Endo I. In one aspect, the DNase treatment that is excluded from therapy comprises, or consists essentially of, or yet further consists of, Pulmozyme® (dornase alpha; Genentech, Inc.).

In another aspect, the method further comprises, or alternatively consisting essentially of, or yet further consists of, contacting the biofilm or *Burkkholderia* with an effective amount of an antimicrobial that inhibits the growth of the *Burkkholderia* causing the biofilm. Non-limiting examples of such include ampicillin, amoxicillin-clavulanate, ceftazidine, ciprofloxacin, imipenem, minocycline, and cefdinir. The contacting can be conducted *in vitro* in or *in vivo.*

Also provided herein is a method of treating an infection or disease caused by a *Burkholderia* infection, in a subject, comprising, or alternatively consisting essentially of, or yet consisting of, administering to the subject an effective amount of an anti-IHF antibody, thereby treating the infection or disease caused by the *Burkholderia* infection.

This disclosure also provides methods for treating or preventing the recurrence of an infection in a CF patient in need thereof, comprising, or alternatively consistin essentially of, or yet further consisting of, administering to the patient an effective amount of an anti-IHF antibody, thereby treating or preventing the recurrence of the infection in the CF patient.

In one aspect, the administration of the anti-IHF antibody is in the absence of a DNase treatment. In one aspect, the DNAse treatment that is excluded from therapy comprises an enzyme that catalyzes the cleavage of phosphodiester linkages in the DNA backbone. Three non-limiting examples of DNase enzymes that are excluded from therapy and known to target not only cruciform structures, but also a variety of secondary structure of DNA include DNAse I, T4 EndoVII and T7 Endo I. In one aspect, the DNase treatment that is excluded from therapy comprises, or consists essentially of, or yet further consists of, Pulmozyme® (dornase alpha; Genentech, Inc.). In a yet further aspect, the method further comprises, or yet further consists essentially of, or yet further consists of, administering to the subject an effective amount of an antimicrobial that inhibits the growth of the *Burkholderia* causing the biofilm. Non-limiting examples of such include ampicillin, amoxicillin-clavulanate, ceftazidine, ciprofloxacin, imipenem, minocycline, and cefdinir.

In each of the methods described above, non-limiting examples of the *Burkholderia* is *Burkholderia cenocepacia (B. cenocepacia)*, *B. multivorans*, *B. mallei, B. cepaci*, *or B. pseudomallei.*

Anti -IHF antibody for use in the above methods is one or more of an anti-IHFa or an anti-IHFβ antibody. In a further aspect, the anti-IHF antibody is an IgG antibody. The antibody can be any of the various antibodies described herein, non-limiting examples of such include a full-length monoclonal antibody, a polyclonal antibody, human antibodies, humanized antibodies, human monoclonal antibody, a recombinant human antibody, a chimeric antibody, a veneered antibody, a diabody, a humanized antibody, an antibody derivative, a recombinant humanized antibody, or a fragment or an antigen binding fragment thereof, so long it exhibits the desired biological activity. In one aspect, the fragment comprises, or alternatively consists essentially of, or yet further consists of the CDR of the antibody. In one aspect, the antibody is detectably labeled or further comprises a detectable label conjugated to it.

Non-limiting examples of anti-IHF antibodies are described herein, and in one aspect, is one or more of an antibody that specifically recognizes and binds a polypeptide identified in Table 2, or the Arm fragment identified therein, or an equivalent of such polypeptide or a polynucleotide or polypeptide comprising one or more of the sequences: : TCTCAACGATTTA (SEQ ID NO. 341); WATCAANNNNTTR (where W is A or T, N is any nucleotide and R is a A or G; (SEQ ID NO. 342); MATIT**K**LDIIEYLSDKYHLS (also referred to herein as hIFA1; (SEQ ID NO. 343); KYHLSKQDTKNVVEN**FLEEI** (also referred to herein as hIFA2; (SEQ ID NO. 344); **FLEEI**RLSLESGQDV**KLSGF** (also referred to herein as hIFA3; (SEQ ID NO. 345); **KLSGFGNF**ELRDKSS**RPGRN** (also referred to herein as hIFA4; (SEQ ID NO. 346); **RPGRNPKTGDVV**PVSARRVV (also referred to herein as hIFA5; (SEQ ID NO. 347); ARRVVTFKPGQKLRARVEKTK (also referred to herein as hIFA6; (SEQ ID NO. 348), or an equivalent thereof or a polynucleotide or peptide having at least 60%, or alternatively at least 65%, or alternatively at least 70%, or alternatively at least 75%, or alternatively 80%, or alternatively at least 85%, or alternatively at least 90%, or alternatively at least 95% identity thereto or for polypeptide sequences, which is encoded by a polynucleotide or its complement that hybridizes under conditions of high stringency to a polynucleotide encoding such polypeptide sequences. Conditions of high stringency are described above and incoporrated herein by reference. Applicants have determined that the bolded and underlined amino acids are heavily conserved and therefore in one aspect, are not modified or altered in desiging an equivalent polypeptide. Additional examples of equivalent polypeptides include, for example a polypeptide consisting of or comprising the above noted polypeptides with the addition of up to 25, or alternatively 20, or alternatively 15, or alternatively up to 10, or alternatively up to 5 random amino acids on either the amine or carboxy termini (or on both).

For these methods, the infection is *in vivo* and in a mammalian host, such as a human patient, e.g., is an immature mammalian host or a pediatric patient.

This disclosure also provides a method for inhibiting, competing or titrating a biofilm present or contributing to CF (e.g., a *B. cenocepacia* induced biofilm), the method comprising, or alternatively consisting essentially of, or yet further consisting of, contacting the biofilm with an interfering agent, thereby inhibiting, competing or titrating the biofilm. The contacting can be performed *in vitro* or *in vivo.*

In another aspect, provided is a method for inhibiting, preventing or breaking down a microbial biofilm in a CF patient or a patient harboring an infection contributing to CF, comprising, or alternatively consisting essentially of, or yet further consisting of contacting the biofilm with an interfering agent, thereby inhibiting, preventing or breaking down the microbial biofilm. The patients can be an animal, mammal or a human patient.

When practiced *in vitro,* the methods are useful to screen for or confirm interfering agents having the same, similar or opposite ability as the polypeptides, polynucleotides, antibodies, host cells, small molecules and compositions of this invention. Alternatively, they can be used to identify which interfering agent is best suited to treat a microbial infection. For example, one can screen for new agents or combination therapies by having two samples containing for example, the DNA BII polypeptide and microbial DNA or biofilm and the agent to be tested. The second sample contains the DNA BII polypeptide and microbial DNA or biofilm and an agent known to active, e.g., an anti-IHF antibody or a small molecule to serve as a positive control. In a further aspect, several samples are provided and the interfering agents are added to the system in increasing dilutions to determine the optimal dose that would likely be effective in treating a subject in the clinical setting. As is apparent to those of skill in the art, a negative control containing the DNA BII polypeptide and the microbial DNA or biofilm can be provided. In a further aspect, the DNA BII polypeptide and the microbial DNA or biofilm are detectably labeled, for example with luminescent molecules that will emit a signal when brought into close contact with each other. The samples are contained under similar conditions for an effective amount of time for the agent to inhibit, compete or titrate the interaction between the DNA BII polypeptide and microbial DNA or biofilm and then the sample is assayed for emission of signal from the luminescent molecules. If the sample emits a signal, then the agent is not effective to inhibit binding.

In another aspect, the *in vitro* method is practiced in a miniaturized chamber slide system wherein the microbial (such as a bacterial) isolate causing a CF infection could be isolated from the human/animal then cultured to allow it to grow as a biofilm *in vitro.* The interfering agent (such as anti-IHF antibody) or potential interfering agent biofilm is added alone or in combination with another agent to the culture with or without increasing dilutions of the potential interfering agent or interfering agent such as an anti-IHF (or other antibody, small molecule, agent etc.) to find the optimal dose that would likely be effective at treating that patient when delivered to the subject where the infection existed. As apparent to those of skill in the art, a positive and negative control can be performed simultaneously.

In a further aspect, the method is practiced in a high throughput platform with the interfering agent (such as anti-IHF antibody) and/or potential interfering agent (alone or in combination with another agent) in a flow cell. The interfering agent (such as anti-IHF antibody) or potential interfering agent biofilm is added alone or in combination with another agent to the culture with or without increasing dilutions of the potential interfering agent or interfering agent such as an anti-IHF (or other antibody, small molecule, agent etc.) to find the optimal dose that would likely be effective at treating that patient when delivered to the subject where the infection existed. Biofilm isolates are sonicated to separate biofilm bacteria from DNA BII polyeptide such as IHF bound to microbial DNA. The DNA BII polypeptide - DNA complexes are isolated by virtue of an anti-IHF antibody on the platform. The microbial DNA is then be released with e.g. a salt wash, and used to identify the biofilm bacteria added. The freed DNA is then identified, e.g., by PCR sequenced. If DNA is not freed, then the interfering agent(s) successfully performed or bound the microbial DNA. If DNA is found in the sample, then the agent did not interfere with DNA BII polypeptide-microbial DNA binding. As is apparent to those of skill in the art, a positive and/or negative control can be simultaneously performed.

The above methods also can be used as a diagnostic test since it is possible that a given bacterial species will respond better to reversal of its biofilm by one agent more than another, this rapid high throughput assay system could allow one skilled in the art to assay a panel of possible anti-IHF-like agents to identify the most efficacious of the group.

The advantage of these methods is that most clinical microbiology labs in hospitals are already equipped to perform these sorts of assays (i.e. determination of MIC, MBC values) using bacteria that are growing in liquid culture (or planktonically). As is apparent to those of skill in the art, bacteria generally do not grow planktonically when they are causing diseases. Instead they are growing as a stable biofilm and these biofilms are significantly more resistant to treatment by antibiotics, antibodies or other therapeutics. This resistance is why most MIC/MBC values fail to accurately predict efficacy *in vivo.* Thus, by determining what "dose" of agent could reverse a bacterial biofilm *in vitro* (as described above) Applicants' pre-clinical assay would be a more reliable predictor of clinical efficacy, even as an application of personalized medicine.

In addition to the clinical setting, the methods can be used to identify and/or confirm interfering agents in an industrial setting.

In a further aspect of the above methods, an antibiotic or antimicrobial known to inhibit growth of the underlying infection is added sequentially or concurrently, to determine if the infection can be inhibited. It is also possible to add the interfering agent to the microbial DNA or DNA BII polypeptide before adding the missing complex to assay for biofilm inhibition.

When practiced *in vivo* in non-human animal, the method provides a pre-clinical screen to identify interfering agents that can be used alone or in combination with other agents to break down biofilms.

In another aspect, provided is a method for inhibiting, preventing or breaking down a microbial biofilm and/or clearing a microbial infection in a CF patient or a patient harboring an infection contributing to CF, comprising, or alternatively consisting essentially of, or yet further consisting of contacting the biofilm with an interfering agent, thereby inhibiting, preventing or breaking down and/or clearing the microbial infection or the microbial biofilm. The patient can be an animal, mammal or human patient.

Also provided is a method for treating or preventing the recurrence of a biofilm in a CF patient or a patient at risk of developing an infection that contributes to the formation of a biofilm, the method comprising, or alternatively consisting essentially of, or yet further consisting of administering to the patient an effective amount of an interfering agent, thereby treating or preventing the recurrence of a microbial infection in the CF patient. The CF patient can be an animal, mammal or human patient.

Also provided is a method for treating or preventing the recurrence of an infection in a CF in a patient in need thereof, comprising, or alternatively consisting essentially of, or yet further consisting of administering to the patient an effective amount of an interfering agent.

The interfering agents can be combined with antimicrobials to further supplement the therapy.. Thus, any of the above methods can further comprise, or consist essentially of, or yet further consist of administration or contacting with an effective amount of the antimicrobial. Non-limiting examples of such include

For the purpose of the above noted *in vitro* and *in vivo* methods, the interfering agent and compositions are described in U.S. Patent Publication No. 2011/0236306, in particular paragraph numbers 238-263 and 277-329,are incorporated herein by reference.

In a further aspect, the methods further comprise, or alternatively consist essentially of, or yet further consist of administering to the subject an effective amount of one or more of an antimicrobial, an antigenic peptide or an adjuvant.

A non-limiting example of an antimicrobial agent is another other vaccine component such as a surface antigen, e.g. a Type IV Pilin protein (see Jurcisek and Bakaletz (2007) J. of Bacteriology 189(10):3868-3875).

The agents and compositions of this invention can be concurrently or sequentially administered with each other or other antimicrobial agents and/or surface antigens. In one particular aspect, administration is locally to the site of the infection by direct injection or by inhalation for example. Other non-limiting examples of administration include by one or more method comprising transdermally, urethrally, sublingually, rectally, vaginally, ocularly, subcutaneous, intramuscularly, intraperitoneally, intranasally, by inhalation or orally.

Microbial infections and disease that can be treated by the methods of this invention include infection that lead to or are associated with CF infection. These microbial infections may be present in the upper, mid and lower airway (otitis, sinusitis, bronchitis but also exacerbations of chronic obstructive pulmonary disease (COPD), chronic cough, complications of and/or primary cause of cystic fibrosis (CF) and community acquired pneumonia (CAP). Thus, by practicing the *in vivo* methods of this invention, these diseases and complications from these infections can also be prevented or treated.

Thus, routes of administration applicable to the methods of the invention include intranasal, intramuscular, urethrally, intratracheal, subcutaneous, intradermal, topical application, intravenous, rectal, nasal, oral, inhalation, and other enteral and parenteral routes of administration. Routes of administration may be combined, if desired, or adjusted depending upon the agent and/or the desired effect. An active agent can be administered in a single dose or in multiple doses. Embodiments of these methods and routes suitable for delivery, include systemic or localized routes. In general, routes of administration suitable for the methods of the invention include, but are not limited to, direct injection, enteral, parenteral, or inhalational routes.

Parenteral routes of administration other than inhalation administration include, but are not limited to, topical, transdermal, subcutaneous, intramuscular, intraorbital, intracapsular, intraspinal, intrasternal, and intravenous routes, i.e., any route of administration other than through the alimentary canal. Parenteral administration can be conducted to effect systemic or local delivery of the inhibiting agent. Where systemic delivery is desired, administration typically involves invasive or systemically absorbed topical or mucosal administration of pharmaceutical preparations.

The interfering agents of the invention can also be delivered to the subject by enteral administration. Enteral routes of administration include, but are not limited to, oral, urethral and rectal (e.g., using a suppository) delivery.

Methods of administration of the active through the skin or mucosa include, but are not limited to, topical application of a suitable pharmaceutical preparation, transdermal transmission, injection and epidermal administration. For transdermal transmission, absorption promoters or iontophoresis are suitable methods. Iontophoretic transmission may be accomplished using commercially available "patches" that deliver their product continuously via electric pulses through unbroken skin for periods of several days or more.

In various embodiments of the methods of the invention, the interfering agent will be administered by inhalation, injection or orally on a continuous, daily basis, at least once per day (QD), and in various embodiments two (BID), three (TID), or even four times a day. Typically, the therapeutically effective daily dose will be at least about 1 mg, or at least about 10 mg, or at least about 100 mg, or about 200 - about 500 mg, and sometimes, depending on the compound, up to as much as about 1 g to about 2.5 g.

Dosing of can be accomplished in accordance with the methods of the invention using capsules, tablets, oral suspension, suspension for intra-muscular injection, suspension for intravenous infusion, gel or cream for topical application, or suspension for intra-articular injection.

Dosage, toxicity and therapeutic efficacy of compositions described herein can be determined by standard pharmaceutical procedures in cell cultures or experimental animals, for example, to determine the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compositions which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

In some embodiments, an effective amount of a composition sufficient for achieving a therapeutic or prophylactic effect, ranges from about 0.000001 mg per kilogram body weight per administration to about 10,000 mg per kilogram body weight per administration. Suitably, the dosage ranges are from about 0.0001 mg per kilogram body weight per administration to about 100 mg per kilogram body weight per administration. Administration can be provided as an initial dose, followed by one or more "booster" doses. Booster doses can be provided a day, two days, three days, a week, two weeks, three weeks, one, two, three, six or twelve months after an initial dose. In some embodiments, a booster dose is administered after an evaluation of the subject's response to prior administrations.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The compositions and related methods of the present invention may be used in combination with the administration of other therapies. These include, but are not limited to, the administration of DNase enzymes, antibiotics, antimicrobials, or other antibodies.

The methods and compositions disclosed herein can include a deoxyribonuclease (DNase) enzyme that acts synergistically with the anti-DNABII antibody. A DNase is any enzyme that catalyzes the cleavage of phosphodiester linkages in the DNA backbone. Three non-limiting examples of DNase enzymes that are known to target not only cruciform structures, but also a variety of secondary structure of DNA include DNAse I, T4 EndoVII and T7 Endo I. In certain embodiments, the effective amount of anti-DNABII antibody needed to destabilize the biofilm is reduced when combined with a DNase. When administered *in vitro,* the DNase can be added directly to the assay or in a suitable buffer known to stabilize the enzyme. The effective unit dose of DNase and the assay conditions may vary, and can be optimized according to procedures known in the art.

The methods and compositions disclosed herein can be combined with antibiotics and/or antimicrobials. Antimicrobials are substances that kill or inhibit the growth of microorganisms such as bacteria, fungi, or protozoans. Although biofilms are generally resistant to the actions of antibiotics, compositions and methods described herein can be used to sensitize the infection involving a biofilm to traditional therapeutic methods for treating infections. In other embodiments, the use of antibiotics or antimicrobials in combination with methods and compositions described herein allow for the reduction of the effective amount of the antimicrobial and/or biofilm reducing agent. Some non-limiting examples of antimicrobials and antibiotics useful in combination with methods of the current invention include amoxicillin, amoxicillin-clavulanate, cefdinir, azithromycin, and sulfamethoxazole-trimethoprim. The therapeutically effective dose of the antimicrobial and/or antibiotic in combination with the biofilm reducing agent can be readily determined by traditional methods. In some embodiments the dose of the antimicrobial agent in combination with the biofilm reducing agent is the average effective dose which has been shown to be effective in other bacterial infections, for example, bacterial infections wherein the etiology of the infection does not include a biofilm. In other embodiments, the dose is 0.1, 0.15, 0.2, 0.25, 0,30, 0,35, 0.40, 0.45, 0.50, 0.55, 0.60, 0.65, 0.70, 0.75, 0.8, 0.85, 0.9, 0.95, 1.1, 1.2, 1.3, 1.4, 1.5, 1.6, 1.7, 1.8,1.9, 2.0, 2.5, 3.0 or 5 times the the average effective dose. The antibiotic or antimicrobial can be added prior to, concurrent with, or subsequent to the addition of the anti-DNABII antibody.

The methods and compositions disclosed herein can be combined with antibodies that treat the bacterial infection. One example of an antibody useful in combination with the methods and compositions described herein is an antibody directed against an unrelated outer membrane protein (i.e. OMP P5). Treatment with this antibody alone does not debulk a biofilm *in vitro.* Combined therapy with this antibody and a biofilm reducing agent and/or a antimicrobial results in a greater effect than that which could be achieved by either reagent used alone at the same concentration. Other antibodies that may produce a synergistic effect when combined with a biofilm reducing agent or methods to reduce a biofilm include anti-rsPilA anti-OMP26, anti-OMP P2, and anti-whole OMP preparations.

The compositions and methods described herein can be used to sensitize the bacterial infection involving a biofilm to common therapeutic modalities effective in treating bacterial infections without a biofilm but are otherwise ineffective in treating bacterial infections involving a biofilm. In other embodiments, the compositions and methods described herein can be used in combination with therapeutic modalities that are effective in treating bacterial infections involving a biofilm, but the combination of such additional therapy and biofilm reducing agent or method produces a synergistic effect such that the effective dose of either the biofilm reducing agent or the additional therapeutic agent can be reduced. In other instances the combination of such additional therapy and biofilm reducing agent or method produces a synergistic effect such that the treatment is enhanced. An enhancement of treatment can be evidenced by a shorter amount of time required to treat the infection.

The additional therapeutic treatment can be added prior to, concurrent with, or subsequent to methods or compositions used to reduce the biofilm, and can be contained within the same formation or as a separate formulation.

### Kits

Kits containing the agents and instructions necessary to perform the *in vitro* and *in vivo* methods are also disclosed herein. Accordingly, the disclosure provides kits for performing these methods as well as instructions for carrying out the methods of this invention such as collecting tissue and/or performing the screen, and/or analyzing the results, and/or administration of an effective amount of an interfering agent as defined herein. These can be used alone or in combination with other suitable antimicrobial agents.

For example, a kit can comprise, or alternatively consist essentially of, or yet further consist of any one or more agent of the group of an isolated or recombinant integration host factor (IHF) polypeptide, polynucleotide or a fragment or an equivalent of each thereof; an isolated or recombinant protein polypeptide identified in Table 1, Table 2, the Arm fragments identified in Table 2, Table 3, Table 4 a DNA binding peptide identified in FIG. 9, or a fragment or an equivalent of each thereof; an isolated or recombinant polynucleotide or polypeptide of SEQ ID NO. 1 through 33 or 341 through 348, or a fragment or an equivalent of each thereof; an isolated or recombinant C-terminal polypeptide of SEQ NO.6 through 11, 28, 29 or those identified in Table 1, Table 4 or a fragment or an equivalent of each thereof; a polypeptide that competes with an integration host factor on binding to a microbial DNA; a four-way junction polynucleotide resembling a Holliday junction, a 3 way junction polynucleotide resembling a replication fork, a polynucleotide that has inherent flexibility or bent polynucleotide; an isolated or recombinant polynucleotide encoding any one of the above noted polypeptides; an antibody that specifically recognizes or binds any one of the above noted polypeptides, or an equivalent or fragment thereof; or a small molecule that competes with the binding of a DNABII protein or polypeptide to a microbial DNA, and instructions for use. The kit can further comprising one or more of an adjuvant, an antigenic peptide or an antimicrobial. Examples of carriers include a liquid carrier, a pharmaceutically acceptable carrier, a solid phase carrier, a pharmaceutically acceptable carrier, a pharmaceutically acceptable polymer, a liposome, a micelle, an implant, a stent, a paste, a gel, a dental implant, or a medical implant.

### Polypeptides

Also provided herein are polypeptide interfering agents and compositions for use in the methods described herein, wherein the interfering agent is of the group:
(a) an isolated or recombinant integration host factor (IHF) polypeptide or a fragment or an equivalent of each thereof;
(b) an isolated or recombinant histone-like protein from *E. coli* strain U93 (HU) polypeptide or a fragment or an equivalent of each thereof;
(c) an isolated or recombinant protein polypeptide identified in Table 1, Table 2, a polypeptide comprising or consisting of the Arm fragments identified in Table 2, Table 3, Table 4 or a DNA binding peptide identified in FIG. 9, or a fragment or an equivalent of each thereof;
(d) an isolated or recombinant polypeptide of SEQ ID NO. 1 through 348, or a fragment or an equivalent thereof;
(e) an isolated or recombinant C-terminal polypeptide of SEQ ID NO. 6 through 11, 28, 29, 42 through 100, Table 1 or those C-terminal polypeptides identified in Table 4 or a fragment or an equivalent of each thereof; or
(f) a polypeptide or polynucleotide that competes with an integration host factor on binding to a microbial DNA.

In one particular aspect, the interfering agent is an isolated or recombinant DNABII polypeptide or a fragment or an equivalent of each thereof. Non-limiting examples of such are an IHF or HU alpha or beta polypeptide; an IHF .alpha. polypeptide; *Moraxella catarrhalis* HU; *E. coli* HupA, HupB, himA, himD; E. faecalis HU (such as V583), HMGB1 and those identified in Table 1.

In another aspect, the interfering agent is an isolated or recombinant polypeptide consisting essentially of an amino acid sequence selected from SEQ ID NO. 1 to 5 or 12 to 27, 30 to 35, 101-340 or a DNA binding peptide identified in FIG. 9.

In another aspect, the isolated or recombinant polypeptide comprises, or alternatively consists essentially of or yet further consists of SEQ ID NO. 1 or 2, with the proviso that the polypeptide is none of SEQ ID NO. 6 to 11, 28, 29, or 42 through 100.

In another aspect, the isolated or recombinant polypeptide comprises, or alternatively consists essentially of, or yet further consists of SEQ ID NO. 3, 4 or 5, with the proviso that the polypeptide is none of SEQ ID NO. 6 to 11, 28, 29, or 42 through 100.

In another aspect, the isolated or recombinant polypeptide comprises, or alternatively consists essentially of, or yet further consists of SEQ ID NO. 12, 14, 16, 18, 20, 22, 24, 26, 30 or 32, with the proviso that the polypeptide is none of SEQ ID NO. 6 to 11, 28, 29, or 42 through 100.

In another aspect, the isolated or recombinant polypeptide comprises, or alternatively consists essentially of, or yet further consists of SEQ ID NO. 13, 15, 17, 19, 21, 23, 25, 27, 31 33, 34, or 35 with the proviso that the polypeptide is none of SEQ ID NO. 6 to 11, 28, 29, or 42 through 100.

In another aspect, the isolated or recombinant polypeptide comprises, or alternatively consists essentially of, or yet further consists of an isolated or recombinant polypeptide of the group of:
a polypeptide comprising SEQ ID NO. 12 and 13;
a polypeptide comprising SEQ ID NO. 14 and 15;
a polypeptide comprising SEQ ID NO. 16 and 17;
a polypeptide comprising SEQ ID NO. 18 and 19;
a polypeptide comprising SEQ ID NO. 20 and 21;
a polypeptide comprising SEQ ID NO. 23 and 24;
a polypeptide comprising SEQ ID NO. 25 and 26;
a polypeptide comprising SEQ ID NO. 30 and 31;
a polypeptide comprising SEQ ID NO. 32 and 33;
a polypeptide comprising SEQ ID NO. 34 and 35;
a polypeptide comprising SEQ ID NO. 337 and 338; or
a polypeptide comprising SEQ ID NO. 339 and 340;
with the proviso that the polypeptide is none of wild-type of any one of IHF alpha, IHF beta or SEQ ID NO. 6 to 11, 28, 29, or 42 through 100.

In another aspect, the isolated or recombinant polypeptide is of the group:
a polypeptide consisting essentially of SEQ ID NO. 12 and 13;
a polypeptide consisting essentially of SEQ ID NO. 14 and 15;
a polypeptide consisting essentially of SEQ ID NO. 16 and 17;
a polypeptide consisting essentially of SEQ ID NO. 18 and 19;
a polypeptide consisting essentially of SEQ ID NO. 20 and 21;
a polypeptide consisting essentially of SEQ ID NO. 23 and 24;
a polypeptide consisting essentially of SEQ ID NO. 25 and 26;
a polypeptide consisting essentially of SEQ ID NO. 30 and 31;
a polypeptide consisting essentially of SEQ ID NO. 32 and 33;
a polypeptide consisting essentially of SEQ ID NO. 34 and 35;
a polypeptide consisting essentially of SEQ ID NO. 337 and 338; or
a polypeptide consisting essentially of SEQ ID NO. 339 and 340;
with the proviso that the polypeptide is none of wild-type of any one of IHF alpha, IHF beta or SEQ ID NO. 6 to 11, 28, 29, or 42 through 100.

Also provided herein is an isolated polynucleotide or polypeptide comprising one or more of the sequences: : TCTCAACGATTTA (SEQ ID NO. 341); WATCAANNNNTTR (where W is A or T, N is any nucleotide and R is a A or G; (SEQ ID NO. 342); MATIT**K**LDIIEYLSDKYHLS (also referred to herein as hIFA1; (SEQ ID NO. 343); KYHLSKQDTKNVVEN**FLEEI** (also referred to herein as hIFA2; (SEQ ID NO. 344); **FLEEI**RLSLESGQD**VKLSGF** (also referred to herein as hIFA3; (SEQ ID NO. 345); **KLSGFGNF**ELRDKSS**RPGRN** (also referred to herein as hIFA4; (SEQ ID NO. 346); **RPGRNPKTGDVV**PVSARRVV (also referred to herein as hIFA5; (SEQ ID NO. 347); ARRVVTFKPGQKLRARVEKTK (also referred to herein as hIFA6; (SEQ ID NO. 348), or an equivalent thereof or a polynucleotide or peptide having at least 60%, or alternatively at least 65%, or alternatively at least 70%, or alternatively at least 75%, or alternatively 80%, or alternatively at least 85%, or alternatively at least 90%, or alternatively at least 95% identity thereto or for polypeptide sequences, which is encoded by a polynucleotide or its complement that hybridizes under conditions of high stringency to a polynucleotide encoding such polypeptide sequences. Conditions of high stringency are described above and incoporrated herein by reference. Applicants have determined that the bolded and underlined amino acids are heavily conserved and therefore in one aspect, are not modified or altered in desiging an equivalent polypeptide. Additional examples of equivalent polypeptides include, for example a polypeptide consisting of or comprising the above noted polypeptides with the addition of up to 25, or alternatively 20, or alternatively 15, or alternatively up to 10, or alternatively up to 5 random amino acids on either the amine or carboxy termini (or on both). In one aspect, the isolated polypeptide is combined with one or more of a detectable label, a carrier such as a pharmaceutically acceptable carrier, or an adjuvant.

Further provided as agents for use in the methods of this invention are fragments or an equivalent of the isolated or recombinant polypeptides described above. An example of a fragment is a C-terminal polypeptide. In a further aspect, the isolated or recombinant polypeptide comprises, or alternatively consists essentially of, or yet further consists of two or more of the isolated or recombinant polypeptides described above.

For example, the isolated or recombinant polypeptide comprises, or alternatively consists essentially of, or yet further consists of any one of SEQ ID. NO. 1 to 5, 12 to 27 or 30 to 33, or a fragment or an equivalent polypeptide, examples of which are identified in Table 1 or shown in Table 2 or the Arm fragment identified in Table 2, Table 3 or Table 4. In one aspect, isolated wild-type polypeptides are excluded, i.e., that the polypeptide is none of SEQ ID NO. 6 through 11, 28, 29, or a wildtype sequence identified in Table 1 or shown in Table 2.

In one aspect, this disclosure provides an isolated or recombinant polypeptide consisting essentially of an amino acid sequence of the group SEQ ID. NO. 1 to 5, 12 to 27 or 30 to 35, 1 to 6 and 13 to 35, or a polypeptide comprising, or alternatively consisting essentially of, or yet further consisting of an amino acid corresponding to the .beta.-3 and/or .alpha.-3 fragments of a Haemophilus influenzae IHFα or IHFβ, non-limiting examples of which include SEQ ID NO. 12 through 27, or a fragment or equivalent thereof of each thereof. In another aspect, the invention provides an isolated or recombinant polypeptide comprising, or alternatively consisting essentially of, or yet further consisting of an amino acid sequence of the group SEQ ID NO. 1 to 4, or a fragment or an equivalent of each thereof, or a polypeptide comprising, or alternatively consisting essentially of, or yet further consisting of an amino acid corresponding to the .beta.-3 and/or .alpha.-3 fragments of a Haemophilus influenzae IHF.alpha. or IHF.beta., non-limiting examples of which include SEQ ID NO. 12 through 27 or a fragment or a biological equivalent thereof which further comprises independently at least 2, or alternatively at least 3, or alternatively at least 4, or alternatively at least 5, or at least 6, or alternatively at least 7, or alternatively at least 8, or alternatively at least 9 or alternatively at least 10 amino acids at the amino and/or carboxyl terminus of the polypeptide. In one aspect, isolated wildtype DNA binding polypeptides are excluded, i.e., that the polypeptide is none of SEQ ID NO. 6 through 11, 28, 29, or 42 through 100 or an isolated wildtype polypeptide sequence listed in Table 1 or shown in Table 2.

In another aspect, this disclosure provides an isolated or recombinant polypeptide comprising, or alternatively consisting essentially of, or yet further consisting of, SEQ ID. NO 1 or 2 alone or in combination with a polypeptide comprising, or alternatively consisting essentially of, or yet further consisting of an amino acid corresponding to the .beta.-3 and/or α-3 fragments of a Haemophilus influenzae IHF-α or IHF.beta., on-limiting examples of which include SEQ ID Nos. 12 through 27 or a fragment or a biological equivalent of each thereof. In one aspect, isolated wildtype DNA binding polypeptides are excluded, i.e., that the polypeptide is none of SEQ ID NO. 6 through 11, 28, 29, or 42 through 100 or an isolated polypeptide sequence listed in Table 1 or shown in Table 2.

In a yet further aspect, this disclosure provides an isolated or recombinant polypeptide comprising or alternatively consisting essentially of, or yet further consisting of, SEQ ID NO. 3 or 4 or a fragment or an equivalent of each thereof alone or in combination with a polypeptide comprising, or alternatively consisting essentially of, or yet further consisting of an amino acid corresponding to the .beta.-3 and/or α-3 fragments of a Haemophilus influenzae IHF-α or IHF.beta., non-limiting examples of which include SEQ ID NO. 12 through 27, and 34-35 or a biological equivalent of each thereof. In one aspect, isolated wildtype DNA binding polypeptides are excluded, i.e., that the polypeptide is none of SEQ ID NO. 6 through 11, 28, 29, or 42 through 100 or an isolated wildtype polypeptide sequence listed in Table 1 or shown in Table 2.

This disclosure also provides isolated or recombinant polypeptides comprising or alternatively consisting essentially of, or yet further consisting of, two or more, or three or more, four or more, five or more, six or more, seven or more, eight or more, nine or more, ten or more, eleven or more, twelve or more, thirteen or more of all fourteen of the isolated polypeptides or a fragment or an equivalent of each thereof. Examples of such include isolated or recombinant polypeptides comprising SEQ ID NO. 1 through 4, e.g., SEQ ID NO. 1 and 2, or alternatively 1 and 3 or alternatively 1 and 4, or alternatively 2 and 3, or alternatively SEQ ID NO. 1, 2 and 3 or alternatively, 2, 3 and 4, or alternatively 1, 3 and 4. The polypeptides can be in any orientation, e.g., SEQ ID NO. 1, 2, and 3 or SEQ ID NO. 3, 2 and 1 or alternatively 2, 1 and 3, or alternatively, 3, 1 and 2. Biological equivalents of these polypeptides are further included in this invention with the proviso that the sequences do not include isolated wildtype sequences such as those identified in Tables 1, 2 and 3.

In another aspect, this disclosure provides an isolated or recombinant polypeptide comprising or alternatively consisting essentially of, or yet further consisting of, SEQ ID NO. 1 or 2 and 3 or 4, or a fragment or an equivalent of each thereof, with the proviso that the polypeptide is none of SEQ ID NO. 6 through 11, and they may further comprise any one or more of SEQ ID Nos. 11 through 26, e.g., 11 and 12, or alternatively 1 and 11, or alternatively 2 and 11, or alternatively, 1 and 12, or alternatively 2 and 12, or alternatively 11, 12 and 1, or alternatively 2, 11 and 12. In this example SEQ ID NO. 1 or 2 is located upstream or amino terminus from SEQ ID NO. 3 or 4, with the proviso that the amino acid sequence is not an isolated wildtype polypeptide, e.g., none of SEQ ID NO. 6 through 11, 28 and 29. In another aspect, the isolated polypeptide comprises SEQ ID NO. 3 or 4 located upstream or amino terminus to SEQ ID NO. 1 or 2. Biological equivalents of these polypeptides are further included in this disclosure with the proviso that the sequence do not include isolated wildtype polypeptides.

In any of the above examples a peptide linker can be added to the N-terminus or C-terminus of the polypeptide. A "linker" or "peptide linker" refers to a peptide sequence linked to either the N-terminus or the C-terminus of a polypeptide sequence. In one aspect, the linker is from about 1 to about 20 amino acid residues long or alternatively 2 to about 10, about 3 to about 5 amino acid residues long. An example of a peptide linker is Gly-Pro-Ser-Leu-Lys-Leu (SEQ ID NO: 37). Other examples include Gly-Gly-Gly; Gly-Pro-Ser-Leu (SEQ ID NO: 38); Gly-Pro-Ser; Pro-Ser-Leu-Lys (SEQ ID NO: 39); Gly-Pro-Ser-Leu-Lys (SEQ ID NO: 40) and Ser-Leu-Lys-Leu (SEQ ID NO: 41).

The isolated polypeptides disclosed herein are intended to include isolated wildtype and recombinantly produced polypeptides and proteins from prokaryotic and eukaryotic host cells, as well as muteins, analogs and fragments thereof, examples of such cells are described above. In some embodiments, the term also includes antibodies and anti-idiotypic antibodies as described herein. Such polypeptides can be isolated or produced using the methods known in the art and briefly described herein.

In a further aspect, the polypeptides are conjugated or linked to a detectable label. Suitable labels are known in the art and described herein.

In a yet further aspect, the polypeptides with or without a detectable label can be contained or expressed on the surface of a host prokaryotic or eukaryotic host cell, such as a dendritic cell.

The proteins and polypeptides are obtainable by a number of processes known to those of skill in the art, which include purification, chemical synthesis and recombinant methods. Polypeptides can be isolated from preparations such as host cell systems by methods such as immunoprecipitation with antibody, and standard techniques such as gel filtration, ion-exchange, reversed-phase, and affinity chromatography. For such methodology, see for example Deutscher et al. (1999) Guide To Protein Purification: Methods In Enzymology (Vol. 182, Academic Press). Accordingly, this invention also provides the processes for obtaining these polypeptides as well as the products obtainable and obtained by these processes.

The polypeptides also can be obtained by chemical synthesis using a commercially available automated peptide synthesizer such as those manufactured by Perkin/Elmer/Applied Biosystems, Inc., Model 430A or 431A, Foster City, Calif., USA. The synthesized polypeptide can be precipitated and further purified, for example by high performance liquid chromatography (HPLC). Accordingly, a process for chemically synthesizing the proteins of this invention by providing the sequence of the protein and reagents, such as amino acids and enzymes and linking together the amino acids in the proper orientation and linear sequence can also be used.

Alternatively, the proteins and polypeptides can be obtained by well-known recombinant methods as described, for example, in Sambrook et al. (1989) supra, using a host cell and vector systems described herein.

Also provided by this application are the polypeptides described herein conjugated to a detectable agent for use in the diagnostic methods. For example, detectably labeled polypeptides can be bound to a column and used for the detection and purification of antibodies. They also are useful as immunogens for the production of antibodies as described below. The polypeptides of this invention may also be used in an *in vitro* assay system to screen for agents or drugs, which modulate cellular processes.

As used herein the term "amino acid" refers to either natural and/or unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is commonly called a polypeptide or a protein.

Peptides of the invention can be modified to include unnatural amino acids. Thus, the peptides may comprise D-amino acids, a combination of and L-amino acids, and various "designer" amino acids (e.g., .beta.-methyl amino acids, C-α.-methyl amino acids, and N-α-methyl amino acids, etc.) to convey special properties to peptides. Additionally, by assigning specific amino acids at specific coupling steps, peptides with α-helices .beta. turns, .beta. sheets, .gamma.-turns, and cyclic peptides can be generated. Generally, it is believed that α-helical secondary structure or random secondary structure is preferred.

The polypeptides of this invention also can be combined with various solid phase carriers, such as an implant, a stent, a paste, a gel, a dental implant, or a medical implant or liquid phase carriers, such as beads, sterile or aqueous solutions, pharmaceutically acceptable carriers, pharmaceutically acceptable polymers, liposomes, micelles, suspensions and emulsions. Examples of non-aqueous solvents include propyl ethylene glycol, polyethylene glycol and vegetable oils. When used to prepare antibodies or induce an immune response *in vivo*, the carriers also can include an adjuvant that is useful to non-specifically augment a specific immune response. A skilled artisan can easily determine whether an adjuvant is required and select one. However, for the purpose of illustration only, suitable adjuvants include, but are not limited to Freund's Complete and Incomplete, mineral salts and polynucleotides. Other suitable adjuvants include monophosphoryl lipid A (MPL), mutant derivatives of the heat labile enterotoxin of *E. coli*, mutant derivatives of cholera toxin, CPG oligonucleotides, and adjuvants derived from squalene.

This disclosure also provides a pharmaceutical composition comprising or alternatively consisting essentially of, or yet further consisting of, any of a polypeptide, analog, mutein, or fragment of this invention, alone or in combination with each other or other agents, such an antibiotic and an acceptable carrier or solid support. These compositions are useful for various diagnostic and therapeutic methods as described herein.

### Polynucleotides

This disclosure also provides isolated or recombinant polynucleotides encoding one or more of the above-identified isolated or recombinant polypeptides and their respective complementary strands. Vectors comprising the isolated or recombinant polynucleotides are further provided examples of which are known in the art and briefly described herein. In one aspect where more than one isolated or recombinant polynucleotide is to be expressed as a single unit, the isolated or recombinant polynucleotides can be contained within a polycistronic vector. The polynucleotides can be DNA, RNA, mRNA or interfering RNA, such as siRNA, miRNA or dsRNA.

In another aspect, this disclosure provides an interfering agent that is a four-way junction polynucleotide resembling a Holliday junction, a 3 way junction polynucleotide resembling a replication fork, a polynucleotide that has inherent flexibility or bent polynucleotide which can treat or inhibit DNA BII polynucleotide from binding to microbial DNA as well treat, prevent or inhibit biofilm formation and associated infections and disorders. One of skill in the art can make such polynucleotides using the information provided herein and knowledge of those of skill in the art. See Goodman and Kay (1999) J. Biological Chem. 274(52):37004-37011 and Kamashev and Rouviere-Yaniv (2000) EMBO J. 19(23):6527-6535.

The disclosure further provides the isolated or recombinant polynucleotide operatively linked to a promoter of RNA transcription, as well as other regulatory sequences for replication and/or transient or stable expression of the DNA or RNA. As used herein, the term "operatively linked" means positioned in such a manner that the promoter will direct transcription of RNA off the DNA molecule. Examples of such promoters are SP6, T4 and T7. In certain embodiments, cell-specific promoters are used for cell-specific expression of the inserted polynucleotide. Vectors which contain a promoter or a promoter/enhancer, with termination codons and selectable marker sequences, as well as a cloning site into which an inserted piece of DNA can be operatively linked to that promoter are known in the art and commercially available. For general methodology and cloning strategies, see Gene Expression Technology (Goeddel ed., Academic Press, Inc. (1991)) and references cited therein and Vectors: Essential Data Series (Gacesa and Ramji, eds., John Wiley & Sons, N.Y. (1994)) which contains maps, functional properties, commercial suppliers and a reference to GenEMBL accession numbers for various suitable vectors.

A polynucleotide derived from the polynucleotides of the invention may encode polypeptides or proteins having diagnostic and therapeutic utilities as described herein as well as probes to identify transcripts of the protein that may or may not be present. These nucleic acid fragments can be prepared, for example, by restriction enzyme digestion of larger polynucleotides and then labeled with a detectable marker. Alternatively, random fragments can be generated using nick translation of the molecule. For methodology for the preparation and labeling of such fragments, see Sambrook, et al. (1989) supra.

Expression vectors containing these nucleic acids are useful to obtain host vector systems to produce proteins and polypeptides. It is implied that these expression vectors must be replicable in the host organisms either as episomes or as an integral part of the chromosomal DNA. Non-limiting examples of suitable expression vectors include plasmids, yeast vectors, viral vectors and liposomes. Adenoviral vectors are particularly useful for introducing genes into tissues *in vivo* because of their high levels of expression and efficient transformation of cells both *in vitro* and *in vivo.* When a nucleic acid is inserted into a suitable host cell, e.g., a prokaryotic or a eukaryotic cell and the host cell replicates, the protein can be recombinantly produced. Suitable host cells will depend on the vector and can include mammalian cells, animal cells, human cells, simian cells, insect cells, yeast cells, and bacterial cells constructed using known methods. See Sambrook, et al. (1989) supra. In addition to the use of viral vector for insertion of exogenous nucleic acid into cells, the nucleic acid can be inserted into the host cell by methods known in the art such as transformation for bacterial cells; transfection using calcium phosphate precipitation for mammalian cells; or DEAE-dextran; electroporation; or microinjection. See, Sambrook et al. (1989) supra, for methodology. Thus, this invention also provides a host cell, e.g. a mammalian cell, an animal cell (rat or mouse), a human cell, or a prokaryotic cell such as a bacterial cell, containing a polynucleotide encoding a protein or polypeptide or antibody.

When the vectors are used for gene therapy *in vivo* or *ex vivo*, a pharmaceutically acceptable vector is preferred, such as a replication-incompetent retroviral or adenoviral vector. Pharmaceutically acceptable vectors containing the nucleic acids of this invention can be further modified for transient or stable expression of the inserted polynucleotide. As used herein, the term "pharmaceutically acceptable vector" includes, but is not limited to, a vector or delivery vehicle having the ability to selectively target and introduce the nucleic acid into dividing cells. An example of such a vector is a "replication-incompetent" vector defined by its inability to produce viral proteins, precluding spread of the vector in the infected host cell. An example of a replication-incompetent retroviral vector is LNL6 (Miller et al. (1989) BioTechniques 7:980-990). The methodology of using replication-incompetent retroviruses for retroviral-mediated gene transfer of gene markers has been established. (Bordignon (1989) PNAS USA 86:8912-8952; Culver (1991) PNAS USA 88:3155; and Rill (1991) Blood 79(10):2694-2700).

Genetically modified cells that contain and/or express the polynucleotides of this invention may be generated. The genetically modified cells can be produced by insertion of upstream regulatory sequences such as promoters or gene activators (see, U.S. Patent No. 5,733,761).

The polynucleotides can be conjugated to a detectable marker, e.g., an enzymatic label or a radioisotope for detection of nucleic acid and/or expression of the gene in a cell. A wide variety of appropriate detectable markers are known in the art, including fluorescent, radioactive, enzymatic or other ligands, such as avidin/biotin, which are capable of giving a detectable signal. In one aspect, one will likely desire to employ a fluorescent label or an enzyme tag, such as urease, alkaline phosphatase or peroxidase, instead of radioactive or other environmentally undesirable reagents. In the case of enzyme tags, calorimetric indicator substrates can be employed to provide a means visible to the human eye or spectrophotometrically, to identify specific hybridization with complementary nucleic acid-containing samples. Thus, this invention further provides a method for detecting a single-stranded polynucleotide or its complement, by contacting target single-stranded polynucleotide with a labeled, single-stranded polynucleotide (a probe) which is a portion of the polynucleotide of this invention under conditions permitting hybridization (preferably moderately stringent hybridization conditions) of complementary single-stranded polynucleotides, or more preferably, under highly stringent hybridization conditions. Hybridized polynucleotide pairs are separated from un-hybridized, single-stranded polynucleotides. The hybridized polynucleotide pairs are detected using methods known to those of skill in the art and set forth, for example, in Sambrook et al. (1989) supra.

The polynucleotide embodied in this invention can be obtained using chemical synthesis, recombinant cloning methods, PCR, or any combination thereof. Methods of chemical polynucleotide synthesis are known in the art and need not be described in detail herein. One of skill in the art can use the sequence data provided herein to obtain a desired polynucleotide by employing a DNA synthesizer or ordering from a commercial service.

The polynucleotides of this invention can be isolated or replicated using PCR. The PCR technology is the subject matter of U.S. Patent Nos. 4,683,195; 4,800,159; 4,754,065; and 4,683,202 and described in PCR: The Polymerase Chain Reaction (Mullis et al. eds., Birkhauser Press, Boston (1994)) or MacPherson et al. (1991) and (1995) supra, and references cited therein. Alternatively, one of skill in the art can use the sequences provided herein and a commercial DNA synthesizer to replicate the DNA. Accordingly, this disclosure also provides a process for obtaining the polynucleotides of this invention by providing the linear sequence of the polynucleotide, nucleotides, appropriate primer molecules, chemicals such as enzymes and instructions for their replication and chemically replicating or linking the nucleotides in the proper orientation to obtain the polynucleotides. In a separate embodiment, these polynucleotides are further isolated. Still further, one of skill in the art can insert the polynucleotide into a suitable replication vector and insert the vector into a suitable host cell (prokaryotic or eukaryotic) for replication and amplification. The DNA so amplified can be isolated from the cell by methods known to those of skill in the art. A process for obtaining polynucleotides by this method is further provided herein as well as the polynucleotides so obtained.

RNA can be obtained by first inserting a DNA polynucleotide into a suitable host cell. The DNA can be delivered by any appropriate method, e.g., by the use of an appropriate gene delivery vehicle (e.g., liposome, plasmid or vector) or by electroporation. When the cell replicates and the DNA is transcribed into RNA; the RNA can then be isolated using methods known to those of skill in the art, for example, as set forth in Sambrook et al. (1989) supra. For instance, mRNA can be isolated using various lytic enzymes or chemical solutions according to the procedures set forth in Sambrook et al. (1989) supra, or extracted by nucleic-acid-binding resins following the accompanying instructions provided by manufactures.

Polynucleotides exhibiting sequence complementarity or homology to a polynucleotide of this invention are useful as hybridization probes. Since the full coding sequence of the transcript is known, any portion of this sequence or homologous sequences, can be used in the methods disclosed herein.

It is known in the art that a "perfectly matched" probe is not needed for a specific hybridization. Minor changes in probe sequence achieved by substitution, deletion or insertion of a small number of bases do not affect the hybridization specificity. In general, as much as 20% base-pair mismatch (when optimally aligned) can be tolerated. Preferably, a probe useful for detecting the aforementioned mRNA is at least about 80% identical to the homologous region. More preferably, the probe is 85% identical to the corresponding gene sequence after alignment of the homologous region; even more preferably, it exhibits 90% identity.

These probes can be used in radioassays (e.g. Southern and Northern blot analysis) to detect, prognose, diagnose or monitor various cells or tissues containing these cells. The probes also can be attached to a solid support or an array such as a chip for use in high throughput screening assays for the detection of expression of the gene corresponding a polynucleotide disclosed herein. Accordingly, this disclosure also provides a probe comprising or corresponding to a polynucleotide disclosed herein, or its equivalent, or its complement, or a fragment thereof, attached to a solid support for use in high throughput screens.

The total size of fragment, as well as the size of the complementary stretches, will depend on the intended use or application of the particular nucleic acid segment. Smaller fragments will generally find use in hybridization embodiments, wherein the length of the complementary region may be varied, such as between at least 5 to 10 to about 100 nucleotides, or even full length according to the complementary sequences one wishes to detect.

Nucleotide probes having complementary sequences over stretches greater than 5 to 10 nucleotides in length are generally preferred, so as to increase stability and selectivity of the hybrid, and thereby improving the specificity of particular hybrid molecules obtained. More preferably, one can design polynucleotides having gene-complementary stretches of 10 or more or more than 50 nucleotides in length, or even longer where desired. Such fragments may be readily prepared by, for example, directly synthesizing the fragment by chemical means, by application of nucleic acid reproduction technology, such as the PCR technology with two priming oligonucleotides as described in U.S. Patent No. 4,603,102 or by introducing selected sequences into recombinant vectors for recombinant production. In one aspect, a probe is about 50-75 or more alternatively, 50-100, nucleotides in length.

The polynucleotides of the present invention can serve as primers for the detection of genes or gene transcripts that are expressed in cells described herein. In this context, amplification means any method employing a primer-dependent polymerase capable of replicating a target sequence with reasonable fidelity. Amplification may be carried out by natural or recombinant DNA-polymerases such as T7 DNA polymerase, Klenow fragment of *E. coli* DNA polymerase, and reverse transcriptase. For illustration purposes only, a primer is the same length as that identified for probes.

One method to amplify polynucleotides is PCR and kits for PCR amplification are commercially available. After amplification, the resulting DNA fragments can be detected by any appropriate method known in the art, e.g., by agarose gel electrophoresis followed by visualization with ethidium bromide staining and ultraviolet illumination.

Methods for administering an effective amount of a gene delivery vector or vehicle to a cell have been developed and are known to those skilled in the art and described herein. Methods for detecting gene expression in a cell are known in the art and include techniques such as in hybridization to DNA microarrays, in situ hybridization, PCR, RNase protection assays and Northern blot analysis. Such methods are useful to detect and quantify expression of the gene in a cell. Alternatively expression of the encoded polypeptide can be detected by various methods. In particular it is useful to prepare polyclonal or monoclonal antibodies that are specifically reactive with the target polypeptide. Such antibodies are useful for visualizing cells that express the polypeptide using techniques such as immunohistology, ELISA, and Western blotting. These techniques can be used to determine expression level of the expressed polynucleotide.

### Antibodies and Derivatives Thereof

This invention also provides an antibody that binds and/or specifically recognizes and binds an isolated polypeptide for use in the methods of the invention. The antibody can be any of the various antibodies described herein, non-limiting examples of such include a polyclonal antibody, a monoclonal antibody, a chimeric antibody, a human antibody, a veneered antibody, a diabody, a humanized antibody, an antibody derivative, a recombinant humanized antibody, or a derivative or antigen binding fragment thereof. In one aspect, the fragment comprises, or alternatively consists essentially of, or yet further consists of the CDR of the antibody. In one aspect, the antibody is detectably labeled or further comprises a detectable label conjugated to it. Also provided is a hybridoma cell line that produces a monoclonal antibody of this invention. Compositions comprising or alternatively consisting essentially of or yet further, consisting of one or more of the above embodiments are further provided herein. Further provided are polynucleotides that encode the amino acid sequence of the antibodies and fragments as well as methods to recombinantly produce the antibody polypeptides and fragments thereof. The antibody polypeptides can be produced in a eukaryotic or prokaryotic cell, or by other methods known in the art and described herein.

Antibodies can be generated using conventional techniques known in the art and are well-described in the literature. Several methodologies exist for production of polyclonal antibodies. For example, polyclonal antibodies are typically produced by immunization of a suitable mammal such as, but not limited to, chickens, goats, guinea pigs, hamsters, horses, mice, rats, and rabbits. An antigen is injected into the mammal, which induces the B-lymphocytes to produce immunoglobulins specific for the antigen. Immunoglobulins may be purified from the mammal's serum. Antibodies specific to IHFα and IHFβ can be generated by injection of polypeptides corresponding to different epitopes of IHFα and IHFβ. For example, antibodies can be generated using the 20 amino acids of each subunit such as TFRPGQKLKSRVENASPKDE (SEQ ID NO. 34) for IHFα and KYVPHFKPGKELRDRANIYG (SEQ ID No. 35) for IHFβ, or alternatively an antibody that specifically recognizes and binds a polynucleotide or peptide comprising one or more of the sequences: : TCTCAACGATTTA (SEQ ID NO. 341); WATCAANNNNTTR (where W is A or T, N is any nucleotide and R is a A or G; (SEQ ID NO. 342); MATIT**K**LDIIEYLSDKYHLS (also referred to herein as hIFA1; (SEQ ID NO. 343); KYHLSKQDTKNVVEN**FLEEI** (also referred to herein as hIFA2; (SEQ ID NO. 344); **FLEEI**RLSLESGQD**VKLSGF** (also referred to herein as hIFA3; (SEQ ID NO. 345); **KLSGFGNF**ELRDKSS**RPGRN** (also referred to herein as hIFA4; (SEQ ID NO. 346); **RPGRNPKTGDVV**PVSARRVV (also referred to herein as hIFA5; (SEQ ID NO. 347); ARRVVTFKPGQKLRARVEKTK (also referred to herein as hIFA6; (SEQ ID NO. 348), or an equivalent thereof or a polynucleotide or peptide having at least 60%, or alternatively at least 65%, or alternatively at least 70%, or alternatively at least 75%, or alternatively 80%, or alternatively at least 85%, or alternatively at least 90%, or alternatively at least 95% identity thereto or for polypeptide sequences, which is encoded by a polynucleotide or its complement that hybridizes under conditions of high stringency to a polynucleotide encoding such polypeptide sequences. Conditions of high stringency are described above and incoporrated herein by reference. Applicants have determined that the bolded and underlined amino acids are heavily conserved and therefore in one aspect, are not modified or altered in desiging an equivalent polypeptide. Additional examples of equivalent polypeptides include, for example a polypeptide consisting of or comprising the above noted polypeptides with the addition of up to 25, or alternatively 20, or alternatively 15, or alternatively up to 10, or alternatively up to 5 random amino acids on either the amine or carboxy termini (or on both)..Additional examples include antibodies that specifically reconginze and bind the polypeptide identified in Table 2 (and incorporated herein by reference). Variations of this methodology include modification of adjuvants, routes and site of administration, injection volumes per site and the number of sites per animal for optimal production and humane treatment of the animal. For example, adjuvants typically are used to improve or enhance an immune response to antigens. Most adjuvants provide for an injection site antigen depot, which allows for a slow release of antigen into draining lymph nodes. Other adjuvants include surfactants which promote concentration of protein antigen molecules over a large surface area and immunostimulatory molecules. Non-limiting examples of adjuvants for polyclonal antibody generation include Freund's adjuvants, Ribi adjuvant system, and Titermax. Polyclonal antibodies can be generated using methods known in the art some of which are described in U.S. Patent Nos. 7,279,559; 7,119,179; 7,060,800; 6,709,659; 6,656,746; 6,322,788; 5,686,073; and 5,670,153.

Monoclonal antibodies can be generated using conventional hybridoma techniques known in the art and well-described in the literature. For example, a hybridoma is produced by fusing a suitable immortal cell line (e.g., a myeloma cell line such as, but not limited to, Sp2/0, Sp2/0-AG14, NSO, NS1, NS2, AE-1, L.5, P3X63Ag8.653, Sp2 SA3, Sp2 MAI, Sp2 SS1, Sp2 SA5, U397, MLA 144, ACT IV, MOLT4, DA-1, JURKAT, WEHI, K-562, COS, RAJI, NIH 3T3, HL-60, MLA 144, NAMAIWA, NEURO 2A, CHO, PerC.6, YB2/O) or the like, or heteromyelomas, fusion products thereof, or any cell or fusion cell derived there from, or any other suitable cell line as known in the art (see, those at the following web addresses e.g., atcc.org, lifetech.com., last accessed on November 26, 2007), with antibody producing cells, such as, but not limited to, isolated or cloned spleen, peripheral blood, lymph, tonsil, or other immune or B cell containing cells, or any other cells expressing heavy or light chain constant or variable or framework or CDR sequences, either as endogenous or heterologous nucleic acid, as recombinant or endogenous, viral, bacterial, algal, prokaryotic, amphibian, insect, reptilian, fish, mammalian, rodent, equine, ovine, goat, sheep, primate, eukaryotic, genomic DNA, cDNA, rDNA, mitochondrial DNA or RNA, chloroplast DNA or RNA, hnRNA, mRNA, tRNA, single, double or triple stranded, hybridized, and the like or any combination thereof. Antibody producing cells can also be obtained from the peripheral blood or, preferably the spleen or lymph nodes, of humans or other suitable animals that have been immunized with the antigen of interest. Any other suitable host cell can also be used for expressing-heterologous or endogenous nucleic acid encoding an antibody, specified fragment or variant thereof, of the present invention. The fused cells (hybridomas) or recombinant cells can be isolated using selective culture conditions or other suitable known methods, and cloned by limiting dilution or cell sorting, or other known methods.

Other suitable methods of producing or isolating antibodies of the requisite specificity can be used, including, but not limited to, methods that select recombinant antibody from a peptide or protein library (e.g., but not limited to, a bacteriophage, ribosome, oligonucleotide, RNA, cDNA, or the like, display library; e.g., as available from various commercial vendors such as MorphoSys (Martinsreid/Planegg, Del.), Biolnvent (Lund, Sweden), Affitech (Oslo, Norway) using methods known in the art. Art known methods are described in the patent literature some of which include U.S. Patent Nos. 4,704,692; 5,723,323; 5,763,192; 5,814,476; 5,817,483; 5,824,514; 5,976,862. Alternative methods rely upon immunization of transgenic animals (e.g., SCID mice, Nguyen et al. (1977) Microbiol. Immunol. 41:901-907 (1997); Sandhu et al. (1996) Crit. Rev. Biotechnol. 16:95-118; Eren et al. (1998) Immunol. 93:154-161 that are capable of producing a repertoire of human antibodies, as known in the art and/or as described herein. Such techniques, include, but are not limited to, ribosome display (Hanes et al. (1997) Proc. Natl. Acad. Sci. USA, 94:4937-4942; Hanes et al. (1998) Proc. Natl. Acad. Sci. USA 95:14130-14135); single cell antibody producing technologies (e.g., selected lymphocyte antibody method ("SLAM") (U.S. Patent No. 5,627,052, Wen et al. (1987) J. Immunol. 17:887-892; Babcook et al. (1996) Proc. Natl. Acad. Sci. USA 93:7843-7848); gel microdroplet and flow cytometry (Powell et al. (1990) Biotechnol. 8:333-337; One Cell Systems, (Cambridge, Mass).; Gray et al. (1995) J. Imm. Meth. 182:155-163; and Kenny et al. (1995) Bio. Technol. 13:787-790); B-cell selection (Steenbakkers et al. (1994) Molec. Biol. Reports 19:125-134).

Antibody derivatives of the present invention can also be prepared by delivering a polynucleotide encoding an antibody of this invention to a suitable host such as to provide transgenic animals or mammals, such as goats, cows, horses, sheep, and the like, that produce such antibodies in their milk. These methods are known in the art and are described for example in U.S. Patent Nos. 5,827,690; 5,849,992; 4,873,316; 5,849,992; 5,994,616; 5,565,362; and 5,304,489.

The term "antibody derivative" includes post-translational modification to linear polypeptide sequence of the antibody or fragment. For example, U.S. Patent No. 6,602,684 B1 describes a method for the generation of modified glycol-forms of antibodies, including whole antibody molecules, antibody fragments, or fusion proteins that include a region equivalent to the Fc region of an immunoglobulin, having enhanced Fc-mediated cellular toxicity, and glycoproteins so generated.

The antibodies disclosed herein may include derivatives that are modified by the covalent attachment of any type of molecule to the antibody such that covalent attachment does not prevent the antibody from generating an anti-idiotypic response. Antibody derivatives include, but are not limited to, antibodies that have been modified by glycosylation, acetylation, pegylation, phosphorylation, amidation, derivatization by known protecting/blocking groups, proteolytic cleavage, linkage to a cellular ligand or other protein, etc. Additionally, the derivatives may contain one or more non-classical amino acids.

Antibody derivatives also can be prepared by delivering a polynucleotide of this invention to provide transgenic plants and cultured plant cells (e.g., but not limited to tobacco, maize, and duckweed) that produce such antibodies, specified portions or variants in the plant parts or in cells cultured therefrom. For example, Cramer et al. (1999) Curr. Top. Microbol. Immunol. 240:95-118 and references cited therein, describe the production of transgenic tobacco leaves expressing large amounts of recombinant proteins, e.g., using an inducible promoter. Transgenic maize have been used to express mammalian proteins at commercial production levels, with biological activities equivalent to those produced in other recombinant systems or purified from natural sources. See, e.g., Hood et al. (1999) Adv. Exp. Med. Biol. 464:127-147 and references cited therein. Antibody derivatives have also been produced in large amounts from transgenic plant seeds including antibody fragments, such as single chain antibodies (scFv's), including tobacco seeds and potato tubers. See, e.g., Conrad et al.(1998) Plant Mol. Biol. 38:101-109 and references cited therein. Thus, antibodies can also be produced using transgenic plants, according to know methods.

Antibody derivatives also can be produced, for example, by adding exogenous sequences to modify immunogenicity or reduce, enhance or modify binding, affinity, on-rate, off-rate, avidity, specificity, half-life, or any other suitable characteristic. Generally part or all of the non-human or human CDR sequences are maintained while the non-human sequences of the variable and constant regions are replaced with human or other amino acids.

In general, the CDR residues are directly and most substantially involved in influencing antigen binding. Humanization or engineering of antibodies can be performed using any known method such as, but not limited to, those described in U.S. Patent Nos. 5,723,323; 5,976,862; 5,824,514; 5,817,483; 5,814,476; 5,763,192; 5,723,323; 5,766,886; 5,714,352; 6,204,023; 6,180,370; 5,693,762; 5,530,101; 5,585,089; 5,225,539; and 4,816,567.

Chimeric, humanized or primatized antibodies of the present invention can be prepared based on the sequence of a murine monoclonal antibody prepared using standard molecular biology techniques. DNA encoding the heavy and light chain immunoglobulins can be obtained from the murine hybridoma of interest and engineered to contain non-murine (e.g., human) immunoglobulin sequences using standard molecular biology techniques. For example, to create a chimeric antibody, the murine variable regions can be linked to human constant regions using methods known in the art (U.S. Patent No. 4,816,567). To create a humanized antibody, the murine CDR regions can be inserted into a human framework using methods known in the art (U.S. Patent No. 5,225,539 and U.S. Patents Nos. 5,530,101; 5,585,089; 5,693,762 and 6,180,370). Similarly, to create a primatized antibody the murine CDR regions can be inserted into a primate framework using methods known in the art (WO 93/02108 and WO 99/55369).

The antibodies disclosed herein also can be modified to create chimeric antibodies. Chimeric antibodies are those in which the various domains of the antibodies' heavy and light chains are coded for by DNA from more than one species. See, e.g., U.S. Patent No. 4,816,567.

Alternatively, the antibodies of this invention can also be modified to create veneered antibodies. Veneered antibodies are those in which the exterior amino acid residues of the antibody of one species are judiciously replaced or "veneered" with those of a second species so that the antibodies of the first species will not be immunogenic in the second species thereby reducing the immunogenicity of the antibody. Since the antigenicity of a protein is primarily dependent on the nature of its surface, the immunogenicity of an antibody could be reduced by replacing the exposed residues which differ from those usually found in another mammalian species antibodies. This judicious replacement of exterior residues should have little, or no, effect on the interior domains, or on the interdomain contacts. Thus, ligand binding properties should be unaffected as a consequence of alterations which are limited to the variable region framework residues. The process is referred to as "veneering" since only the outer surface or skin of the antibody is altered, the supporting residues remain undisturbed.

The procedure for "veneering" makes use of the available sequence data for human antibody variable domains compiled by Kabat et al. (1987) Sequences of Proteins of Immunological Interest, 4th ed., Bethesda, Md., National Institutes of Health, updates to this database, and other accessible U.S. and foreign databases (both nucleic acid and protein). Non-limiting examples of the methods used to generate veneered antibodies include EP 519596; U.S. Patent No. 6,797,492; and described in Padlan et al. (1991) Mol. Immunol. 28(4-5):489-498.

The term "antibody derivative" also includes "diabodies" which are small antibody fragments with two antigen-binding sites, wherein fragments comprise a heavy chain variable domain (VH) connected to a light chain variable domain (VL) in the same polypeptide chain. (See for example, EP 404,097; WO 93/11161; and Hollinger et al. (1993) Proc. Natl. Acad. Sci. USA 90:6444-6448.) By using a linker that is too short to allow pairing between the two domains on the same chain, the domains are forced to pair with the complementary domains of another chain and create two antigen-binding sites. (See also, U.S. Patent No. 6,632,926 to Chen et al. which discloses antibody variants that have one or more amino acids inserted into a hypervariable region of the parent antibody and a binding affinity for a target antigen which is at least about two fold stronger than the binding affinity of the parent antibody for the antigen).

The term "antibody derivative" further includes engineered antibody molecules, fragments and single domains such as scFv, dAbs, nanobodies, minibodies, Unibodies, and Affibodies (Holliger & Hudson (2005) Nature Biotech 23(9):1126-36; U.S. Patent Publication No. US 2006/0211088; PCT Publication No. WO2007/059782; US Patent No. 5,831,012).

The term "antibody derivative" further includes "linear antibodies". The procedure for making linear antibodies is known in the art and described in Zapata et al. (1995) Protein Eng. 8(10):1057-1062. Briefly, these antibodies comprise a pair of tandem Fd segments (V_{H} -C_{H} 1-VH -C_{H}1) which form a pair of antigen binding regions. Linear antibodies can be bispecific or monospecific.

The antibodies of this invention can be recovered and purified from recombinant cell cultures by known methods including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. High performance liquid chromatography ("HPLC") can also be used for purification.

Antibodies of the present invention include naturally purified products, products of chemical synthetic procedures, and products produced by recombinant techniques from a eukaryotic host, including, for example, yeast, higher plant, insect and mammalian cells, or alternatively from a prokaryotic host as described above. A number of antibody production systems are described in Birch & Radner (2006) Adv. Drug Delivery Rev. 58: 671-685.

The term "antibody" also is intended to include antibodies of all immunoglobulin isotypes and subclasses. Particular isotypes of a monoclonal antibody can be prepared either directly by selecting from an initial fusion, or prepared secondarily, from a parental hybridoma secreting a monoclonal antibody of different isotype by using the sib selection technique to isolate class switch variants using the procedure described in Steplewski et al. (1985) Proc. Natl. Acad. Sci. USA 82:8653 or Spira et al. (1984) J. Immunol. Methods 74:307. Alternatively, recombinant DNA techniques may be used.

The isolation of other monoclonal antibodies with the specificity of the monoclonal antibodies described herein can also be accomplished by one of ordinary skill in the art by producing anti-idiotypic antibodies. Herlyn et al. (1986) Science 232:100. An anti-idiotypic antibody is an antibody which recognizes unique determinants present on the monoclonal antibody of interest.

In some aspects of this invention, it will be useful to detectably or therapeutically label the antibody. Suitable labels are described supra. Methods for conjugating antibodies to these agents are known in the art. For the purpose of illustration only, antibodies can be labeled with a detectable moiety such as a radioactive atom, a chromophore, a fluorophore, or the like. Such labeled antibodies can be used for diagnostic techniques, either *in vivo,* or in an isolated test sample.

The coupling of antibodies to low molecular weight haptens can increase the sensitivity of the antibody in an assay. The haptens can then be specifically detected by means of a second reaction. For example, it is common to use haptens such as biotin, which reacts avidin, or dinitrophenol, pyridoxal, and fluorescein, which can react with specific anti-hapten antibodies. See, Harlow and Lane (1988) supra.

The variable region of the antibodies of the present invention can be modified by mutating amino acid residues within the VH and/or VL CDR 1, CDR 2 and/or CDR 3 regions to improve one or more binding properties (e.g., affinity) of the antibody. Mutations may be introduced by site-directed mutagenesis or PCR-mediated mutagenesis and the effect on antibody binding, or other functional property of interest, can be evaluated in appropriate *in vitro* or *in vivo* assays. Preferably conservative modifications are introduced and typically no more than one, two, three, four or five residues within a CDR region are altered. The mutations may be amino acid substitutions, additions or deletions.

Framework modifications can be made to the antibodies to decrease immunogenicity, for example, by "backmutating" one or more framework residues to the corresponding germline sequence.

In addition, the antibodies of the invention may be engineered to include modifications within the Fc region to alter one or more functional properties of the antibody, such as serum half-life, complement fixation, Fc receptor binding, and/or antigen-dependent cellular cytotoxicity. Such modifications include, but are not limited to, alterations of the number of cysteine residues in the hinge region to facilitate assembly of the light and heavy chains or to increase or decrease the stability of the antibody (U.S. Patent No. 5,677,425); and amino acid mutations in the Fc hinge region to decrease the biological half life of the antibody (U.S. Patent No. 6,165,745).

Additionally, the antibodies of the invention may be chemically modified. Glycosylation of an antibody can be altered, for example, by modifying one or more sites of glycosylation within the antibody sequence to increase the affinity of the antibody for antigen (U.S. Patents Nos. 5,714,350 and 6,350,861). Alternatively, to increase antibody-dependent cell-mediated cytotoxicity, a hypofucosylated antibody having reduced amounts of fucosyl residues or an antibody having increased bisecting GlcNac structures can be obtained by expressing the antibody in a host cell with altered glycosylation mechanism (Shields et al. (2002) J. Biol. Chem. 277:26733-26740; Umana et al. (1999) Nat. Biotech. 17:176-180).

The antibodies of the invention can be pegylated to increase biological half-life by reacting the antibody or fragment thereof with polyethylene glycol (PEG) or a reactive ester or aldehyde derivative of PEG, under conditions in which one or more PEG groups become attached to the antibody or antibody fragment. Antibody pegylation may be carried out by an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. The antibody to be pegylated can be an aglycosylated antibody. Methods for pegylating proteins are known in the art and can be applied to the antibodies of the invention (EP 0 154 316 and EP 0 401 384).

Additionally, antibodies may be chemically modified by conjugating or fusing the antigen-binding region of the antibody to serum protein, such as human serum albumin, to increase half-life of the resulting molecule. Such approach is for example described in EP 0322094 and EP 0 486 525.

The antibodies or fragments thereof of the present invention may be conjugated to a diagnostic agent and used diagnostically, for example, to monitor the development or progression of a disease and determine the efficacy of a given treatment regimen. Examples of diagnostic agents include enzymes, prosthetic groups, fluorescent materials, luminescent materials, bioluminescent materials, radioactive materials, positron emitting metals using various positron emission tomographies, and nonradioactive paramagnetic metal ions. The detectable substance may be coupled or conjugated either directly to the antibody or fragment thereof, or indirectly, through a linker using techniques known in the art. Examples of suitable enzymes include horseradish peroxidase, alkaline phosphatase, beta-galactosidase, or acetylcholinesterase. Examples of suitable prosthetic group complexes include streptavidin/biotin and avidin/biotin. Examples of suitable fluorescent materials include umbelliferone, fluorescein, fluorescein isothiocyanate, rhodamine, dichlorotriazinylamine fluorescein, dansyl chloride or phycoerythrin. An example of a luminescent material includes luminol. Examples of bioluminescent materials include luciferase, luciferin, and aequorin. Examples of suitable radioactive material include ¹²⁵I, ¹³¹I, Indium-111, Lutetium-171, Bismuth-212, Bismuth-213, Astatine-211, Copper-62, Copper-64, Copper-67, Yttrium-90, Iodine-125, Iodine-131, Phosphorus-32, Phosphorus-33, Scandium-47, Silver-111, Gallium-67, Praseodymium-142, Samarium-153, Terbium-161, Dysprosium-166, Holmium-166, Rhenium-186, Rhenium-188, Rhenium-189, Lead-212, Radium-223, Actinium-225, Iron-59, Selenium-75, Arsenic-77, Strontium-89, Molybdenum-99, Rhodium-105, Palladium-109, Praseodymium-143, Promethium-149, Erbium-169, Iridium-194, Gold-198, Gold-199, and Lead-211. Monoclonal antibodies may be indirectly conjugated with radiometal ions through the use of bifunctional chelating agents that are covalently linked to the antibodies. Chelating agents may be attached through amines (Meares et al. (1984) Anal. Biochem. 142:68-78); sulfhydral groups (Koyama (1994) Chem. Abstr. 120:217262t) of amino acid residues and carbohydrate groups (Rodwell et al. (1986) PNAS USA 83:2632-2636; Quadri et al. (1993) Nucl. Med. Biol. 20:559-570).

Further, the antibodies or fragments may be conjugated to a therapeutic agent for example a antimicrobial that will treat or prevent the recurrence of a *Burklederia* infection. Suitable therapeutic agents include ceftazidime, ciprofloxacin, imipenem and minocycline.

Additional suitable conjugated molecules include ribonuclease (RNase), DNase I, an antisense nucleic acid, an inhibitory RNA molecule such as a siRNA molecule, an immunostimulatory nucleic acid, aptamers, ribozymes, triplex forming molecules, and external guide sequences. Aptamers are small nucleic acids ranging from 15-50 bases in length that fold into defined secondary and tertiary structures, such as stem-loops or G-quartets, and can bind small molecules, such as ATP (U.S. Patent No. 5,631,146) and theophiline (U.S. Patent No. 5,580,737), as well as large molecules, such as reverse transcriptase (U.S. Patent No. 5,786,462) and thrombin (U.S. Patent No. 5,543,293). Ribozymes are nucleic acid molecules that are capable of catalyzing a chemical reaction, either intramolecularly or intermolecularly. Ribozymes typically cleave nucleic acid substrates through recognition and binding of the target substrate with subsequent cleavage. Triplex forming function nucleic acid molecules can interact with double-stranded or single-stranded nucleic acid by forming a triplex, in which three strands of DNA form a complex dependant on both Watson-Crick and Hoogsteen base-pairing. Triplex molecules can bind target regions with high affinity and specificity.

The functional nucleic acid molecules may act as effectors, inhibitors, modulators, and stimulators of a specific activity possessed by a target molecule, or the functional nucleic acid molecules may possess a de novo activity independent of any other molecules.

The therapeutic agents can be linked to the antibody directly or indirectly, using any of a large number of available methods. For example, an agent can be attached at the hinge region of the reduced antibody component via disulfide bond formation, using cross-linkers such as N-succinyl 3-(2-pyridyldithio)proprionate (SPDP), or via a carbohydrate moiety in the Fc region of the antibody (Yu et al. (1994) Int. J. Cancer 56: 244; Upeslacis et al. "Modification of Antibodies by Chemical Methods," Monoclonal antibodies: principles and applications, Birch et al. (eds.), pp. 187-230 (Wiley-Liss, Inc. 1995); Price, "Production and Characterization of Synthetic Peptide-Derived Antibodies," in Monoclonal antibodies: Production, engineering and clinical application, Ritter et al. (eds.), pp. 60-84 (Cambridge University Press 1995)).

Techniques for conjugating therapeutic agents to antibodies are well known (Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody in Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates" 1982 Immunol. Rev. 62:119-58).

The antibodies of the invention or antigen-binding regions thereof can be linked to another functional molecule such as another antibody or ligand for a receptor to generate a bi-specific or multi-specific molecule that binds to at least two or more different binding sites or target molecules. Linking of the antibody to one or more other binding molecules, such as another antibody, antibody fragment, peptide or binding mimetic, can be done, for example, by chemical coupling, genetic fusion, or noncovalent association. Multi-specific molecules can further include a third binding specificity, in addition to the first and second target epitope.

Bi-specific and multi-specific molecules can be prepared using methods known in the art. For example, each binding unit of the bi-specific molecule can be generated separately and then conjugated to one another. When the binding molecules are proteins or peptides, a variety of coupling or cross-linking agents can be used for covalent conjugation. Examples of cross-linking agents include protein A, carbodiimide, N-succinimidyl-S-acetylthioacetate (SATA), 5,5'-dithiobis(2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohaxane-I-carboxylate (sulfo-SMCC) (Karpovsky et al., 1984 J. Exp. Med. 160:1686; Liu et al., 1985 Proc. Natl. Acad. Sci. USA 82:8648). When the binding molecules are antibodies, they can be conjugated by sulfhydryl bonding of the C-terminus hinge regions of the two heavy chains.

The antibodies or fragments thereof of the present invention may be linked to a moiety that is toxic to a cell to which the antibody is bound to form "depleting" antibodies. These antibodies are particularly useful in applications where it is desired to deplete an NK cell.

The antibodies of the invention may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

The antibodies also can be bound to many different carriers, for example pharmaceutically acceptable carriers. Thus, this invention also provides compositions containing the antibodies and another substance, active or inert. Examples of well-known carriers include glass, polystyrene, polypropylene, polyethylene, dextran, nylon, amylase, natural and modified cellulose, polyacrylamide, agarose, and magnetite. The nature of the carrier can be either soluble or insoluble for purposes of the invention. Those skilled in the art will know of other suitable carriers for antibodies, or will be able to ascertain such, using routine experimentation.

The following examples are intended to illustrate, but not limit the invention.

The scope of the invention is defined by the appended claims.

### EXPERIMENTAL PROCEDURES

### EXPERIMENT NO. 1

### MATERIALS AND METHODS

**Ethics statement.** Prior to collection of sputa from pediatric patients at Nationwide Children's Hospital, written informed consent and authorization was obtained from the parent(s) or a legally authorized representative. This procedure was in concordance with all institutional and federal guidelines and was performed according to a protocol approved by the Institutional Review Board at Nationwide Children's Hospital, Columbus, Ohio.

**Biofilm formation** ***in vitro.** B. cenocepacia* strains (including strains K56-2, DFA2 and JRL2) were inoculated from a frozen stock into LB broth (BBL) and grown overnight at 37°C with shaking at 200 rpm. Cultures were then diluted 1:100 in fresh broth, the optical density read at 600 nm and adjusted to a final concentration of 106 CFU *B. cenocepacial* ml medium. The bacteria were further diluted 1:2500 in LB broth and 200 µl of the bacterial suspension was added to each well of a LabTek II 8-well chambered coverglass (LabTek). Slides were incubated static for 16 hrs at 37°C in a humidified atmosphere at which time medium was aspirated and replaced with fresh LB broth. After an additional 8 hrs (24 hrs total incubation time), biofilms were either stained or treated with antiserum or antibiotic. Assays were performed a minimum of three times.

**Distribution of IHF and DNA within a *B. cenocepacia* biofilm.** To examine the relative distribution of IHF within biofilms formed by *B. cenocepacia* strains, unfixed 24 hr biofilms were incubated with naive rabbit serum or rabbit anti-*E*. *coli* IHF (Goodman et al. (2011) Mucosal Immunol. 4:625-637) and revealed with goat anti-rabbit IgG conjugated to AlexaFluor 594 (Invitrogen). DNA within the biofilms was stained with anti-dsDNA antibody (Abcam, Inc.) and revealed with goat anti-mouse IgG conjugated to AlexaFluor 647 (Molecular Probes). Intact bacterial cells were stained with the membrane stain FilmTracer™ FM® 1-43 Green Biofilm Stain (Molecular Probes) according to manufacturer's instructions. Images were collected with a Zeiss 510 meta-laser scanning confocal microscope (Carl Zeiss) using a 63X objective. 3-D images were reconstructed with AxioVision Rel. 4.8 (Carl Zeiss).

To determine the relative abundance of eDNA in a 24 hr biofilm formed by *B. cenocepacia*, the DNA to bacterium ratio was determined using Zeiss image acquisition software wherein the relative fluorescent intensity of anti-dsDNA labeling to FilmTracer™ was compared. Biofilms formed by nontypeable *Haemophilus influenzae* were also established (Goodman et al. (2011) Mucosal Immunol. 4:625-637; Jurcisek et al. (2011) J. Vis. Exp.) and similarly stained.

**Distribution of IHF within human sputa.** To visualize IHF labeling with human sputa, sputum samples were collected from 3 pediatric patients with CF that were also culture positive for *B. cenocepacia* under an approved IRB protocol. Sputa were embedded in OCT compound (Fisher Scientific) and snap frozen in the vapor phase of liquid nitrogen. Ten micron serial sections were cut and adhered to glass slides. Slides were air-dried, fixed in cold acetone, equilibrated in buffer containing 0.05M Tris-HCl, 0.15M NaCl and 0.05% Tween 20 (pH 7.4) then blocked with image-iT FX signal enhancer (Molecular Probes) and Background Sniper (BioCare Medical). Sections were incubated with polyclonal anti-IHF or naive rabbit serum, and revealed with goat anti-rabbit IgG-Alexafluor 594 (Invitrogen). DNA was counterstained with Prolong Gold anti-fade reagent with DAPI (Molecular Probes). Images were viewed as described prior.

Resolution of established *B. cenocepacia* biofilms. To assess the ability of antiserum to resolve a biofilm formed *in vitro,* 24 hr biofilms were first established using strain K56-2 as described, then exposed to either naive rabbit serum or rabbit anti-*E*. *coli* IHF diluted 1:50 in LB broth, or LB broth only, for 16 hrs. Biofilms were then stained with LIVE/DEAD® *Bac*Light™ Bacterial Viability kit for microscopy (Molecular Probes) as previously described (Jurcisek et al. (2011) J. Vis. Exp.), then fixed with a solution of 16% paraformaldehyde- 2.5% glutaraldehyde- 4% acetic acid in 0.2M phosphate buffer, pH 7.4, prior to immediate imaging by confocal microscopy as described prior. Quantitation of average thickness and mean biomass was determined via COMSTAT2 analysis (Heydorn et al. (2000) Microbiology 146 (Pt 10):2409-2415). As an additional measure to confirm that application of naive and immune rabbit serum did not induce cell death, both planktonic and biofilm adherent *B. cenocepacia* were collected 16 hrs after treatment, stained the unfixed cells with LIVE/DEAD® *Bac*Light™ Bacterial Viability and Counting kit for flow cytometry (Molecular Probes) and distinguished between live and dead bacteria using a C6 calibrated flow cytometer (Accuri). Assays were performed a minimum of three times.

**Enrichment of IgG from whole serum.** To confirm that biofilm resolution was mediated by antibody and not other serum components, IgG was purified from rabbit anti-IHF serum with HiTrap Protein G HP columns (GE Healthcare) according to manufacturer's instructions and collected both the eluent that passed through the column after application of serum and the IgG-enriched fraction. To demonstrate that anti-IHF and IgG-enriched anti-IHF recognized purified IHF in addition to native IHF expressed by *B. cenocepacia* strain K56-2, SDS-PAGE and Western blotting were performed. Briefly, 0.5 µg purified IHF ([25]; the gift of Howard Nash, NIH) and 5 µg *B. cenocepacia* whole cell lysate were separated on a 4-15% Mini-PROTEAN TGX gel (BioRad) in Tris/Glycine/SDS buffer (BioRad) then transferred on to nitrocellulose membranes (Invitrogen). Membranes were blocked overnight at 4°C in 3% skim milk in Tris-buffered saline with 0.5% Tween20 (Fisher Scientific) prior to incubation with rabbit anti-IHF or IgG-enriched anti-IHF followed by goat anti-rabbit IgG-HRP (Invitrogen). Blots were developed with CN/DAB substrate kit (Pierce) and images captured with BioRad GS800 densitometer.

**Synergistic effect of anti-IHF and antibiotics in biofilm resolution.** Examination of the synergistic effect of antibiotics combined with antiserum directed against IHF when applied to a biofilm formed by *B. cenocepacia* strain K56-2 required determination of the minimum inhibitory concentration (MIC) of several antibiotics for planktonic cultures of *B. cenocepacia* strain K56-2. To do so, 100 CFU *B. cenocepacia* were inoculated into LB broth containing two-fold serial dilutions of the following antibiotics: ceftazidime, ciprofloxacin, imipenem, meropenem, minocycline, sulfamethoxazole-trimethoprim or tobramycin (Sigma). Cultures were incubated static for 24 hrs, and turbidity of the culture assessed. The MIC for planktonic *B. cenocepacia* was identified as the concentration of antibiotic at which no bacterial growth was observed. With this information, established *B. cenocepacia* biofilms were treated with a 1:50 dilution of anti-IHF, antibiotic at the determined MIC, anti-IHF plus antibiotic or medium alone for 16 hrs prior to viability stain with LIVE/DEAD® *Bac*Light™ Bacterial Viability kit and visualization by confocal microscopy as previously described. The following concentration of antibiotics were used: ceftazidime- 16 µg/ml, ciprofloxacin- 2 µg/ml, imipenem- 32 µg/ml, meropenem- 16 µg/ml, minocycline- 4 µg/ml, sulfamethoxazole-trimethoprim- 16 µg/ml and tobramycin- 512 µg/ml. Quantitation of average thickness and mean biomass was determined via COMSTAT2 analysis. Assays were performed a minimum of three times.

**Treatment of established biofilms with Pulmozyme®.** To examine the outcome of treatment of established *B. cenocepacia* strain K56-2 biofilms with Pulmozyme® (dornase alpha; Genentech, Inc.), established biofilms were treated with a 1:50 dilution of anti-IHF, 0.25 mg of Pulmozyme®, anti-IHF plus Pulmozyme® or medium alone for 16 hrs prior to viability stain and visualization by confocal microscopy. Quantitation of average thickness and mean biomass was determined via COMSTAT2 analysis. Assays were performed a minimum of three times.

**Impact of anti-IHF antibody on *B. cenocepacia* survival within macrophages**. *B. cenocepacia* strain MHK1 (Hamad et al. (2010) Appl. Environ. Microbiol. 76:3170-3176) which has a mutation in an antibiotic efflux pump that confers gentamicin sensitivity but does not alter trafficking of the mutant in macrophages was used for this assay. Strain MHK1 was cultured as described above then treated with naive serum or anti-IHF serum at a concentration of 1:1000 for 15 minutes. Treated *B. cenocepacia* were then added to murine bone marrow -derived macrophages for 1 hr. To kill extracellular bacteria, Iscove's media (GIBCO) containing 10% heat-inactivated FBS (GIBCO) and 50 µg gentamicin/ml (GIBCO) was added for 30 min. Macrophages were lysed at 2, 4 and 6 hrs post-infection and lysates were plated to determine CFU *B. cenocepacia*/ ml. Assays were performed a minimum of three times.

**DNase footprinting experiments.** The 386 bp of upstream DNA from *BCAL0339* and *BCAL0340* was amplified by the PCR reaction. The isotopically end labeled (32P) amplicon was used in a binding reaction with crystallographically pure *E. coli* IHF (Rice et al. (1996) Cell 87:1295-1306; the gift of Howard Nash, NIH) and subsequently in DNase I footprinting assays (Hung et al. (2011) J. Bacteriol. 193:3642-3652).

**Statistical methods.** To determine significant differences in average biofilm thickness and biomass, and in macrophage association assays, two sample paired T-tests were performed using GraphPad Prism software, version 6.00.

### RESULTS

**Evidence for the presence of abundant extracellular DNA (eDNA) in biofilms formed by *B. cenocepacia in vitro.*** To first characterize the basic structure of *B. cenocepacia* biofilms when formed *in vitro, B. cenocepacia* strain K56-2 statically was grown in a chamber slide for 24 hrs before labeling with FilmTracer FM 1-43. As shown in **FIG. 1A**, *B. cenocepacia* formed a robust biofilm of approx. 26 µm height with characteristic towers. To now determine whether *B. cenocepacia* incorporated DNA into its biofilm matrix, the unfixed biofilm was labeled with a monoclonal antibody to detect the presence of dsDNA (white). The biofilm formed contained an abundant amount of eDNA that was particularly dense at the base of the biofilm (**FIG. 1B**), suggesting that this matrix component might be serving an essential role in bacterial adherence and anchoring during early stages of biofilm development. Interestingly, while visually apparent, further COMSTAT analysis of both a *B. cenocepacia* biofilm and one formed by nontypeable *Haemophilus influenzae* (NTHI) (data not shown) then labeled in an identical manner and using separate laser channels (to detect bacteria and DNA) confirmed in a more objective manner that *B. cenocepacia* incorporated approximately 30% more eDNA per bacterial cell into its biofilm than does this additional important human airway pathogen (Jurcisek et al. (2007) J. Bacteriol. 189:3868-3875).

**Demonstration of a DNABII protein within biofilms generated by B. *cenocepacia.*** To determine whether biofilms formed by *B. cenocepacia* strain K56-2 contained a member of the DNABII family of proteins, a biofilm formed *in vitro* by *B. cenocepacia* strain K56-2 was incubated with rabbit anti-IHF antibody [antiserum against *E. coli* IHF that cross-reacts with multiple DNABII family members (Goodman et al. (2011) Mucosal Immunol. 4:625-637)] and observed labeling throughout the biofilm (**FIG. 1C**).

To now assess whether observations made *in vitro* were relevant to the clinical condition of CF patients, as well as to determine whether, as seen with NTHI [20], IHF was positioned at the vertices of crossed strands of eDNA present within the biofilm formed by *B. cenocepacia,* were obtained sputum samples from CF patients known to be infected with *B. cenocepacia.* Samples were snap frozen and immunolabeled using the same anti-IHF antibody. The inventors observed heavy labeling of 3/3 (100%) of sputum samples recovered to date. Notably, it was observed specific labeling of virtually 100% of the vertices formed by overlapping dsDNA strands present within these samples (**FIG. 2**). Given the large amount of eDNA present within the EPS matrix of a *B. cenocepacia-*produced biofilm, and the fact that IHF appeared to be critically positioned within this matrix, it was that targeting this protein for intervention might result in structural collapse of the biofilm. If this collapse occurred, it was further reasoned that this might greatly facilitate access of immune effectors, antibiotics or other therapeutic agents to bacteria formerly protected by the EPS, thus promoting their eradication.

**Demonstration of the ability of anti-IHF to disrupt a biofilm formed by *B*. *cenocepacia in vitro.*** To test one of these hypotheses, 24 hr *B. cenocepacia* strain K56-2 biofilms was treated with either medium (**FIG. 3A**), naive rabbit serum (diluted 1:50) (**FIG**. **3B**) or rabbit anti-IHF serum (at an arbitrarily selected dilution of 1:50) (**FIG. 3C**). Following 24 hrs of incubation, these biofilms werea analyzed via COMSTAT software and found that treatment with naive rabbit serum induced a small and statistically non-significant change in biofilm thickness but had no effect on mean biomass over that seen when sterile medium was used (**FIGS. 3G****&3H**). This effect is attributed to non-specific antibodies within whole rabbit serum that cross react with outer membrane proteins of multiple Gram negative bacteria. Conversely however, treatment with antiserum directed against IHF resulted in: 44% reduction in thickness, 56% reduction in biomass, and 52% reduction in height compared to naive serum. These latter effects were statistically significant compared to use of either sterile medium or naive serum (**FIGS. 3G****&3H**). To demonstrate that treatment with antiserum directed against IHF did not kill resident bacteria thus accounting for the observed disruption of the biofilms, after treatment the inventors recovered both the planktonic sub-population of bacteria contained within the culture medium as well as that bacterial sub-population that remained within the biofilm and subjected both preparations to analysis by flow cytometry to determine relative proportion of live to dead bacteria. Regardless of treatment used, the inventors found that ≥80% of bacteria within the planktonic sub-population and ≥93% of those that has remained resident within the biofilm were viable. This result suggested that treatment with anti-IHF mediated the release of viable bacteria into the planktonic phase.

To demonstrate that the biofilm disruption activity observed upon treatment with rabbit anti-IHF serum was primarily due to IgG antibodies directed against IHF in this polyclonal but hyperimmune serum, the inventors enriched this serum for IgG as described in Methods (above) then assayed both the fraction enriched for IgG (**FIG. 3D**) as well as the serum fraction that flowed through the column (**Fig 3E**) for relative ability to disrupt a pre-formed *B. cenocepacia* biofilm. As can be seen in **FIGS. 3D****&3E**, as well as plotted in **3G&3H**, the IgG enriched antiserum preparation retained the activity of the whole rabbit anti-IHF; however there was no ability of the effluent from the enrichment column to similarly disrupt the biofilm. Western blots were performed to demonstrate that antibodies contained within both whole anti-IHF serum as well as the fraction enriched for IgG recognized purified IHF protein as well as the mono-, di- and tri-meric forms of this protein within whole cell lysates of B. cenocepacia strain K56-2 (**FIG. 3F**); the DNABII family member, PG0121 (HUβ from *Porphyromonas gingivalis*), also shows the capacity to maintain multimers during SDS PAGE (S. Goodman, personal communication).

**Demonstration of synergistic interaction between anti-IHF and traditional antibiotics.** To ascertain whether anti-IHF mediated biofilm disruption might now render resident bacteria more susceptible to treatment with other existing potential therapeutic agents, pre-formed *B. cenocepacia* biofilms were exposed to each of seven antibiotics (using the determined MIC for each against planktonically grown *B. cenocepacia* strain K56-2 as described in Experimental Procedures) either alone or in combination with anti-IHF serum (at a 1:50 dilution). As shown in **FIG. 4** [using incubation with ceftazidime (at 16 µg/ml) as an example], whereas treatment with anti-IHF (**FIG. 4D**) induced a reduction in biofilm height, thickness and biomass over that of an untreated biofilm (**FIG. 4A**), neither of these treatments induced much bacterial cell death (**FIGS. 4B&E**). Further, antibiotic treatment alone had little observable effect on the *B. cenocepacia* induced biofilm (**FIG. 4G**), and while bacterial cell death had increased over that observed following treatment with either sterile medium or anti-IHF alone, this effect was nonetheless minimal (**FIG. 4H**). When used together however, there was a 43% reduction in height (**FIG. 4J**) and an obvious and notable increase in bacterial cell death (as indicated by red/orange color) (**FIG. 4K**) over that observed when antibiotics were used alone (compare **FIGS. 4H&K**), suggesting a synergistic interaction between anti-IHF and ceftazidime. A significant reduction in average biofilm height (**FIG. 4M**) and biomass (**FIG. 4N**) between treatment with anti-IHF plus antibiotic versus antibiotic alone was similarly obtained following treatment of *B. cenocepacia* biofilms with ciprofloxacin plus anti-IHF, imipenem plus anti-IHF and minocycline plus anti-IHF (*p*< 0.05).

**Demonstration that use of Pulmozyme (DNase) may be contraindicated in CF patients that are infected with** ***B. cenocepacia.*** Treatment with DNase is used as a standard of care for patients with CF, and given that *B. cenocepacia* incorporates an abundance of eDNA into its biofilm, the inventors hypothesized that use of anti-IHF in combination with DNase would also potentially have a synergistic effect in terms of debulking and eradicating bacteria within a *B. cenocepacia*-induced biofilm *in vitro* as the inventors previously showed occurs with NTHI (Goodman et al. (2011) Mucosal Immunol. 4:625-637). However, this was not the case. In fact, in multiple repeated assays, exposure of a *B. cenocepacia* biofilm to DNase induced a significantly more robust biofilm in terms of average thickness (**FIGS**. **5B****&****D**) than one treated with diluent alone (**FIG. 5A**). These DNase-treated biofilms were only slightly increased in maximum height (compare 32 µm to 28 µm), however the mean increase in surface to volume ratio was *12*%; the mean increase in biomass was *174%* and when compared for average thickness, and the biofilm formed by *B. cenocepacia* that had been treated with DNase was *204%* thicker than that formed by *B. cenocepacia* that had been treated with diluent alone. Whereas antiserum directed against IHF was still able to effectively reduce these enhanced biofilms (**FIG. 5C****,D&E**), given the unexpected results obtained with DNase alone, pursuit of this line of investigation for potential synergistic use became counterintuitive. Importantly, this result has significant potential clinical implications for CF patients infected with *B. cenocepacia* as repeated use of DNase may actually *exacerbate* their disease and contribute to the very poor clinical outcome that exists for these patients.

**Demonstration that incubation *of B. cenocepacia* with antibodies directed at a DNABII protein but not naive serum results in reduced bacterial recovery from macrophages**. *B. cenocepacia* can persist and replicate within murine and human CF macrophages. However, WT murine macrophages or macrophages derived from non-CF patients restrict *B. cenocepacia* infection by delivery of the organism to the lysosome for degradation. Given that it has recently been demonstrated that IHF associated with uropathogenic *E. coli* influences the ability of this bacterium to efficiently colonize the bladder (Justice et al. (2012) PLoS One 7:e48349), the inventors wondered if incubation of *B. cenocepacia* with antiserum directed against IHF might influence its interaction with macrophages, given the importance of this host cell in clearance of bacteria from the CF lung. Using a variant of strain K56-2 where gentamycin sensitivity was introduced (strain MHK1); it was determined the number of *B. cenocepacia* that multiplied intracellularly (after killing extracellular bacteria via gentamycin treatment). Whereas the number of macrophage-associated *B. cenocepacia* at 2 hours post infection was not significantly different between those pre-treated with naive serum *versus* those pre-treated with anti-IHF serum (at a 1:1000 dilution) which suggested equivalent uptake by macrophages at this time point, by 6 hrs post infection *B. cenocepacia* pre-treated with anti-IHF serum were significantly impeded for growth within CF macrophages (*p* < 0.05) (**FIG. 6**). Thus, pre-treatment of *B. cenocepacia* with anti-IHF promoted their clearance by CF macrophages; however the mechanism which underlies this observation remains to be determined and is the subject of ongoing investigation.

**Demonstration of the association of T3SS or T6SS with incorporation of DNABII protein(s) into the *B. cenocepacia* biofilm matrix.** To begin to attempt to elucidate the molecular mechanism(s) by which *B. cenocepacia* incorporates both eDNA and DNABII protein(s) into their biofilms, the inventors incubated biofilms formed in 24 hrs by either *B. cenocepacia* strain K56-2 (parental isolate), *B. cenocepacia* strain JRL2 (*ΔbcsV*; type III secretion system mutant - T3SS) or *B. cenocepacia* strain DFA2 (Δ*BcsK*; type VI secretion system mutant - T6SS) with anti-IHF antibody (Aubert et al. (2010) J. Biol. Chem. 285:35988-35998). In the biofilm formed by the parental isolate, positive labeling was distributed through the biofilm (**FIG. 7A**) as described earlier. In the biofilm formed by the T3SS mutant, whereas the biofilm itself was overall less robust than that formed by the parental isolate (biomass 4.5 µm3/µm2 compared to 18 µm3/µm2 for the parental isolate; average thickness 5.5 µm compared to 25.2 µm for the parental isolate), labeling via anti-IHF serum was again present throughout the biofilm, however as observed with the parental isolate, labeling appeared to be much stronger at the base of the biofilm (**FIG. 7B**). Conversely, however, there was very little labeling of the biofilm formed by the T6SS mutant (**FIG. 7C**), which was also significantly less robust than that formed by the parental isolate (biomass 2.9 µm3/µm2; thickness 3.9 µm). This latter observation was not unexpected as the T6SS of *B. cenocepacia* is known to be associated with biofilm formation (Aubert et al. (2008) Infect. Immun. 76: 1979-1991). Collectively however, these data suggested that export of DNABII proteins (and possibly eDNA as well) was dependent upon the T6SS of *B. cenocepacia.*

Given the affect that a T6SS mutant had on the relative amount of IHF and eDNA present in a biofilm built *in vitro* (**FIG. 8**), the inventors questioned whether IHF might possibly act *intracellularly* in *B. cenocepacia* to affect transcription of the T6SS gene cluster (*BCAL0340* to *BCAL0348*; the *BcsK* gene is equivalent to *BCAL0342*). As a first step in this regard, the upstream region of this gene cluster was examined and the sequence TCTCAACGATTTA, was identified, a near perfect match to the IHF binding consensus sequence WATCAANNNNTTR (where W is A or T, N is any nucleotide and R is a A or G). In the presence of 50 nM *E. coli* IHF a strong DNase footprint is visible (**FIG. 7D**) covering the region 25 to 52 bp upstream of the coding sequence of BCAL0340 and overlapping this match to the consensus sequence. This result could indicate that IHF self regulates its own release as well as perhaps that of eDNA that is incorporated into the biofilm matrix.

### DISCUSSION

Cystic Fibrosis (CF) is a hallmark example of a chronic and persistent disease that defies current treatment modalities. Biofilms resident within the lungs of CF patients contribute significantly to both pathogenesis and chronicity. A biofilm is a highly-organized, multicellular community encased in an extra-cellular polymeric matrix or substance (or EPS) that is affixed to an inert or biological surface and is the preferred lifestyle of all bacteria in nature. Bacterial populations within a biofilm, as opposed to their planktonic or free-living counterparts, have a reduced growth rate (due to nutrient limitation), a distinct transcriptome (Post et al. (2007) Curr. Opin. Otolaryngol. Head Neck Surg. 15:347-351; Post et al. (2004) Curr. Opin. Otolaryngol. Head Neck Surg. 12:185-190) and a substantially increased resistance not only to effectors of innate and acquired immunity, but also to the action of antibiotics (Slinger et al. (2006) Diagn. Microbiol. Infect. Dis. 56:247-253). Moreover, the EPS presents a formidable physical barrier to phagocytic cells and other bacterial clearance mechanisms (both physical and physiological) and thereby, biofilms are highly recalcitrant to eradication (Flemming et al. (2010) Nat. Rev. Microbiol. 8:623-633). Diseases wherein biofilms play a major role in pathogenesis and chronicity, such as CF, thus require novel methods for treatment and prevention. Whereas the composition of the EPS of biofilms is highly diverse among *genera*, as well as influenced by the environment in which it is formed, a very common and critical component is the incorporation of extracellular DNA (eDNA). Although the mechanism by which eDNA is released by the microbe and/or incorporated into the biofilm matrix has not yet been elucidated for many pathogens, eDNA is nonetheless of tremendous interest both in terms of its biological functionality, as well as its role as a structural component of the biofilm.

Disclosed herein is that *Burkholderia cenocepacia* incorporates an abundance of eDNA into the biofilms it forms. The presence of this wealth of eDNA is likely to provide exceptional protection to resident *B. cenocepacia* as a physical barrier and, as we have recently shown, eDNA within a biofilm can also bind effectors of innate immunity (Jones et al. (2012) J. Innate Immun), thus limiting or preventing their access to bacterial cells within. Specifically with regard to *B. cenocepacia,* Peeters et al. (Peeters et al. (2008) J. Hosp. Infect. 70:361-368) showed that sessile *B. cenocepacia* were highly resistant to chlorhexidine, hydrogen peroxide and 5% bleach, even after treatment for 5 mins. Moreover, analysis of FDA product recall data for non-sterile pharmaceutical products from 1998-2006 showed that 48% of recalls were due to contamination by either *B. cepacia, Pseudomonas* species or *Ralstonia picketti* (Jimenez (2007) PDA J. Pharm. Sci. Technol. 61:383-399). For non-sterile and sterile products, *B. cenocepacia* was the most frequently isolated species. Collectively, these data indicate that *B. cenocepacia* contamination of surfaces, equipment and pharmaceutical devices, likely in the form of a biofilm, serves as a source of infection for not only CF patients, but for any hospitalized, ventilated (Graindorge et al. (2010) Diagn. Microbiol. Infect. Dis. 66:29-40; Lucero et al. (2011) Am. J. Infect. Control. 39:775-778) and/or immunocompromised patient *without* CF (Vandamme et al. (1997) Int. J. Syst. Bacteriol. 47:1188-1200). These observations inspired the attempt to develop a novel immunotherapeutic strategy for CF patients, particularly those infected with *B. cenocepacia.* To do so, attention was focused on eDNA and a family of proteins known to bind to this extracellular DNA, the DNABII proteins.

Disclosed herein is that *B. cenocepacia* incorporates an abundance of eDNA into their biofilms. This eDNA is associated with a DNABII protein that interacts with antiserum directed against isolated native IHF produced by *E. coli.* When *B. cenocepacia* biofilms are examined to determine the spatial distribution of this DNABII protein, it was found that as observed for NTHI (Goodman et al. (2011) Mucosal Immunol. 4:625-637), positive labeling was associated with each vertice of crossed strands of eDNA present within the biofilm. Biofilms produced by *B. cenocepacia* were susceptible to disruption by anti-IHF but not naive serum in an *in vitro* assay system. Further, this disruptive effect of antiserum directed against IHF rendered bacteria within a *B. cenocepacia*-induced biofilm susceptible to the killing action of several antibiotics traditionally, but usually ineffectively, used to treat CF patients. These antibiotics were either not effective or were significantly less effective at killing *B. cenocepacia* within a biofilm *in vitro* in the absence of treatment with anti-IHF serum. Whereas the action of anti-IHF serum resulted in disruption of *B. cenocepacia* formed biofilms by targeting a lynchpin protein responsible for bending and stabilizing eDNA into the lattice structure seen within these biofilms, DNase was not found to be effective when incubated with *B. cenocepacia* biofilms *in vitro.* In fact, unlike the reductive effect observed with biofilms formed by either NTHI (Gustave et al. (2012) J. Cyst. Fibros.) or *P. aeruginosa* (Whitchurch et al. (2002) Science 295:1487), treatment of a *B. cenocepacia* biofilm with DNase induced the formation of a markedly more robust biofilm, suggesting that treatment of CF patients who are infected with *B. cenocepacia* would be contraindicated. It was also demonstrated that that pre-treatment of *B. cenocepacia* with antiserum directed against IHF appeared to facilitated the routing of ingested *B. cenocepacia* to a more effective degradative pathway within murine CF macrophages as there was a statistically significant increase in killing of ingested *B. cenocepacia* that were treated with anti-IHF at 6 hours post-infection than were those that had been pre-incubated with naive serum. Whereas the mechanism(s) for this observation are not yet known, we hypothesize that binding of anti-IHF to bacterial cell-associated extracellular IHF may have played a role in this regard. Lastly, in an attempt to begin to unravel the molecular mechanism by which *B. cenocepacia* incorporates both eDNA and DNABII protein(s) into its biofilm, we examined biofilms built by both a T3SS and T6SS mutant of the strain K56-2 isolate used here. Whereas both mutant biofilms were compromised overall in terms of relative biofilm robustness, that built by the T6SS mutant was absent of labeling via antiserum directed against IHF, suggesting that secretion of this protein as well as robust biofilm formation was dependent upon an active T6SS. Moreover, we identified a putative IHF binding site directly upstream of the T6SS gene cluster, suggesting that IHF may regulate its own export. Indeed a formal transcriptomal analysis of IHF deficient mutants may further delineate IHF's role in *B. cenocepacia* pathogenesis; it is already known that IHF is both part of the extracellular matrix and regulates virulence factor expression in uropathogenic *E. coli* (Justice et al. (2012)). The mechanisms that underlie these observations are being further investigated.

Despite tremendous recent advances in our ability to better manage patients with CF, these individuals are only expected to survive until their mid-30s, even today. At least 90% of CF patients die of respiratory failure after enduring many years of chronic, recurrent and persistent bacterial infection of the lungs. Bacteria dwelling within a biofilm in the lungs of CF patients present a formidable obstacle. Thereby, in order to design novel and effective strategies to better treat and/or prevent the long-term bacterial infections characteristic of CF, it is necessary to understand both the unique biology of biofilms, as well as determine how one might undermine these structures to mediate a therapeutic or preventative 'cure'. Herein, we showed that targeting a bacterial protein that stabilizes eDNA present within a biofilm is highly effective for reducing or eradicating that structure *in vitro.* Moreover treatment of a *B. cenocepacia* induced biofilm with anti-IHF worked synergistically with multiple standard antibiotics to render resident bacterial cells sensitive to killing. Lastly, the inventors discovered that pre-treatment of *B. cenocepacia* with antiserum directed against IHF significantly inhibited their survival when ingested by murine CF macrophages. Based on data obtained to date, an approach which targets DNABII proteins associated with eDNA within a *B. cenocepacia* biofilm shows promise in providing a potential new approach for treatment of CF patients, and particularly those colonized with *B. cenocepacia.* Most importantly, given that multiple human pathogens appear to use a similar strategy wherein eDNA within the biofilm is associated with a member of the DNABII family of nucleic acid binding proteins, the approach developed here is likely to have utility for other respiratory tract pathogens which often precede and facilitate *B. cenocepacia* infection of the CF lung (George et al. (2009) FEMS Microbiol. Lett. 300:153-164).

### EXPERIMENT NO. 2

### Bacterial strain, biofilm formation, IHF and sera

NTHI 86-028NP is a minimally passaged clinical isolate cultured from the nasopharynx of a child undergoing tympanostomy tube insertion for chronic otitis media. Formation of NTHI biofilms in 8-well chambered coverglass slides has been described (Jurcisek et al. (2011) J. Vis. Esp). For all biofilm assays, duplicate wells were viewed on a Zeiss 510 Meta-laser scanning confocal microscope, images compiled with Zeiss Zen software and biomass and/or mean biofilm thickness values calculated with COMSTAT2 software (Heydorn et al. (2000) Microbiology 146(Pt10):2395-2407). All biofilm assays were repeated a minimum of three times, on separate days. Data represent mean + SEM.

Purified *E. coli* IHF and rabbit antiserum against purified *E. coli* IHF ('anti-IHF*_{E}*. *_{coli}*') were gifts from Howard Nash (Granston et al. (1993) J. Mol. Biol. 234:45-59; Rice et al. (1996) Cell 87:1295-1306). Naive rabbit serum was purchased from Spring Valley Laboratories.

### Quantitation of IHF-specific IgG

IHF-specific IgG was purified from polyclonal serum using HiTrap Protein G HP columns (GE Healthcare). Quantitation of IHF-specific IgG in both polyclonal and IgG-enriched anti-IHF_{*E*. *coli*} was determined by slot blot versus purified IHF_{*E*.} *_{coli}.* A standard curve was generated using rabbit reference serum versus purified rabbit IgG (Bethyl Laboratories, Inc.) and band intensity analyzed using AlphaView software (ProteinSimple).

### Resolution of mature biofilms

Biofilms were established for 24-, 48-, and 96 hr, or 1- and 2 weeks. To maintain bacterial viability, medium (brain heart infusion broth supplemented with 2 µg ml⁻¹ each of β-NAD and heme) was changed twice daily. Biofilms were incubated with medium, anti-IHF*_{E}*. *_{coli}* (a 1:50 dilution, equivalent to 4.4 µg IHF-specific IgG well⁻¹ for biofilms of ≤48 hr or a 1:10 dilution, equivalent to 22.0 µg IHF-specific IgG well⁻¹ for biofilms of ≥96 hr) or an equivalent volume of naive serum. After 16 hr, biofilms were stained with BacLight™ Bacterial Viability Kit (Molecular Probes), fixed in a solution of 1.6% paraformaldehyde, 2.5% glutaraldehyde and 4.0% acetic acid in 0.1M phosphate buffer and viewed as described.

### Kinetics of biofilm resolution

Biofilms established for 24 hr were incubated with medium or either anti-IHF_{*E*. *coli*} or naive serum diluted 1:50 for 0, 6, 12, 16 or 24 hr prior to viability staining and fixation as described. For the 0 hr time point, treatment was applied, and then immediately removed. To maintain bacterial viability of biofilms treated for 24 hr, treatments were replaced after 16 hr and incubated an additional 8 hr. To attempt to completely eradicate a 24 hr biofilm, a 1:5 dilution of anti-IHF*_{E. coli}* or an equivalent volume of naive serum was applied.

### Inhibition of direct contact between anti-IHF antibodies and NTHI biofilms

IgG-enriched anti-IHF_{*E*. *coli*} was covalently coupled to agarose beads (>45 µm diameter) via AminoLink Plus kit (Thermo Scientific). To determine whether direct contact of anti-IHF_{*E*. *coli*} antibodies with the biofilm was required to induce resolution, 24 hr biofilms were established in optical bottom 96-well plates, then incubated with: medium, 1:50 dilution of anti-IHF_{*E*. *coli*} or an equivalent volume of naive serum, applied directly to the biofilm (80 µl total volume) or after insertion of a 5 µm pore size HTS Transwell (Corning) into the 96-well plate, medium, 0.5-, 5.0- or 50.0 µg IgG-enriched anti-IHF_{*E*. *coli*} covalently bound to agarose beads, or an equal volume of IgG-enriched naive serum bound to agarose beads, were placed into the apical chamber (80 µl total volume). Biofilms were incubated for 16 hr prior to processing as described. To confirm that IHF-specific antibody did not diffuse into the basolateral chamber, supernatants from the basolateral chamber were collected and assayed by Western blotting for reactivity to purified IHF_{*E*. *coli*}.

To determine whether biofilm resolution by anti-IHF*_{E. coli}* could be blocked by steric hindrance, 80 µl of naked agarose beads were added to the apical chamber one hr prior to layering 50.0 µg IgG-enriched anti-IHF*_{E. coli}* bound to beads or a comparable volume of IgG-enriched from naive serum coupled to beads. Plates were incubated an additional 16 hr, then biofilms were stained, viewed and analyzed as described.

To identify whether the ability of IHF-specific antibody to sequester free IHF was limited by relative accessibility, 50.0 µg IgG-enriched anti- IHF_{*E*. *coli*} conjugated to beads or an equivalent volume of IgG-enriched from naive serum bound to beads was applied to the apical chamber of a transwell in which the basolateral chamber contained a 24 hr NTHI biofilm. After 6 hr, the contents of apical chamber were mixed by stirring, incubated an additional 10 hr, then the biofilms were stained, viewed and analyzed as described.

### Adsorption of anti-IHF_{E. coli}

To neutralize anti-IHF_{*E*.*coli*}-mediated biofilm debulking, IHF-specific antibodies were adsorbed from serum by incubation with purified IHF_{*E*. *coli*}. Aliquots of anti-IHF*_{E. coli}* (4.4 µg IHF-specific IgG) were incubated with 2.2- or 4.4 µg purified IHF_{*E*. *coli*}, saline diluent, or a recombinant protein of equivalent molecular mass called 'rsPilA' (Novotny et al. (2009) PLoS One 8:e67629), for 1 hr. Western blot was performed to confirm adsorption of anti-IHF*_{E. coli}*. To assess the functional consequence of adsorption of anti-IHF*_{E. coli}*, the adsorbed sera were then applied to 24 hr NTHI biofilms per standard treatment and processing protocol.

### Synergy of anti-IHF_{E. coli} and antibiotics against NTHI biofilms

To visualize changes in viability of NTHI biofilms upon exposure to antibiotics typically used to treat NTHI infections, 24 hr biofilms were established and incubated with 1:50 dilution of anti-IHF*_{E. coli}* or naive serum, ampicillin (32.0 µg ml⁻¹), cefdinir (0.25 µg ml⁻¹) or amoxicillin (1.0 µg ml⁻¹) plus clavulanate-lithium (0.5 µg ml⁻¹) for 16 hr. Each antibiotic was used at the MIC₉₀ for planktonic NTHI as determined via standard broth microdilution method (Biedenbach et al. (2003) Diagn. Microbiol. Infect. Dis. 46:55-61; Tristam et al. (2007) Clin. Microl. Rev. 20:368-389).

To quantitate NTHI adherent within the biofilm and bacteria newly released into the planktonic form after treatment, 24 hr biofilms were incubated with each antibiotic at the MIC₉₀ or a 4- or 8-fold dilution thereof, with or without antiserum. To culture newly released NTHI, supernatants were collected by aspiration, the biofilm gently washed twice with sterile saline to remove loosely adherent bacteria, and NTHI within the biofilm were recovered by repeated forceful pipetting. Planktonic and adherent bacteria were plated separately to determine the CFU NTHI ml⁻¹ and these values were combined to demonstrate total CFU bacteria as shown. Data represent mean + SEM of three independent assays.

### Synergy of anti-IHF_{E.coli} and antibiotics against planktonic NTHI

NTHI were prepared as described (Jurcisek et al. (2011) J. Vis. Exp.) and 10⁶ CFU NTHI inoculated into wells of a 96-well plate prior to incubation with antibiotic at the MIC₉₀ or a 4- or 8-fold dilution thereof, with or without 1:50 dilution of anti-IHF_{*E*.*coli*} or an equivalent volume of naive serum. After 16 hr, cultures were serially diluted and plated on to chocolate agar to semi-quantitate CFU NTHI/ well. Data represent mean + SEM of three independent assays.

### Epitope mapping NTHI IHF

To identify immunodominant regions within IHF, a series of twelve 20-mer synthetic peptides with 5-residue overlaps were synthesized to mimic the N- to C-terminus of the α-subunit of IHF predicted to be expressed by NTHI strain 86-028NP ('IHF_{NTHI}'). Synthesis, purification and sequence confirmation of all synthetic peptides was performed by Ohio Peptide, LLC. Archived samples of polyclonal sera recovered from chinchillas that had been immunized with either native IHF*_{E}*.*_{coli}* or IHF_{*E*.*coli*} pre-bound to an excess of double stranded DNA (Goodman et al. (2011) Mucosal. Immunol. 4:625-637) were used to map immunodominant epitopes of IHF. Analysis of interaction between IHF_{NTHI} synthetic peptides and antibodies present in chinchilla sera was determined using a Biacore 3000 (GE Healthcare) as described (Novotny et al. (2000) Infect. Immun. 68:2119-2128; Novotny et al. (2009) Vaccine 28:279-289). Reactivity of chinchilla sera to IHF_{NTHI} peptides was rendered using PyMol software (Schrödinger) to generate 3D model images.

### Assessment of IHF_{NTHI} epitope-specific antisera to disrupt NTHI biofilms

Based on results obtained from the epitope mapping study, two regions within the IHF_{NTHI} α-subunit were selected to generate polyclonal chinchilla antiserum: IhfA-3_{NTHI} (a non-reactive region) and IhfA-5_{NTHI} (reactive by antibodies against IHF_{*E*. *coli*} but nonreactive by anti-IHF_{*E*. *coli*} prebound to DNA). NTHI biofilms established for 24 hr were treated with 1:50 dilution of the following chinchilla sera: anti-IHF_{*E*. *coli*}, anti-IHF_{*E*.*coli*} pre-bound to DNA, naive serum, anti-IhfA-3_{NTHI} or anti-IhfA-5_{NTHI} for 16 hr prior to staining and assessment. Animal work was performed following the NIH Guide for the Care and Use of Laboratory Animals and under a protocol approved by the Nationwide Children's Hospital Institutional Animal Care and Use Committee.

### Statistical analyses

Statistical analyses were performed using GraphPad Prism software. Comparisons of biofilm biomass and thickness were determined using one-way analysis of variance (ANOVA) followed by Tukey's multiple comparisons test set at 5%. Significant differences in CFU NTHI following treatment were determined by one-way ANOVA followed by the Holm-Sidak test for multiple comparisons; a *p*-value ≤ 0.05 was considered significant.

### RESULTS

### Anti-IHF induced resolution of NTHI biofilms

Previous work demonstrates that polyclonal rabbit antiserum against *E*. *coli* IHF (or 'anti-IHF_{*E*. *coli*}') used at a 1:50 dilution, equivalent to 4.4 µg IHF-specific IgG ml⁻¹, disrupts a 24-hr NTHI biofilm *in vitro* (Goodman et al. (2011) Mucosal Immunol. 4:625-637). We next examined the relative effectiveness of anti-IHF_{*E*. *coli*} on both early-forming and mature NTHI biofilms. Biofilms formed for 16, 24, 48 or 96 hr and 1 or 2 weeks prior to treatment showed a marked decrease in remaining biofilm after incubation with anti-IHF_{*E*. *coli*}, whereas biofilms exposed to naive serum were comparable to those maintained in medium (**FIG. 10A**). Quantitatively, biomasses of 16-, 24- and 48-hr biofilms exposed to anti-IHF_{*E*. *coli*} were significantly reduced 94%, 86%, and 74%, respectively, compared to naive serum (*p*<0.05, **FIG. 10B**). To achieve a similar effect against more mature biofilms, anti-IHF_{*E*. *coli*} was diluted 1:10. Consequently, the biomasses for 96-hr or 1- or 2-week biofilms were significantly reduced by 74%, 43%, and 57%, respectively, compared to naive serum (*p*<0.01 or 0.05). As NTHI biofilms mature *in vitro,* the relative quantity of eDNA increases (Jones et al. (2013) J. Innate Immun. 5:24-38), thus it was not unexpected that a greater concentration of IHF-specific antiserum was required to achieve a similar reduction in biomass of these 'older' and more dense biofilms. Collectively, these data demonstrated that anti-IHF_{*E*. *coli*} was significantly effective and capable of disrupting both early-forming and mature NTHI biofilms.

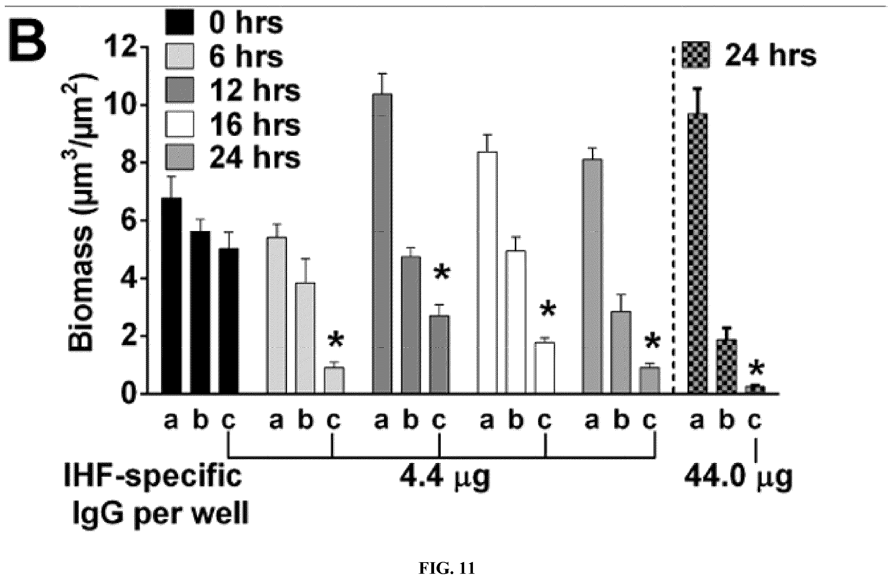

The kinetics of anti-IHF-mediated disruption were then examined. Biofilm established for 24 hrs were subsequently treated for 0, 6, 12, 16 or 24 hr with medium or either anti-IHF_{*E*. *coli*} or naive serum at a dilution of 1:50. Application, then instantaneous removal of treatments did not induce alteration in biofilm biomass relative to medium (**FIGS. 11A** **&** **11B**). However, biofilms exposed anti-IHF_{*E*. *coli*} for 6, 12, 16 or 24 hr demonstrated 76%, 43%, 65%, or 67% reduction in biomass compared to naive serum, respectively (*p*<0.01) with maximal reduction observed at the 6 hr time point. It was previously reported that the modest effect of naive serum from non-SPF animals compared to biofilms maintained in medium (Goodman et al. (2011) Mucosal Immunol. 4:625-637; Novotny et al. (2013) PLoS One 8:e67629). The inventors next examined whether a greater concentration of anti-IHF_{*E*. *coli*} or an extended exposure time could further reduce, or eradicate a pre-formed biofilm. Incubation of NTHI biofilms with anti-IHF_{*E*. *coli*} diluted 1:5 resulted in a decrease in biomass by 86% compared to naive serum (*p*<0.01; **FIGS. 11A** **&** **11B**). This outcome appeared maximal as neither a greater concentration of anti-IHF_{*E*. *coli*} nor an increase in treatment time to 24 hr fully eradicated all viable bacteria. In either case, a single layer of bacteria remained after treatment, which suggested that there was no target for anti-IHF_{*E*. *coli*} in these monolayers.

### Direct contact was not required for anti-IHF to disrupt NTHI biofilms

Up to this point, anti-IHF was applied directly to the NTHI biofilms. To now determine if direct contact between anti-IHF_{*E*. *coli*} and the biofilm was required, NTHI biofilms were established in the basolateral chamber of a transwell. IgG-enriched anti-IHF_{*E*. *coli*} covalently bound to agarose beads was placed into the apical chamber, thus physically separating the antibodies from the biofilm by the presence of a membrane. The inventors first confirmed that anti-IHF_{*E*. *coli*} coupled to agarose beads did not diffuse into the basolateral chamber by collection of medium within the basolateral chamber 24 hr after placement of antibody-bound beads into the apical chamber of the transwell by Western blot (**FIG. 18**). Compared to application of IHF_{*E*. *coli*} directly to the biofilm (**FIG. 12A**), biofilm reduction in the presence of anti-IHF_{*E*. *coli*} tethered to agarose beads within the apical chamber of the transwell was equivalent (**FIG. 12C**), and a significant reduction in biomass (*p*<0.05) was observed compared to tethered IgG from naive serum (**FIGS. 12B & 12F**). Although three concentrations of antibodies coupled to agarose beads were assayed, and all were effective, dose-dependent biofilm disruption was not observed. These data suggested a saturation of available antibody binding sites at the interface between the biofilm medium and the transwell membrane. To test this theory, a layer of naked agarose beads was placed below those to which IHF_{*E*. *coli*} antibodies had been bound, thus sterically blocking the ability to bind 'free' IHF. As anticipated, no disruption of the biofilm was observed (**FIGS. 12D & 12F**). However, biofilm disruption was restored upon mixing the naked beads and anti-IHF-bound antibodies (**FIG. 12E**). Collectively, these data demonstrated that anti-IHF_{*E*. *coli*} did not require direct contact with an NTHI biofilm to mediate disruption, and further, that this disruption was likely mediated by a forced equilibrium shift as free IHF is captured by antibodies when this protein naturally disassociated from eDNA within the biofilm (i.e. was in an 'off' state).

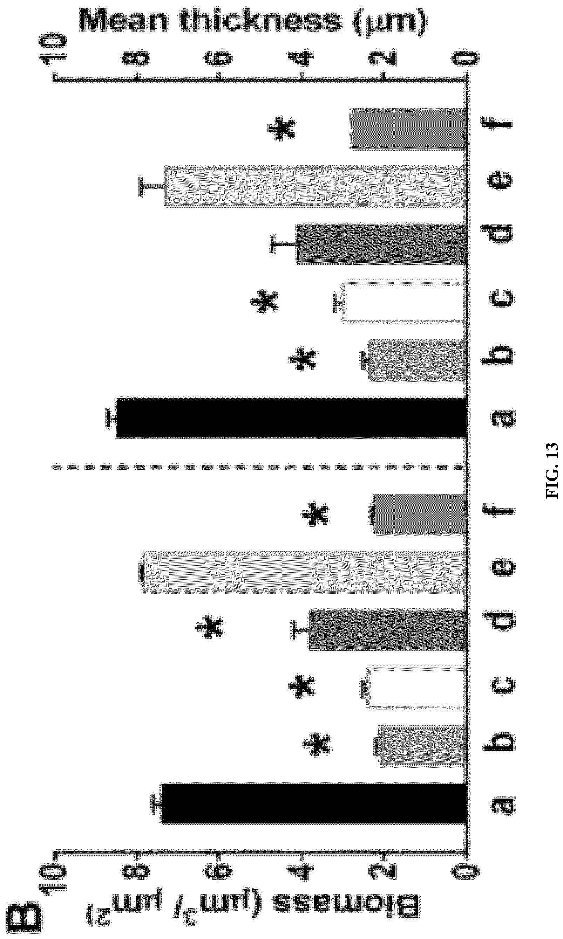

To demonstrate that the observed biofilm disruption was specifically mediated by antibodies directed against IHF and not due to the influence of other components within the hyperimmune rabbit serum (as this serum was not heat-activated prior to use) aliquots of anti-IHF_{*E*. *coli*} were adsorbed with either purified IHF, or as a negative control, an NTHI protein of comparable molecular mass called rsPilA (Novotny et al. (2009) Vaccine 28:279-289). The inventors first established that the arbitrarily-selected 1:50 dilution of antiserum utilized in the biofilm assays equated to application of 4.4 µg IHF-specific IgG to each biofilm. Western blotting revealed reduced reactivity of IHF-specific antibody after incubation with increasing concentrations of purified IHF (**FIG. 19**). This result was specific, as no decrease in reactivity was noted by incubation with the unrelated recombinant NTHI protein. Further, incubation of biofilms with the IHF-adsorbed sera showed a reduced ability to effectively disrupt the pre-formed biofilms, compared to whole serum (**FIGS. 13A** **&** **13B**). These data revealed that the observed NTHI biofilm disruption was mediated by IHF-specific antibodies within the polyclonal rabbit serum.

### Synergy of anti-IHF with antibiotics

The inventors next examined whether biofilm disruption by anti-IHF_{*E*. *coli*} increased susceptibility of the biofilm-resident bacteria to killing by antibiotics commonly used to treat NTHI infections. The minimum inhibitory concentration required to inhibit the growth of 90% (MIC₉₀) of planktonically-grown NTHI was determined prior (Tristram et al. (2007) Clin. Microbiol. Rev. 20:368-389) and the following concentrations were employed: ampicillin (32 µg ml⁻¹), amoxicillin-clavulanate (1 µg ml⁻¹ and 0.5 µg ml⁻¹, respectively) and cefdinir (0.25 µg ml⁻¹). Twenty four hr NTHI biofilms were then exposed to anti-IHF_{*E*. *coli*}, diluted 1:50, antibiotic or a combination of anti-IHF_{*E*. *coli*} plus antibiotic. As anticipated, anti-IHF_{*E*. *coli*} mediated significant biofilm disruption (**FIG. 14A**), however incubation with any of the three antibiotics at the MIC₉₀ for planktonic NTHI did not result in bacterial death as determined by viability stain (**FIGS. 14B-14D**) and the mean biofilm thickness and biomass was comparable between medium and antibiotic alone (**FIG. 14E**). Notably, treatment of an established NTHI biofilm with a combination of anti-IHF_{*E*. *coli*} and any of the three antibiotics induced a marked alteration in biofilm architecture and a statistically significant reduction in mean biomass (*p* ≤ 0.05), compared to treatment with antibiotic alone. Moreover, bacterial death was noted within the biofilms treated with anti-IHF_{*E*. *coli*} plus any of the three antibiotics. These data suggested that anti-IHF-mediated disruption of NTHI biofilms rendered the resident bacteria more susceptible to the actions of previously ineffective antimicrobials.

To extend this line of investigation beyond descriptive data of physical disruption of pre-formed biofilms, whether anti-IHF_{*E*. *coli*} exposure augmented antibiotic-mediated killing of the newly released bacteria was examined. To do so, the three targeted antibiotics were assayed at both the MIC₉₀ for planktonic NTHI (Biedenbach et al. (2003) Diagn. Microbiol. Infect. Dis. 46:291-294; Tristram et al. (2007) Clin. Microbiol. Rev. 20:368-389) as well as at a 4-fold or 8-fold dilution thereof. When incubated with any of the three antibiotics at the MIC₉₀, no significant difference in adherent colony-forming units (CFU) bacteria ml⁻¹ relative to medium (**FIGS. 15A-15C**) was observed. However, addition of anti-IHF_{*E*. *coli*} resulted in a significant reduction in number of NTHI that remained adherent to the chamber slide compared to medium (*p*<0.05) (Goodman et al. (2011) Mucosal Immunol. 4:625-637). Moreover, use of any antibiotic at the MIC₉₀ in combination with anti-IHF_{*E*. *coli*} induced an even greater significant reduction in the number of adherent bacteria (*p*<0.05) relative to medium alone. This reduction was maintained at an 8-fold less concentrations of ampicillin (4 µg ml⁻¹) and amoxicillin-clavulante (0.125 & 0.0625 µg each ml⁻¹), as well as at a 4-fold less concentration of cefdinir (0.0625 µg ml⁻¹).

To demonstrate that disruption of biofilms by anti-IHF_{*E*. *coli*} mediated enhanced killing of bacteria in both the adherent and planktonic populations, the total viable bacteria per well following treatment with each antibiotic delivered either with or without anti-IHF_{*E*. *coli*} (**FIGS. 16D-16F**) was examined. For all antibiotics, a significant reduction in total viable CFU NTHI/chamber slide well was observed when the antibiotic was used at either the MIC₉₀ or a 4-fold or 8-fold dilution thereof in combination with a 1:50 dilution of anti-IHF_{*E*. *coli*}. In all cases, these differences were significant compared to treatment with antibiotic alone, anti-IHF_{*E*. *coli*} alone or antibiotic plus naive serum (*p*< 0.05).

To determine if the newly released NTHI, or perhaps those still in less stringent association with the disrupted biofilm, were unusually susceptible to the action of these antibiotics NTHI broth cultures were treated with a 1:50 dilution of either anti-IHF_{*E*. *coli*} or naive serum, delivered alone or in the presence of ampicillin, amoxicillin/clavulanate, or cefdinir. Antibiotics were used at both the MIC₉₀ for planktonic NTHI and a 4-fold or 8-fold dilution thereof. No enhancement of antibiotic-mediated killing was observed upon exposure of planktonically grown NTHI to anti-IHF_{*E*. *coli*} alone (**FIGS. 20A-20C**), suggesting that NTHI newly released from the biofilm by the action of anti-IHF_{*E*. *coli*} were phenotypically unique from either their biofilm or planktonic counterparts.

### Identification of immunodominant regions within IHF

Previous work showed that immunization of chinchillas with native IHF_{*E*.*coli*} induced the formation of antibodies that rapidly resolved established NTHI biofilms within chinchilla middle ears during experimental OM; however, immunization with IHF_{*E*.*coli*} that had been pre-complexed to DNA (the form likely present in nature, during disease) did not resolve disease (Goodman et al. (2011) Mucosal Immunol. 4:625-637). Collectively, the data to date imply that there is a conserved domain within DNABII proteins (IHF and HU) of many bacteria which can be targeted for effective disruption of biofilm structure and that this domain is masked or occluded when IHF/HU is in association with DNA. To determine the location of this effective/masked domain, IHF from NTHI (referred to as 'IHF_{NTHI}')was epitope mapped using a series of 20-mer overlapping peptides designed to mimic the deduced N- to C-terminus of the α-subunit of this DNABII protein. These peptides were screened with antisera recovered from chinchillas that had been immunized with either native IHF_{*E*.*coli*} or IHF_{*E*.*coli*} that had been complexed to an excess of DNA (Goodman et al. (2011) Mucosal Immunol. 4:625-637). Antiserum against native IHF_{*E*.*coli*} was reactive with peptides predicted to represent the DNA-binding tip regions (**FIG. 16A**) whereas antiserum against the IHF_{*E*.*coli*}-DNA complex yielded the greatest reactivity to peptides representing the N-terminal tails of IHF_{NTHI} (**FIG. 16B**). This result was logical as the DNA-binding tip regions are likely occluded when IHF_{*E*.*coli*} is bound to DNA as shown in **FIG. 16C**; therefore whereas the tail region is exposed, the tip binding regions are predicted to be immunologically inaccessible.

As our epitope mapping study revealed specific regions within the IHF molecule to which serum antibodies were reactive, the inventors next sought to determine if antibodies directed against this targeted epitope were as effective as those directed against the native protein. To do so, two peptides were selected with which to generate immune serum in chinchillas: the peptide IhfA-5_{NTHI}, representing the reactive DNA tip-binding region within the α-subunit of IHF (**FIG. 16D**) and as a negative control, IhfA-3_{NTHI}, which represents a peptide of equal size but that was unreactive by epitope mapping (**FIG. 16D**). Purified IHF_{*E*.*coli*} and IHF_{*E*.*coli*} pre-complexed to DNA served as comparative immunogens. As expected, compared to naive chinchilla serum, NTHI biofilms incubated with anti-IHF_{*E*.*coli*} complexed to DNA or anti-IhfA-3_{NTHI} were not altered in biofilm morphology or biomass (**FIG. 16E & 16F**). However, similar to that observed with anti-IHF_{*E*.*coli*}, incubation with anti-IhfA-5_{NTHI} was equally efficacious to induce a significant reduction in biofilm biomass (*p*< 0.01) compared to naive serum.

### DISCUSSION

Bacterial biofilms are a significant contributor to most recurrent and chronic bacterial diseases, including those of the respiratory tract, urogenital tract and oral cavity. Biofilms are recalcitrant to action by the host immune system and antimicrobial agents, necessitating the development of novel treatment modalities for diseases with a biofilm component. eDNA is a prevalent component of the biofilm EPS of many microbes, and we previously demonstrated that members of the DNABII family of proteins play a crucial role in stabilizing the biofilm structure as exposure of biofilms to antibodies directed against IHF mediates significant disruption (Goodman et al. (2011) Mucosal Immunol. 4:625-637).

As NTHI biofilm age, there is an increase in the eDNA concentration within the EPS (Jones et al. (2013) J. Innate Immun. 5:24-38), and by inference, a concordant relative increase in the concentration of associated DNABII proteins. Here we showed that older biofilms thus required a greater concentration of anti-IHF_{*E*.*coli*} to mediate disruption. The ability of anti-IHF_{*E*.*coli*} to disrupt an established 24 hr NTHI biofilm was rapid, with maximal effects occurring within 6 hr of exposure (76% reduction in biomass and 71% reduction in mean thickness compared to naive serum) and no further disruption occurred after an additional 24 hr incubation with a single treatment. Regardless of the relative increase in concentration of anti-IHF_{*E*.*coli*} antibodies or exposure time, complete eradication of NTHI could not be accomplished. Instead a monolayer of viable bacteria remained, suggesting that in the absence of an EPS containing eDNA and IHF, there is no target for anti-IHF directed therapy.

Thereby, a combinatorial approach would likely be ideal and result in the ability to use already existing antibiotics, or other therapeutics, to resolve these diseases.

To better define the mechanism of action, whether direct contact between the biofilm and anti-IHF_{*E*.*coli*} antibodies was necessary for biofilm disruption was investigated, as it was previously hypothesized that such was not the case (Goodman et al. (2011) Mucosal Immunol. 4:625-637). As expected, separation of antibodies tethered to agarose beads from the biofilm by a microporous membrane did not inhibit biofilm disruption, suggesting that direct contact was not needed. Instead, collectively the data indicated that antibodies directed against anti_{*E*.*coli*} captured free IHF as it disassociated from eDNA within the biofilm as part of the normal equilibrium between IHF with DNA. Indeed, epitope mapping experiments point to competitive inhibition of the DNA binding domain of IHF as the site of antibody action. The presence of an excess of antibodies to IHF thus shifts this equilibrium, mediating collapse or disruption of the biofilm structure.

As treatment of a biofilm with anti-IHF mediates release of bacteria into the planktonic phase (Goodman et al. (2011) Mucosal Immunol. 4:625-637), whether anti-IHF_{*E*.*coli*} could act in a combinatorial manner with traditional antibiotics to augment their killing ability was tested. For three antibiotics traditionally used to treat recalcitrant respiratory tract infections due to NTHI, it was determined that this was indeed the case. Treatment of established biofilms with anti-IHF_{*E*.*coli*} plus antibiotic rendered resident bacteria susceptible to killing. Moreover, these newly released bacteria demonstrated increased susceptibility to killing that was not due to exposure to anti-IHF_{*E*.*coli*} alone. All three antibiotics tested were able to mediate killing when used at a concentration 4- to 8-fold less than the MIC₉₀ for planktonically growing cells, thus demonstrating true synergy. These findings also suggested the possibility of a unique phenotype for NTHI that had been newly released from biofilm growth, compared to either those resident within a biofilm or planktonically grown. Importantly, a similar observation has been made for *S. pneumoniae* in pioneering work by Anders Hakansson, who found that pneumococci released from biofilms, as mediated via several treatments, have a unique transcriptome and increased virulence compared to both bacteria growing as a biofilm as well as to planktonic bacteria grown in rich medium (Marks et al. (2013) MBio. 4).

Collectively, these data support a model to describe the mechanism by which antiserum against IHF induces disruption of an established biofilm. Exposure of biofilms to anti-IHF induces an equilibrium shift between IHF molecules bound to eDNA within a biofilm (or 'on') and those in an 'off' state. IHF molecules free in the surrounding aqueous milieu are removed, forcing bound IHF to dissociate from biofilm eDNA, thus mediating a structural collapse of the biofilm. These observations demonstrate the potential to target a molecule of critical structural importance to biofilm integrity for the treatment of multiple diseases with a biofilm component.

Unless otherwise defined, all technical and scientific terms used herein have the same meaning as commonly understood by one of ordinary skill in the art to which this invention belongs. All nucleotide sequences provided herein are presented in the 5' to 3' direction.

The inventions illustratively described herein may suitably be practiced in the absence of any element or elements, limitation or limitations, not specifically disclosed herein. Thus, for example, the terms "comprising", "including," containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed.

The foregoing description and examples are intended to illustrate and not limit the scope of the invention.

**TABLE 1**

| **Gram (+) - only HU, Gram (-) - all have HU some also IHF** | | | |
|---|---|---|---|
| **Bacteria strain** | **Abbreviation** | **Protein name(s)** | |
| *S. sobrinus* 6715 | *Ss* | 1310 | (HU) (not fully sequenced) |
| *S. pyogenes* MGAS10270 | *Spyog* | Spy1239 | (HU) |
| *S. gordonii* Challis NCTC7868 | *Sg* | SGO_0701 | (HlpA) |
| *S. agalactiae* (Group B Strep)2603V/R | GBS | SAG_0505 | (Hup) |
| *S. mutans* UA159 | *Sm* | Smu_589 | (HU) |
| *S. pneumoniae* R6 | *Spneu* | spr1020 | (HU) |
| *S. gallolyticus* UCN34 (*S. bovis)* | *Sgall* | YP_003430069 | (HlpA) |
| *S. aureus* MW2 | *Sa* | MW1362 | (HU) |
| *S. epidermidis* RP62A | *Se* | SERP1041 | (Hup) |
| *E. coli K12-MG1655* | *Ec* | b1712 | (HimA) |
| | | b0912 | (HimD) |
| | | | (HupA) |
| | | | (HupB) |
| *H. influenza KW20 Rd* | *Hi* | HI1221 | (HimA) |
| | | HI1313 | (HimD) |
| | | HI0430 | (HupA) |
| *Salmonella enteric serovar typhi CT18* | *Salm* | Sty1771 | (HimA) |
| | | Sty0982 | (HimD) |
| *Aggregatibacter actinomycetemcomitans* D11S-1 | *Aa* | YP_003255965 | (IHFalpha) |
| | | YP_003256209 | (IhfB) |
| | | YP_003255304 | (HU) |
| *P. gingivalis* W83 | *Pg* | PG_0121 | (Hup-1) |
| | | PG_1258 | (Hup-2) |
| *N. gonorrhoeae* FA1090 (Oklahoma) | *Ng* | NGO603 | (IHFβ) |
| | | NGO030 | (IHFα) |
| *N. meningitides* MC58 | *Nm* | NMB_0729 | (HimA) |
| | | NMB_1302 | (HimA) |
| *P. aeruginosa* | *Pa* | PA3161 | (HimD) |
| | | PA1804 | (HupB) |
| | | PA2758 | (HimA) |
| *H. pylori* 26695 | *Hp* | Hp0835 | (Hup) |
| *B. burgdorferi* B31 | *Bb* | BB_0232 | (Hbb) |
| *Moraxella catarrhalis* RH4 | *Mc* | YP_003626307 | (HimA) |
| | | YP_003627027 | (HimD) |
| | | YP_003626775 | (HupB) |
| *V. cholera* El Tor N16961 | *Vc* | VC_0273 | (HupA) |
| | | VC_1914 | (HipB) |
| | | VC_1919 | (HupB) |
| | | VC_1222 | (HimA) |
| *Burkholderia cenocepacia* HI2424 | *Bc* | Bcen2424_1048 | (IHFB) |
| | | Bcen2424_1481 | (IHFA) |
| *Burkholderia pseudomallei* 668 | *Bp* | BURPS668_2881 | (IHFB) |
| | | BURPS668_1718 | (IHFA) |
| *Mycobacterium tuberculosis* CDC1551 | *Mtb* | MT_3064 | (HU) |
| *Mycobacterium smegmatis* MC2 | Ms | MSMEG_2389 | (Hup) |
| *Treponema denticola* ATCC 35405 | *Td* | TDE_1709 | (HU) |
| *Treponema palladum* Nichols | *Tp* | TP_0251 | (DNA binding protein II) |
| *Prevotella melaninogenica* ATCC 25845 | *Pm* | PREME0022_2103 | (HupB) |
| | | PREME0022_0268 | (HupA) |
| | | PREME0022_0341 | (Hup) |
| | | PREME0022_0340 | (HimA) |
| *Prevotella intermedia* 17 | *Pi* | PIN_A0704 | (Hup) |
| | | PIN_A1504 | (Hup-2) |
| | | PIN_0345 | (HimA) |
| | | PIN_0343 | (Hypothetical protein) |
| *Bordetella pertusis* Tohama 1 | *Bpert* | BP2572 | (IhfA) |
| | | BP3530 | (HupB) |
| | | BP0951 | (IhfB) |
| *Enterococcus faecalis V583* | *Ef* | Ef1550 | (hup) |

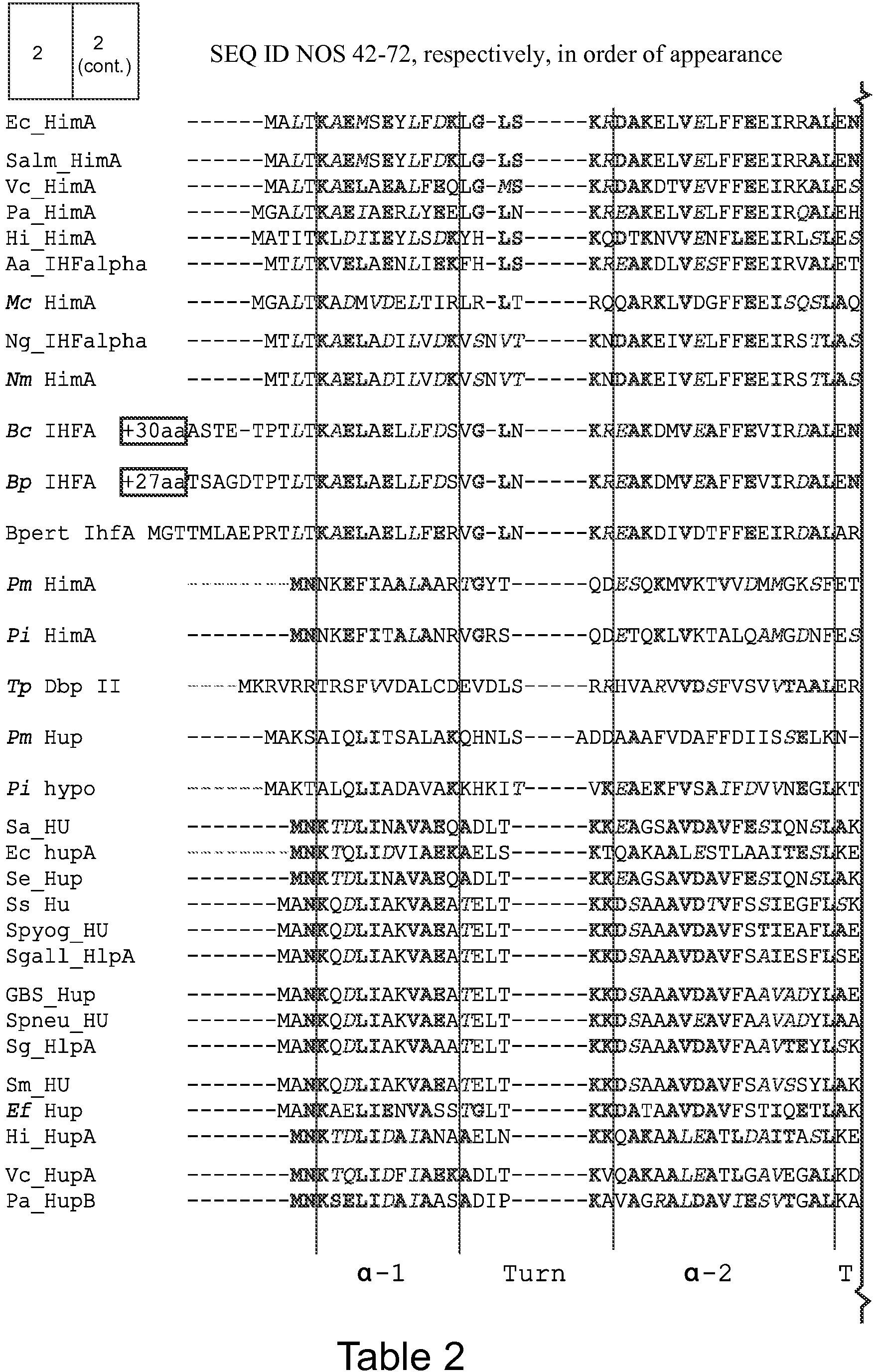

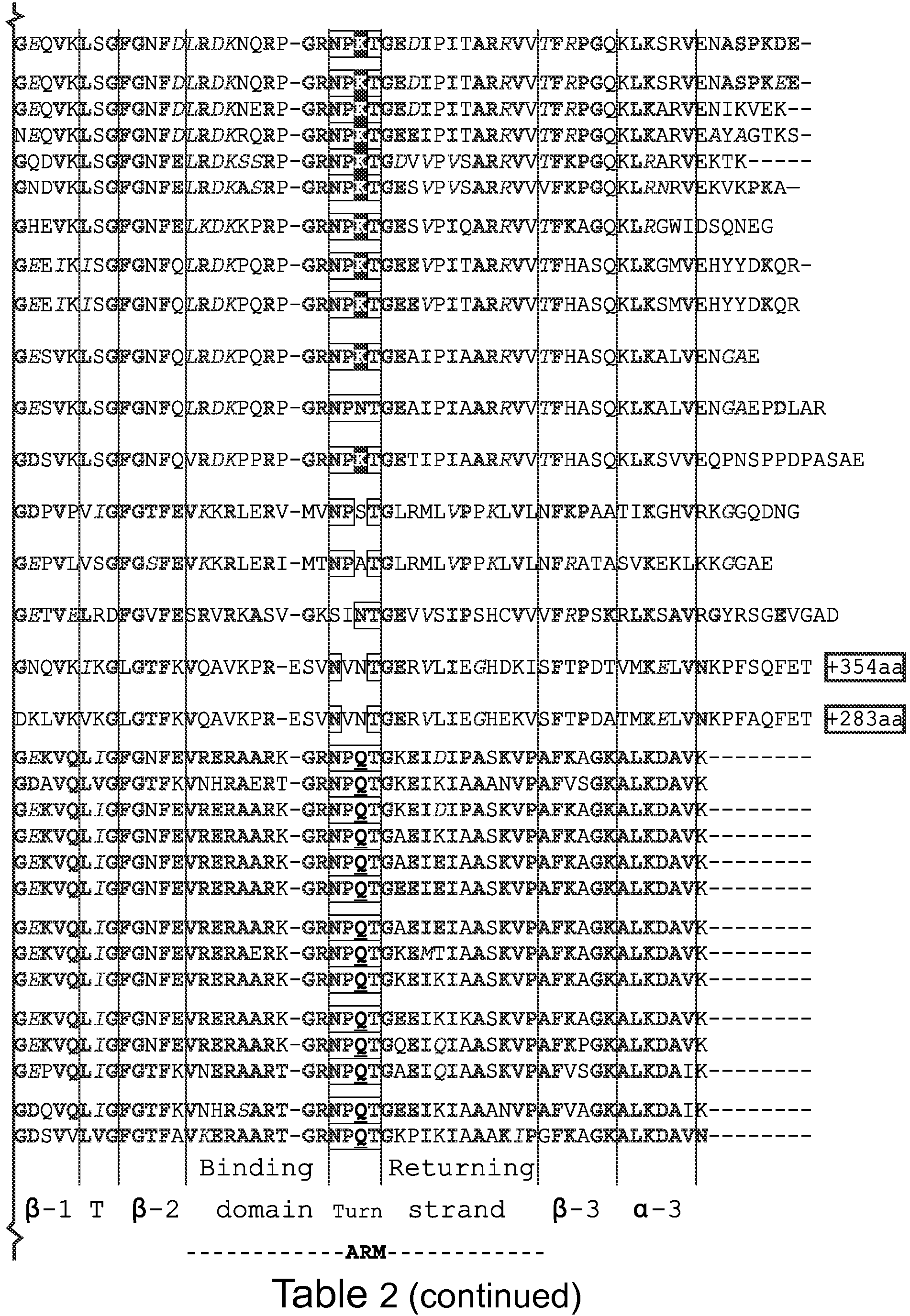

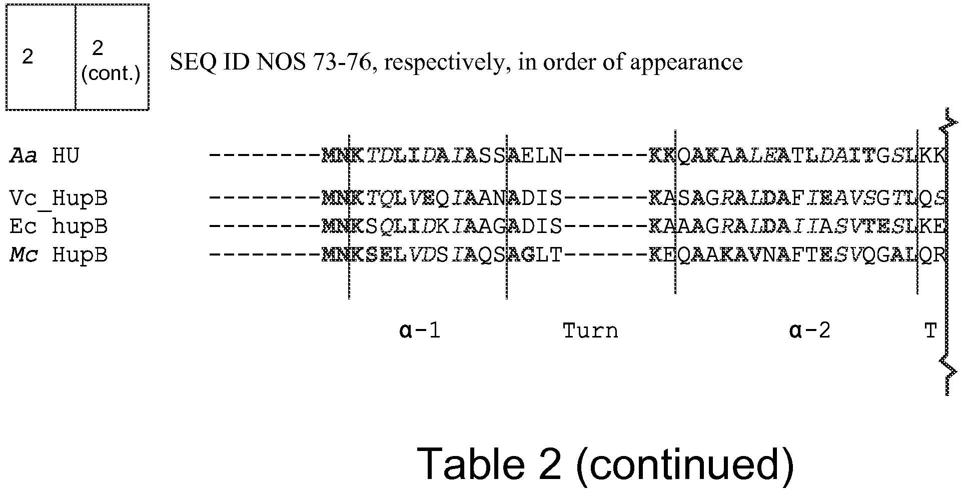

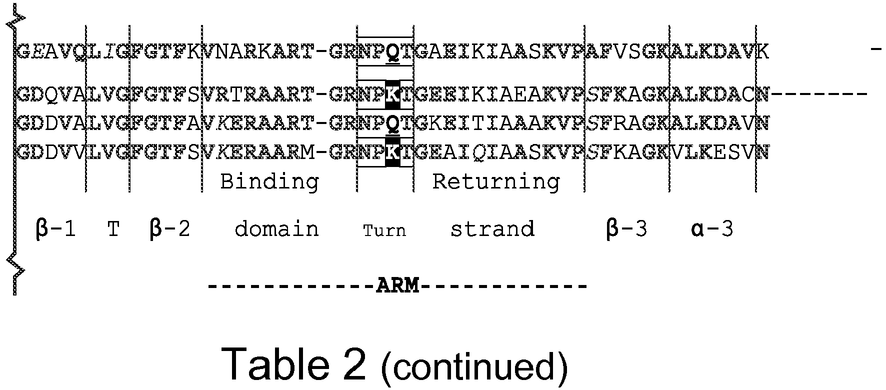

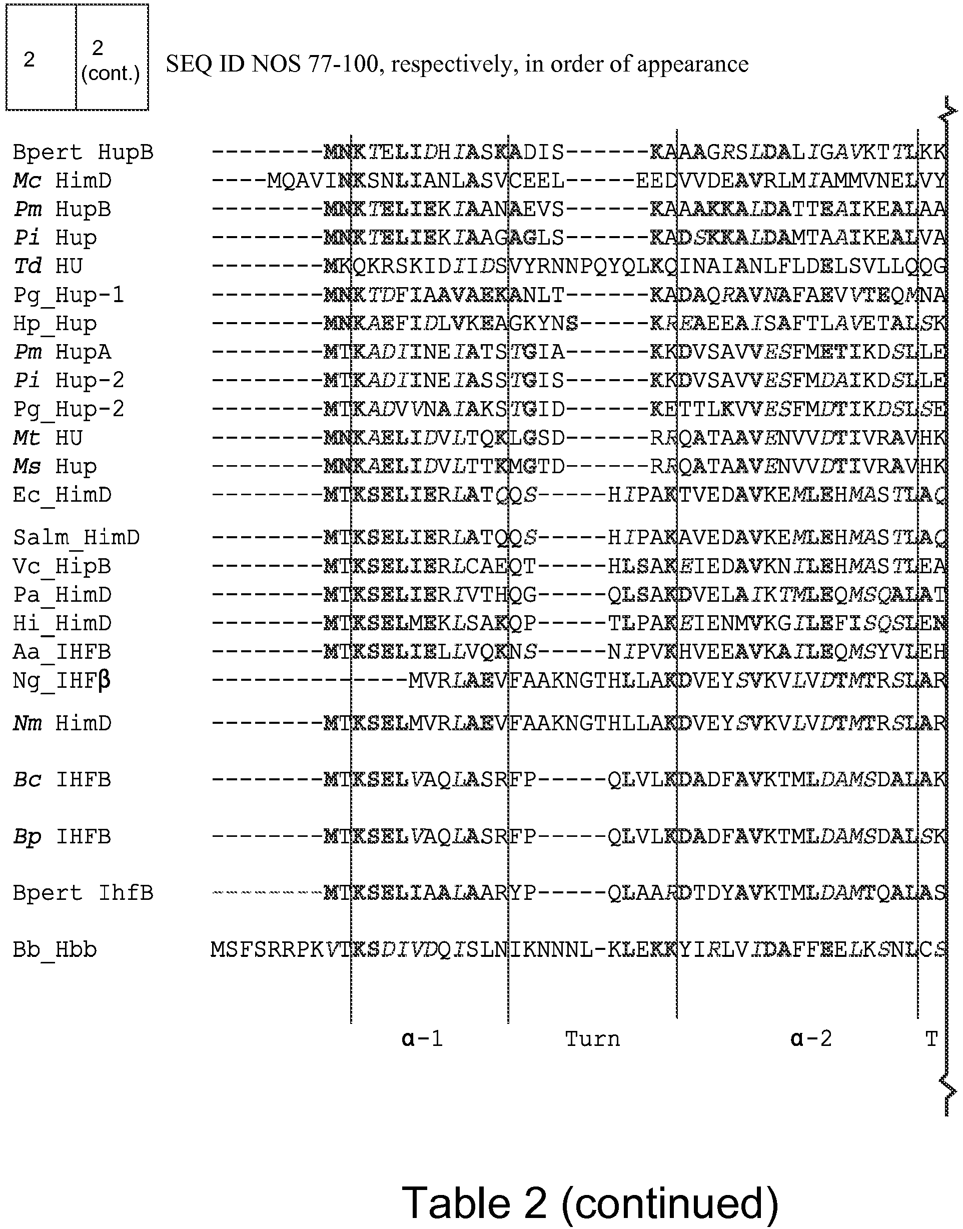

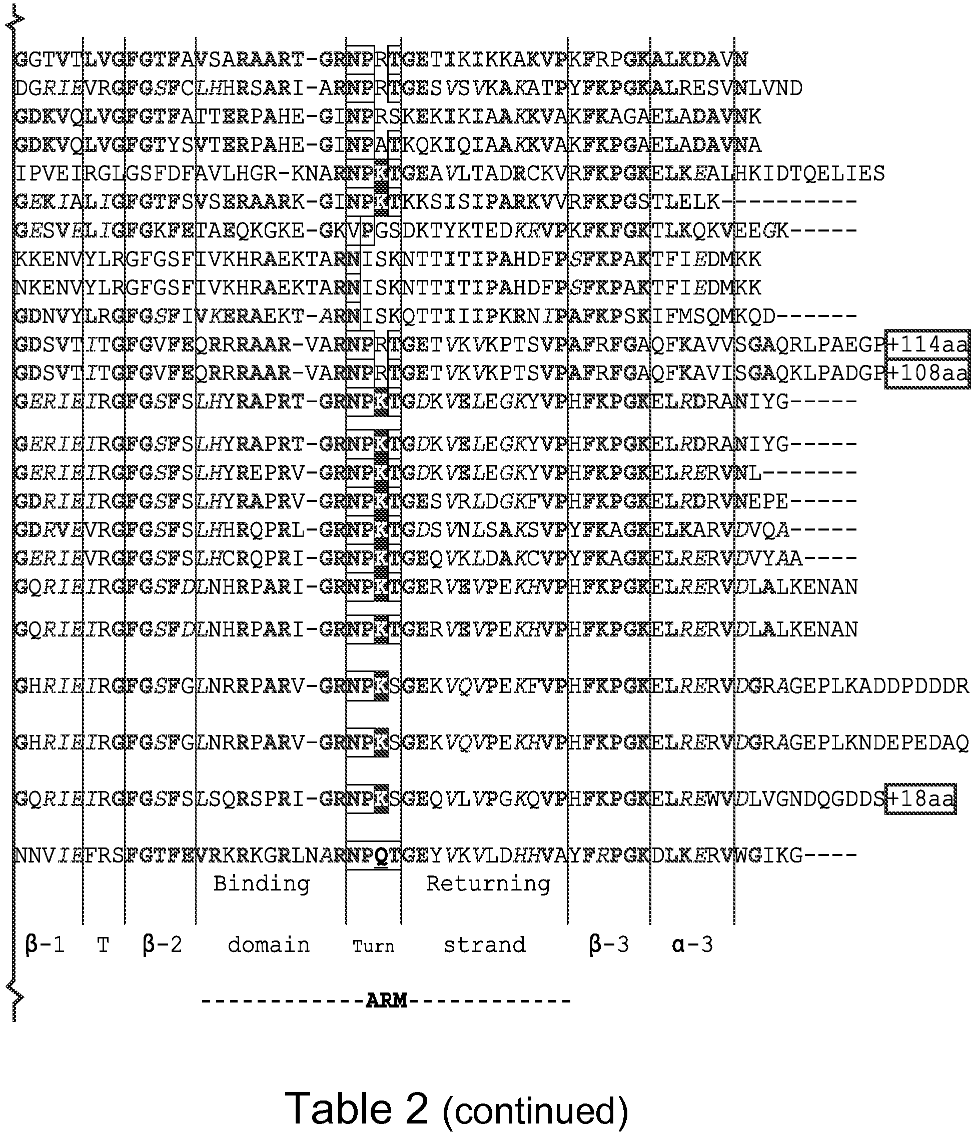

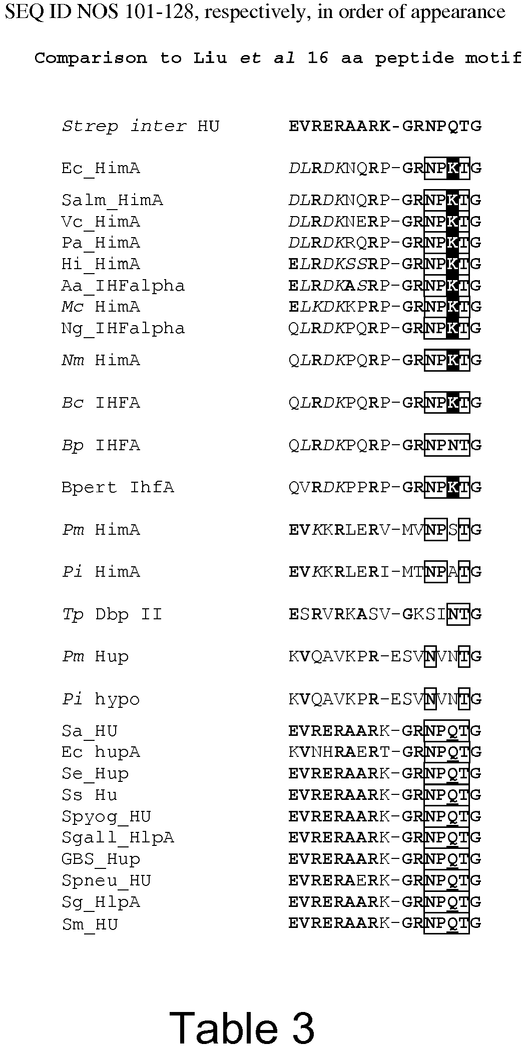

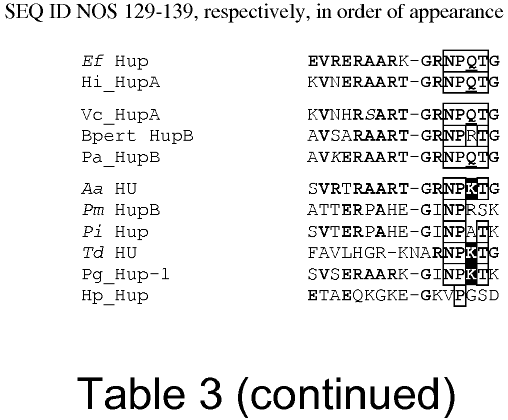

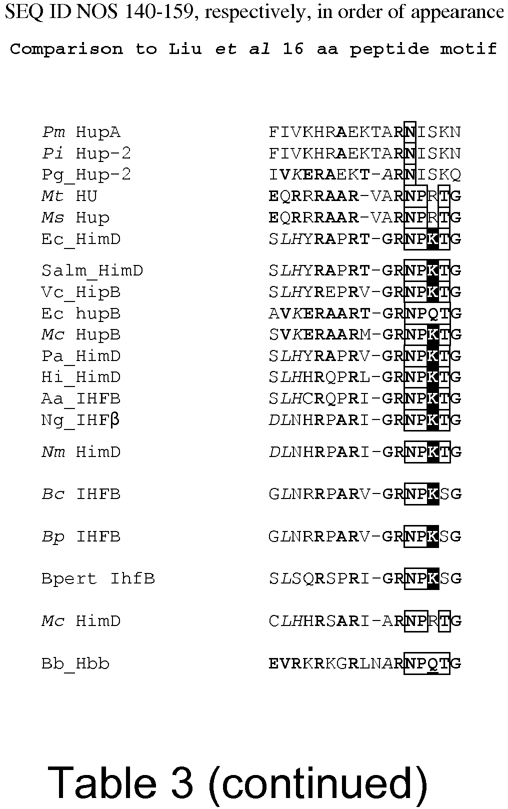

**TABLE 4 (SEQ ID NOS 160-336, respectively, in order of appearance)**

| **Bacteria strain, protein name** | **β3 sequence** | **α3 sequence** | **C-terminal 20 aa** |
|---|---|---|---|
| *S. pyogenes* MGAS10270, HU | AFKAGK | ALKDAVK | IAASKVPAFKAGKALKDAVK |
| *S. gallolyticus* UCN34 (*S. bovis),* HlpA | AFKAGK | ALKDAVK | IAASKVPAFKAGKALKDAVK |
| *S. sobrinus* 6715 HU | AFKAGK | ALKDAVK | IAASKVPAFKAGKALKDAVK |
| *S. agalactiae* (Group B Strep)2603V/R Hup | AFKAGK | ALKDAVK | IAASKVPAFKAGKALKDAVK |
| *S. pneumoniae* R6 HU | AFKAGK | ALKDAVK | IAASKVPAFKAGKALKDAVK |
| *S. gordonii* Challis NCTC7868, HlpA | AFKAGK | ALKDAVK | IAASKVPAFKAGKALKDAVK |
| *S. mutans* UA159, HU | AFKAGK | ALKDAVK | IKASKVPAFKAGKALKDAVK |
| *Enterococcus faecalis V583,* Hup | AFKPGK | ALKDAVK | IAASKVPAFKPGKALKDAVK |
| *S. aureus* MW2, HU | AFKAGK | ALKDAVK | IPASKVPAFKAGKALKDAVK |
| *S. epidermidis* RP62A Hup | AFKAGK | ALKDAVK | IPASKVPAFKAGKALKDAVK |
| *H. influenza KW20 Rd* HupA | AFVSGK | ALKDAIK | IAASKVPAFVSGKALKDAIK |
| *Aggregatibacter actinomycetemcomitans* D11S-1 HU | AFVSGK | ALKDAVK | IAASKVPAFVSGKALKDAVK |
| *V. cholera* El Tor N16961, HupA | AFVAGK | ALKDAIK | IAAANVPAFVAGKALKDAIK |
| *E. coli K12-MG1655* hupA | AFVSGK | ALKDAVK | IAAANVPAFVSGKALKDAVK |
| *P. aeruginosa* HupB | GFKAGK | ALKDAVN | IAAAKIPGFKAGKALKDAVN |
| *E. coli K12-MG1655* hupB | SFRAGK | ALKDAVN | IAAAKVPSFRAGKALKDAVN |
| *V. cholera* El Tor N16961 HupB | SFKAGK | ALKDACN | IAEAKVPSFKAGKALKDACN |
| *Bordetella pertusis* Tohama 1 HupB | KFRPGK | ALKDAVN | IKKAKVPKFRPGKALKDAVN |
| *Prevotella melaninogenica* ATCC 25845 HupB | KFKAGA | ELADAVNK | AAKKVAKFKAGAELADAVNK |
| *Prevotella intermedia* 17 Hup | KFKPGA | ELADAVNA | AAKKVAKFKPGAELADAVNA |
| *Moraxella catarrhalis* RH4 HupB | SFKAGK | VLKESVN | IAASKVPSFKAGKVLKESVN |
| *P. gingivalis* W83 Hup-1 | RFKPGS | TLELK | ISIPARKVVRFKPGSTLELK |
| *H. pylori* 2669 Hup | KFKPGK | TLKQKVEEGK | KRVPKFKPGKTLKQKVEEGK |
| *Prevotella melaninogenica* ATCC 25845 HupA | SFKPAK | TFIEDMKK | PAHDFPSFKPAKTFIEDMKK |
| *Prevotella intermedia* 17 Hup-2 | SFKPAK | TFIEDMKK | PAHDFPSFKPAKTFIEDMKK |
| *P. gingivalis* W83 Hup-2 | AFKPSK | IFMSQMKQD | KRNIPAFKPSKIFMSQMKQD |
| *Mycobacterium tuberculosis* CDC1551 HU | AFRPGA | QFKAVVSGAQRLPAEGPAVKRG | AKRPATKAPAKKATARRGRK |
| *Mycobacterium smegmatis* MC2 Hup | AFRPGA | QFKAVISGAQKLPADGPAVKRG | TKAPAKKAAAKKAPAKKGRR |
| *Prevotella melaninogenica* ATCC 25845 HimA | NFKPAA | TIKGHVRKGGQDNG | NFKPAATIKGHVRKGGQDNG |
| *Prevotella intermedia* 17 HimA | NFRATA | SVKEKLKKGGAE | VLNFRATASVKEKLKKGGAE |
| *E. coli K12-MG1655* HimA | TFRPGQ | KLKSRVENASPKDE | TFRPGQKLKSRVENASPKDE |
| *Salmonella enteric serovar typhi CT18* HimA | TFRPGQ | KLKSRVENASPKEE | TFRPGQKLKSRVENASPKEE |
| *V. cholera* El Tor N1696 HimA | TFRPGQ | KLKARVENIKVEK | VTFRPGQKLKARVENIKVEK |
| *P. aeruginosa* HimA | TFRPGQ | KLKARVEAYAGTKS | TFRPGQKLKARVEAYAGTKS |
| *Burkholderia cenocepacia* HI2424 IHFA | TFHASQ | KLKALVENGAE | RVVTFHASQKLKALVENGAE |
| *Burkholderia pseudomallei* 668 IHFA | TFHASQ | KLKALVENGAEPDLAR | HASQKLKALVENGAEPDLAR |
| *Bordetella pertusis* Tohama 1 IhfA | TFHASQ | KLKSVVEQPNSPPDPASAE | QKLKSVVEQPNSPPDPASAE |
| *N. gonorrhoeae* FA1090 (Oklahoma) IHFa | TFHASQ | KLKGMVEHYYDKQR | TFHASQKLKGMVEHYYDKQR |
| *N. meningitides* MC58 HimA | TFHASQ | KLKSMVEHYYDKQR | TFHASQKLKSMVEHYYDKQR |
| *H. influenza KW20 Rd* HimA | TFKPGQ | KLRARVEKTK | RRVVTFKPGQKLRARVEKTK |
| *Aggregatibacter actinomycetemcomitans* D11S-1 HimA | VFKPGQ | KLRNRVEKVKPKA | VVFKPGQKLRNRVEKVKPKA |
| *Moraxella catarrhalis* RH4 HimA | TFKAGQ | KLRGWIDSQNEG | VVTFKAGQKLRGWIDSQNEG |
| *Treponema palladium* Nichols DNA_binding_protein_II | VFRPSK | RLKSAVRGYRSGEVGAD | PSKRLKSAVRGYRSGEVGAD |
| *Prevotella melaninogenica* ATCC 25845 Hup | SFTPDT | VMKELVNKPFSQFETVVINDGV | MQAGDTMKVPKVELRPEYRK |
| *Prevotella intermedia* 17 hypothetical | SFTPDA | TMKELVNKPFAQFETVVLNDGV | SAGDTMKVPKVELRPQYRTK |
| *E. coli K12-MG1655* HimD | HFKPGK | ELRDRANIYG | KYVPHFKPGKELRDRANIYG |
| *Salmonella enteric serovar typhi CT18 b*HimD | HFKPGK | ELRDRANIYG | KYVPHFKPGKELRDRANIYG |
| *V. cholera* El Tor N1696 HipB | HFKPGK | ELRERVNL | EGKYVPHFKPGKELRERVNL |
| *P. aeruginosa* HimD | HFKPGK | ELRDRVNEPE | KFVPHFKPGKELRDRVNEPE |
| *H. influenza KW20 Rd* HimD | YFKAGK | ELKARVDVQA | KSVPYFKAGKELKARVDVQA |
| *Aggregatibacter actinomycetemcomitans* D11S-1 IHFB | YFKAGK | ELRERVDVYAA | CVPYFKAGKELRERVDVYAA |
| *N. gonorrhoeae* FA1090 (Oklahoma) IHFβ | HFKPGK | ELRERVDLALKENAN | FKPGKELRERVDLALKENAN |
| *N. meningitides* MC58 HimD | HFKPGK | ELRERVDLALKENAN | FKPGKELRERVDLALKENAN |
| *Burkholderia cenocepacia* HI2424 IHFB | HFKPGK | ELRERVDGRAGEPLKADDPDDDR | ERVDGRAGEPLKADDPDDDR |
| *Burkholderia pseudomallei* 668 IHFB | HFKPGK | ELRERVDGRAGEPLKNDEPEDAQ | ERVDGRAGEPLKNDEPEDAQ |
| *Bordetella pertusis* Tohama 1 IhfB | HFKAGK | ELREWVDLVGNDQGDDSSNGSS | DSSNGSSDPLQSVMDMHAMH |
| *Moraxella catarrhalis* RH4 HimD | YFKPGK | ALRESVNLVND | ATPYFKPGKALRESVNLVND |
| *B. burgdorferi* B31 Hbb | YFRPGK | DLKERVWGIKG | HVAYFRPGKDLKERVWGIKG |
| *Treponema denticola* ATCC 35405 HU | RFKPGK | ELKEALHKIDTQELIES | PGKELKELKEALHKIDTQELIES |

## Claims

1. An anti-IHF antibody composition for (i) use in treating or preventing the recurrence of a *Burkholderia* infection in a Cystic Fibrosis (CF) patient in need thereof, (ii) use in treating an infection or disease caused by *Burkholderia* in a CF patient infected with *Burkholderia,*
wherein the antibody specifically recognizes and binds the amino acid sequence RPGRNPKTGDVVPVSARRVV.

2. The antibody composition for use according to claim 1, wherein the anti-IHF antibody is administered to the patient in the absence of a DNase treatment.

3. The antibody composition for use according to claim 1 or 2, and an antimicrobial that inhibits the growth of the *Burkholderia*, optionally *Burkholderia cenocepacia* or *B. multivorans*.

4. The antibody composition for use according to any one of claims 1 to 3 wherein the anti-IHF antibody is an IgG antibody, a polyclonal antibody, a monoclonal antibody or an antigen binding fragment thereof.

5. The antibody composition for use according to any one claims 1 to 4, wherein the subject is a mammal, optionally a human patient, and/or a pediatric patient.

6. The antibody composition for use according to any one of claims 1 to 5 wherein the subject infected with *Burkholderia* is a cystic fibrosis subject.

7. The antibody composition for use according to any one of claims 1 to 6, further comprising a step of contacting the biofilm or *Burkholderia* with an effective amount of an antimicrobial that inhibits the growth of the *Burkholderia* producing the biofilm.

8. An isolated peptide consisting of the sequence RPGRNPKTGDVVPVSARRVV and optionally further comprising an adjuvant or detectable label.

9. An isolated antibody that specifically recognizes and binds the isolated peptide of claim 8 that is optionally a polyclonal antibody, a monoclonal antibody or an antigen binding fragment thereof, that optionally further comprises a pharmaceutically acceptable carrier.

10. A hybridoma cell line producing the monoclonal antibody of claim 9.

11. An isolated polynucleotide encoding an isolated peptide of claim 8 or the antibody of claim 9 or 10.

## Patentansprüche

1. Anti-IHF-Antikörperzusammensetzung zur (i) Verwendung bei der Behandlung oder Vorbeugung des Wiederauftretens einer *Burkholderia*-Infektion in einem Patienten mit Zystischer Fibrose (cystic fibrosis, CF), der dessen bedarf, (ii) Verwendung bei der Behandlung einer Infektion oder Erkrankung, die durch *Burkholderia* in einem CF-Patienten, der mit *Burkholderia* infiziert ist, verursacht wurde,
wobei der Antikörper die Aminosäuresequenz RPGRNPKTGDVVPVSARRVV spezifisch erkennt und bindet.

2. Antikörperzusammensetzung zur Verwendung nach Anspruch 1, wobei der Anti-IHF-Antikörper dem Patienten in Abwesenheit einer DNase-Behandlung verabreicht wird.

3. Antikörperzusammensetzung zur Verwendung nach Anspruch 1 oder 2 und ein antimikrobielles Mittel, das das Wachstum von *Burkholderia,* gegebenenfalls *Burkholderia cenocepacia* oder *B*. *multivorans* hemmt.

4. Antikörperzusammensetzung zur Verwendung nach einem der Ansprüche 1 bis 3, wobei der Anti-IHF-Antiköper ein IgG-Antikörper, ein polyclonaler Antikörper, ein monoclonaler Antikörper oder ein Antigen-bindendes Fragment davon ist.

5. Antikörperzusammensetzung zur Verwendung nach einem der Ansprüche 1 bis 4, wobei das Individuum ein Säuger, gegebenenfalls ein humaner Patient und/oder ein pädiatrischer Patient ist.

6. Antikörperzusammensetzung zur Verwendung nach einem der Ansprüche 1 bis 5, wobei das Individuum, das mit *Burkholderia* infiziert ist, ein Individuum mit Zystischer Fibrose ist.

7. Antikörperzusammensetzung zur Verwendung nach einem der Ansprüche 1 bis 6, des Weiteren umfassend einen Schritt des Inkontaktbringens des Biofilms oder *Burkholderia* mit einer wirksamen Menge eines antimikrobiellen Mittels, das das Wachstum des *Burkholderia,* das den Biofilm herstellt, hemmt.

8. Isoliertes Peptid, das aus der Sequenz RPGRNPKTGDVVPVSARRVV besteht und gegebenenfalls des Weiteren ein Adjuvans oder eine nachweisbare Markierung umfasst.

9. Isolierter Antikörper, der das isolierte Peptid nach Anspruch 8 spezifisch erkennt und bindet, welcher gegebenenfalls ein polyclonaler Antikörper, ein monoclonaler Antikörper oder ein Antigen-bindendes Fragment davon ist, und der gegebenenfalls des Weiteren einen pharmazeutisch verträglichen Träger umfasst.

10. Hybridomzelllinie, die den monoclonalen Antikörper nach Anspruch 9 herstellt.

11. Isoliertes Polynucleotid, das ein isoliertes Peptid nach Anspruch 8 oder den Antikörper nach Anspruch 9 oder 10 codiert.

## Revendications

1. Composition d'anticorps anti-IHF pour (i) une utilisation dans le traitement ou la prévention de la récurrence d'une infection par *Burkholderia* chez un patient atteint de fibrose kystique (CF) qui en a besoin, (ii) une utilisation dans le traitement d'une infection ou d'une maladie causée par *Burkholderia* chez un patient CF infecté par *Burkholderia,*
dans laquelle l'anticorps reconnaît spécifiquement et se lie à la séquence d'acides aminés RPGRNPKTGDVVPVSARRVV.

2. Composition d'anticorps pour une utilisation selon la revendication 1, dans laquelle l'anticorps anti-IHF est administré au patient en l'absence d'un traitement à la DNase.

3. Composition d'anticorps pour une utilisation selon la revendication 1 ou 2, et antimicrobien qui inhibe la croissance de la *Burkholderia,* facultativement de la *Burkholderia cenocepacia* ou de la *B. multivorans.*

4. Composition d'anticorps pour une utilisation selon l'une quelconque des revendications 1 à 3, dans laquelle l'anticorps anti-IHF est un anticorps IgG, un anticorps polyclonal, un anticorps monoclonal ou un fragment de liaison antigène de celui-ci.

5. Composition d'anticorps pour une utilisation selon l'une quelconque des revendications 1 à 4, dans laquelle le sujet est un mammifère, facultativement un patient humain et/ou un patient pédiatrique.

6. Composition d'anticorps pour une utilisation selon l'une quelconque des revendications 1 à 5, dans laquelle le sujet est infecté par *Burkholderia* est un sujet atteint de fibrose kystique.

7. Composition d'anticorps pour une utilisation selon l'une quelconque des revendications 1 à 6, comportant en outre une étape consistant à mettre en contact le biofilm de *Burkholderia* avec une quantité efficace d'un antimicrobien qui inhibe la croissance de la *Burkholderia* produisant le biofilm.

8. Peptide isolé constitué de la séquence RPGRNPKTGDVVPVSARRVV et comportant en outre facultativement un adjuvant ou un marqueur détectable.

9. Anticorps isolé qui reconnaît spécifiquement et se lie au peptide isolé de la revendication 8 qui est facultativement un anticorps polyclonal, un anticorps monoclonal ou un fragment de liaison antigène de celui-ci, qui comporte en outre facultativement un vecteur pharmaceutiquement acceptable.

10. Lignée cellulaire d'hybridome produisant l'anticorps monoclonal selon la revendication 9.

11. Polynucléotide isolé codant un peptide isolé selon la revendication 8 ou l'anticorps selon la revendication 9 ou 10.
